(19) Europäisches Patentamt
European Patent Office
Office européen des brevets

(11) EP 0 468 470 B1

(12) EUROPÄISCHE PATENTSCHRIFT

(45) Veröffentlichungstag und Bekanntmachung des Hinweises auf die Patenterteilung:
**16.04.1997 Patentblatt 1997/16**

(21) Anmeldenummer: **91112404.8**

(22) Anmeldetag: **22.07.1991**

(51) Int Cl.⁶: **C07D 403/14**, A61K 31/415, C07D 235/08, C07D 403/10, C07D 403/04, A61K 31/445, A61K 31/40, A61K 31/55

(54) **Benzimidazole, diese Verbindungen enthaltende Arzneimittel und Verfahren zu ihrer Herstellung**

Benzimidazoles, medicaments containing them and process for their preparation

Benzimidazoles, médicaments les contenant et procédé pour leur préparation

(84) Benannte Vertragsstaaten:
**AT BE CH DE DK ES FR GB GR IT LI LU NL SE**

(30) Priorität: **23.07.1990 DE 4023369**
**04.10.1990 DE 4031287**
**20.02.1991 DE 4105324**

(43) Veröffentlichungstag der Anmeldung:
**29.01.1992 Patentblatt 1992/05**

(73) Patentinhaber: **Dr. Karl Thomae GmbH**
**D-88397 Biberach (DE)**

(72) Erfinder:
- **Narr, Berthold Dr. Dipl-Chem**
  **W-7950 Biberach 1 (DE)**
- **Hauel, Norbert Dr. Dipl-Chem**
  **W-7950 Biberach 1 (DE)**
- **van Meel, Jacques Dr.**
  **W-7951 Mittelbiberach (DE)**
- **Wienen, Wolfgang Dr.**
  **W-7951 Äpfingen (DE)**
- **Entzeroth, Michael Dr.**
  **W-7951 Warthausen (DE)**
- **Ries, Uwe Dr.**
  **W-7950 Biberach 1 (DE)**

(56) Entgegenhaltungen:
**EP-A- 0 291 969**
**EP-A- 0 400 835**
**EP-A- 0 420 237**
**EP-A- 0 426 021**
**WO-A-91/00281**
**US-A- 4 880 804**

Bemerkungen:
Die Akte enthält technische Angaben, die nach dem Eingang der Anmeldung eingereicht wurden und die nicht in dieser Patentschrift enthalten sind.

EP 0 468 470 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

Printed by Jouve, 75001 PARIS (FR)

**Beschreibung**

In der US-A-4,880,804 werden u.a. 4'-[(2-Alkyl-benzimidazol-1-yl)-methyl]biphenyl-2-carbonsäuren und 4'-[(2-Alkyl-benzimidazol-1-yl)-methyl]-2-(1H-tetrazol-1-yl)-biphenyle beschrieben, welche im Benzimidazolring durch eine Alkanoylaminomethylgruppe substituiert sind und Angiotensin-II-Antagonisten darstellen.

Es wurde nun gefunden, daß die neuen Benzimidazole der allgemeinen Formel

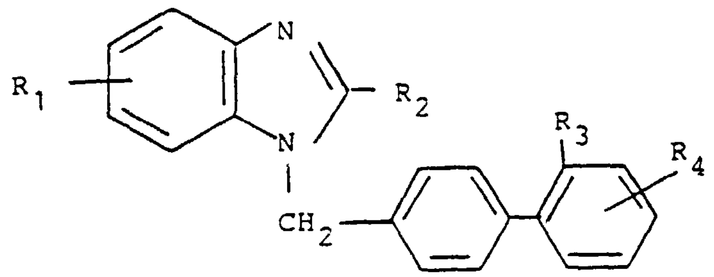

, (I)

deren 1-, 3-Isomerengemische sowie deren Salze, insbesondere für die pharmazeutische Anwendung deren physiologisch verträglichen Salze mit anorganischen oder organischen Säuren oder Basen, noch wertvollere Angiontensin-Antagonisten, insbesondere Angiontensin-II-Antagonisten, darstellen.

In der obigen allgemeinen Formel bedeutet

$R_1$ eine Tetrahydrobenzimidazolyl- oder Imidazopyridinylgruppe,

eine gegebenenfalls im Phenylkern durch ein Fluor-, Chlor- oder Bromatom, durch eine Alkylgruppe mit 1 bis 3 Kohlenstoffatomen, durch eine Alkoxygruppe mit 1 bis 3 Kohlenstoffatomen oder durch eine Trifluormethylgruppe substituierte Benzimidazolyl- oder Benzoxazolylgruppe, wobei die NH-Gruppe der vorstehend erwähnten Imidazolringe zusätzlich durch eine Alkylgruppe mit 1 bis 6 Kohlenstoffatomen oder durch eine Cycloalkylgruppe mit 3 bis 7 Kohlenstoffatomen substituiert sein kann,

eine durch eine Bicyclohexylcarbonyl- oder Biphenylcarbonylgruppe substituierte Aminogruppe, die am N-Atom jeweils zusätzlich durch eine Alkylgruppe mit 1 bis 3 Kohlenstoffatomen substituiert sein können,

eine durch eine Bicyclohexyl- oder Biphenylgruppe substituierte Aminocarbonylaminogruppe, die zusätzlich durch eine oder zwei Alkylgruppen mit jeweils 1 bis 3 Kohlenstoffatomen am N-Atom substituiert sein kann,

mit Ausnahme der 2-Oxo-3,4-tetramethylen-pyrrolidin-1-yl-gruppe eine gegebenenfalls durch eine oder zwei Alkylgruppen mit 1 bis 3 Kohlenstoffatomen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt ist,

eine gegebenenfalls durch eine Alkyl- oder Phenylalkylgruppe mit jeweils 1 bis 3 Kohlenstoffatomen im Alkylteil substituierte 3,4,5,6-Tetrahydro-2(1H)-pyrimidinongruppe,

eine gegebenenfalls durch eine Alkylgruppe mit 1 bis 3 Kohlenstoffatomen oder durch eine Phenylgruppe mono- oder disubstituierte Maleinsäureamido- oder Maleinsäureimidogruppe, wobei die Substituenten gleich oder verschieden sein können,

eine gegebenenfalls durch eine Alkylgruppe mit 1 bis 6 Kohlenstoffatomen oder durch eine Cycloalkylgruppe mit 3 bis 7 Kohlenstoffatomen substituierte Imidazolin- oder Imidazolgruppe,

eine gegebenenfalls durch eine Alkylgruppe mit 1 bis 3 Kohlenstoffatomen, durch eine Phenylalkylgruppe mit 1 bis 3 Kohlenstoffatomen im Alkylteil, durch eine Tetramethylen-, Pentamethylen- oder Hexamethylengruppe substituierte Imidazolidindiongruppe,

eine durch eine Alkylsulfonylgruppe mit 4 bis 6 Kohlenstoffatomen oder durch eine Phenylalkylsulfonylgruppe substituierte Alkylamino- oder Phenylalkylaminogruppe, in denen der Alkylteil jeweils 1 bis 3 Kohlenstoffatome

enthalten kann,

eine durch eine Naphthalinsulfonylgruppe substituierte Amino- oder Alkylaminogruppe, welche im Naphthalinring durch eine Dialkylaminogruppe, durch eine oder zwei Alkoxygruppen substituiert sein können, wobei der Alkylteil jeweils 1 bis 3 Kohlenstoffatome enthalten kann,

eine Pyridazin-3-on- oder Dihydro-pyridazin-3-on-gruppe, die in 2-Stellung durch eine gegebenenfalls durch eine Phenylgruppe substituierte Alkylgruppe mit 1 bis 3 Kohlenstoffatomen und zusätzlich im Kohlenstoffgerüst durch 1 oder 2 Alkylgruppen mit jeweils 1 bis 3 Kohlenstoffatomen substituiert sein kann,

eine durch 2 Alkylgruppen mit jeweils 1 bis 3 Kohlenstoffatomen substituierte Pyrrolidino-, Piperidino- oder Hexamethyleniminogruppe,

eine 7-Nitro-benzofurazan-4-yl-aminoalkanoylaminogruppe, in der der Alkanoylteil 2 oder 3 Kohlenstoffatome enthalten kann,

eine Heptamethylenimino-, 1H,3H-Chinazolin-2,4-dion-3-yl-, Pentamethylen-oxazolin-2-yl-, Benzofurancarbonylamino- oder 7-Nitro-benzofurazan-4-yl-amino-gruppe

oder, wenn $R_3$ eine Carboxygruppe und $R_2$ eine n-Butylgruppe darstellen, $R_1$ in 6-Stellung auch eine durch eine Phenylsulfonyl-, Cyclohexylmethylaminocarbonyl-, 2-Carboxycyclohexylmethylcarbonyl-, 2-tert.Butoxycarbonylcyclohexylmethylcarbonyl-, 2-Carboxy-3,4,5,6-tetrahydrobenzoyl-, N-Methylphenylaminocarbonyl- oder 3-Cyclohexylpropylgruppe substituierte Aminogruppe, eine durch eine Propylsulfonyl-, Phenylsulfonyl-, Methylphenylsulfonyl- oder Chlorphenylsulfonylgruppe substituierte Methylaminogruppe, eine durch eine Phenylsulfonyl- oder Methoxyphenylsulfonylgruppe substituierte n-Pentylaminogruppe, eine durch eine Methylphenylsulfonyl- oder Methoxyphenylsulfonylgruppe substituierte n-Propylaminogruppe, eine durch eine Benzoyl- oder Chlorphenylsulfonylgruppe substituierte Isopropylaminogruppe, eine N-Acetylcyclohexylmethylamino-, 3,4,5,6-Tetrahydrophthalimido-, Hexahydrohomophthalimido-, N-Methansulfonyl-2-phenylethylamino-, N-Chlorphenylsulfonyl-benzylamino-, Piperidino-, 4-Methyl-piperidino- oder Hexamethyleniminogruppe

oder, wenn $R_3$ eine Carboxygruppe und $R_2$ eine n-Butylgruppe darstellen, $R_1$ in 5- oder 6-Stellung auch eine 2-Oxo-1,2-dihydro-3,4-tetramethylen-pyrrolidin-1-yl-gruppe

oder, wenn $R_3$ eine Carboxygruppe und $R_2$ eine Methyl-, Ethyl-, n-Propyl-, n-Butyl- oder Methylmercaptogruppe darstellen, $R_1$ in 6-Stellung auch eine Pyrrolidinocarbonylaminogruppe

oder, wenn $R_3$ eine Tetrazolylgruppe und $R_2$ eine n-Butylgruppe darstellen, $R_1$ in 5- oder 6-Stellung auch eine durch eine Methylaminocarbonyl- oder Cyclohexylaminocarbonylgruppe substituierte n-Pentylaminogruppe oder in 6-Stellung eine 3,3-Dimethyl-glutarsäureimido- oder 4,4-Tetramethylen-glutarsäureimidogruppe,

oder, wenn $R_3$ eine Tetrazolylgruppe und $R_2$ eine Ethyl- oder n-Propylgruppe darstellen, $R_1$ in 6-Stellung auch eine N-Benzolsulfonyl-methylaminogruppe

oder, wenn $R_3$ eine tert.Butoxycarbonylgruppe und $R_2$ eine N-Butylgruppe darstellen, $R_1$ in 6-Stellung auch eine 2-Carboxycyclohexylmethylcarbonylamino- oder Pyrrolidinocarbonylaminogruppe,

$R_2$ ein Wasserstoffatom oder eine geradkettige oder verzweigte Alkylgruppe mit 1 bis 5 Kohlenstoffatomen, in welcher eine Methylengruppe durch ein Schwefelatom ersetzt sein kann,

$R_3$ eine Carboxy-, Cyano-, 1H-Tetrazolyl- oder 1-Triphenylmethyl-tetrazolylgruppe oder eine Alkoxycarbonylgruppe mit insgesamt 2 bis 5 Kohlenstoffatomen und

$R_4$ ein Wasserstoff-, Fluor-, Chlor- oder Bromatom

sowie die Verbindungen

4'[[2-n-Butyl-6-(piperidin-1-yl)-benzimidazol-1-yl]methyl]biphenyl-2-carbonsäure und

4'[[2-n-Butyl-6-(4-methylpiperidin-1-yl)-benzimidazol-1-yl]methyl]biphenyl-2-carbonsäure.

Gegenstand der vorliegenden Erfindung sind somit die neuen vorstehend erwähnten Benzimidazole, wobei die entsprechenden Cyano-, tert.Butoxycarbonyl- und Triphenylmethylverbindungen insbesondere wertvolle Zwischenprodukte darstellen. Ein weiterer Gegenstand der vorliegenden Erfindung sind somit neue Arzneimittel, welche eine der oben erwähnten pharmakologisch wirksamen Verbindungen der allgemeinen Formel I oder ein entsprechendes physiologisch verträgliches Salz enthalten und insbesondere zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen geeignet sind.

Für die bei der Definition der Reste $R_1$ und $R_2$ eingangs erwähnten Bedeutungen kommt beispielsweise

für $R_1$ die der Benzimidazol-2-yl-, 1-Methyl-benzimidazol-2-yl-, 1-Ethyl-benzimidazol-2-yl-, l-n-Propyl-benzimidazol-2-yl-, 1-Isopropyl-benzimidazol-2-yl-, l-n-Butyl-benzimidazol-2-yl-, 1-n-Pentyl-benzimidazol-2-yl-, l-n-Hexyl-benzimidazol-2-yl-, 1-Cyclopropyl-benzimidazol-2-yl-, 1-Cyclopentylbenzimidazol-2-yl-, 1-Cyclohexyl-benzimidazol-2-yl-, 1-Cycloheptyl-benzimidazol-2-yl-, 1,5-Dimethyl-benzimidazol-2-yl-, 1,6-Dimethyl-benzimidazol-2-yl-, 1-Methyl-5-methoxy-benzimidazol-2-yl-, 1-Methyl-5-fluor-benzimidazol-2-yl-, 1-Methyl-5-chlor-benzimidazol-2-yl-, 1-Methyl-5-brom-benzimidazol-2-yl-, 1-Methyl-5-trifluormethyl-benzimidazol-2-yl-, Tetrahydrobenzimidazol-2-yl-, 1-Methyl-tetrahydro-benzimidazol-2-yl-, l-Ethyl-tetrahydro-benzimidazol-2-yl-, l-n-Propyl-tetrahydrobenzimidazol-2-yl-, 1-Isopropyl-tetrahydro-benzimidazol-2-yl-, l-n-Butyl-tetrahydro-benzimidazol-2-yl-, l-n-Pentyltetrahydro-benzimidazol-2-yl-, l-n-Hexyl-tetrahydro-benzimidazol-2-yl-, 1-Cyclopropyl-tetrahydro-benzimidazol-2-yl-, l-Cyclopentyl-tetrahydro-benzimidazol-2-yl-, 1-Cyclohexyltetrahydro-benzimidazol-2-yl-, l-Cycloheptyl-tetrahydro-benzimidazol-2-yl-, Benzoxazol-2-yl-, 5-Methyl-benzoxazol-2-yl-, 5-Methoxy-benzoxazol-2-yl-, 5-Trifluormethyl-benzoxazol-2-yl-, 5-Fluor-benzoxazol-2-yl-, 5-Chlor-benzoxazol-2-yl-, 5-Brom-benzoxazol-2-yl, 4-Biphenylylcarbonylamino-, 4-Cyclohexylcarbonylamino-, N-Methyl-4-biphenylylcarbonylamino-, N-Ethyl-4-cyclohexylcarbonylamino-, N-n-Propyl-4-biphenylylcarbonylamino-, N-Isopropyl-4-cyclohexylcarbonylamino-, 2-Hydroxy-cyclopentylamino-, 2-Hydroxy-cyclohexylamino-, 2-Hydroxy-cycloheptylamino-, 3-Hydroxy-cyclopentylamino-, 3-Hydroxy-cyclohexylamino-, 3-Hydroxy-cycloheptylamino-, 4-Hydroxy-cyclohexylamino-, 4-Hydroxy-cycloheptylamino-, N-Methyl-2-hydroxy-cyclopentylamino-, N-Ethyl-2-hydroxy-cyclohexylamino-, N-Isopropyl-2-hydroxy-cycloheptylamino-, N-Methyl-3-hydroxy-cyclopentylamino-, N-Ethyl-3-hydroxy-cyclohexylamino-, N-n-Propyl-3-hydroxy-cycloheptylamino-, N-Methyl-4-hydroxy-cyclohexylamino-, N-Ethyl-4-hydroxy-cycloheptylamino-, 4-Biphenylylaminocarbonylamino-, 4-Bicyclohexylaminocarbonylamino-, N-(4-Biphenylylaminocarbonyl)-methylamino-, N-(4-Bicyclohexylaminocarbonyl)-methylamino-, N-(Methyl-4-biphenylylaminocarbonyl)-methylamino-, N-(Methyl-4-bicyclohexylaminocarbonyl)-methylamino-, N-(4-Biphenylylaminocarbonyl)-ethylamino-, N-(4-Bicyclohexylaminocarbonyl)-isopropylamino-, N-(Ethyl-4-biphenylylaminocarbonyl)-methylamino-, N-(Methyl-4-bicyclohexylaminocarbonyl)-ethylamino-, Pyrrolidin-2-on-1-yl-, Piperidin-2-on-1-yl-, Hexamethylenimin-2-on-1-yl-, Propansultam-1-yl-, Butansultam-1-yl-, Pentansultam-1-yl-, 3,4,5,6-Tetrahydro-2(1H)-pyrimidon-1-yl-, 3-Methyl-3,4,5,6-tetrahydro-2(1H)-pyrimidon-1-yl-, 3-Ethyl-3,4,5,6-tetrahydro-2(1H)-pyrimidon-1-yl-, 3-n-Propyl-3,4,5,6-tetrahydro-2(1H)-pyrimidon-1-yl-, 3-Isopropyl-3,4,5,6-tetrahydro-2(1H)-pyrimidon-1-yl-, 3-Benzyl-3,4,5,6-tetrahydro-2(1H)-pyrimidon-1-yl-, 3-(2-Phenylethyl)-3,4,5,6-tetrahydro-2(1H)-pyrimidon-1-yl-, 3-(3-Phenylpropyl)-3,4,5,6-tetrahydro-2 (1H)-pyrimidon-1-yl-, 4-Hydroxybutylamino-, 5-Hydroxypentylamino-, 6-Hydroxyhexylamino-, Maleinsäureimido-, 2-Methyl-maleinsäureimido-, 2-Phenyl-maleinsäureimido-, 2,3-Dimethyl-maleinsäureimido-, 2,3-Diphenyl-maleinsäureimido-, 2-Methyl-maleinsäureamido-, 3-Methyl-maleinsäureamido-, 2,3-Dimethyl-maleinsäureamido-, 2-Phenyl-maleinsäureamido-, 3-Phenyl-maleinsäureamido-, 2,3-Diphenyl-maleinsäureamido-, 3-Methyl-2-phenyl-maleinsäureamido-, 2-Methyl-3-phenyl-maleinsäureamido-, Imidazolin-2-yl-, 1-Methyl-imidazolin-2-yl-, 1-Ethyl-imidazolin-2-yl-, 1-Propyl-imidazolin-2-yl-, Imidazolidin-2,4-dion-3-yl-, 5-Methyl-imidazolidin-2,4-dion-3-yl-, 5-Ethyl-imidazolidin-2,4-dion-3-yl-, 5-n-Propylimidazolidin-2,4-dion-3-yl-, 5-Benzyl-imidazolidin-2,4-dion-3-yl-, 5-(2-Phenylethyl)-imidazolidin-2,4-dion-3-yl-, 5-(3-Phenylpropyl)-imidazolidin-2,4-dion-3-yl-, 5,5-Tetramethylen-imidazolidin-2,4-dion-3-yl-, 5,5-Pentamethylen-imidazolidin-2,4-dion-3-yl-, 5,5-Hexamethylen-imidazolidin-2,4-dion-3-yl-, 5,5-Dimethyl-imidazolidin-2,4-dion-3-yl-, 5,5-Diethylimidazolidin-2,4-dion-3-yl-, N-n-Butansulfonyl-methylamino-, N-n-Pentansulfonyl-methylamino-, N-n-Hexansulfonyl-methylamino-, N-Phenylmethansulfonyl-methylamino-, N-(2-Phenylethansulfonyl)-methylamino-, N-(3-Phenylpropansulfonyl)-methylamino-, N-n-Butansulfonyl-ethylamino-, N-n-Pentansulfonyl-isopropylamino-, N-n-Hexansulfonyl-ethylamino-, N-Phenylmethansulfonyl-ethylamino-, N-(2-Phenylethansulfonyl)-n-propylamino-, N-(3-Phenylpropansulfonyl)-ethylamino-, Naphthalin-1-sulfonylamino-, Naphthalin-2-sulfonylamino-, 5-Dimethylamino-napthalin-1-sulfonylamino-, N-(Naphthalin-1-sulfonyl)-methylamino-, N-(Naphthalin-2-sulfonyl)-ethylamino-, N-(5-Dimethylamino-napthalin-1-sulfonyl)-methylamino-, N-(5-Methoxy-naphthalin-1-sulfonyl)-methylamino-, N-(5,6-Dimethoxy-naphthalin-2-sulfonyl)-ethylamino-, 4,5-Dihydro-2H-pyridazin-3-on-6-yl-, 2-Methyl-4,5-dihydro-2H-pyridazin-3-on-6-yl-, 2-Ethyl-4,5-dihydro-2H-pyridazin-3-on-6-yl-, 2-n-Propyl-4,5-dihydro-2H-pyridazin-3-on-6-yl-, 2-Isopropyl-4,5-dihydro-2H-pyridazin-3-on-6-yl-, 2-Benzyl-4,5-dihydro-2H-pyridazin-3-on-6-yl-, 2-(2-Phenylethyl)-4,5-dihydro-2H-pyridazin-3-on-6-yl-, 2-(3-Phenylpropyl)-4,5-dihydro-2H-pyridazin-3-on-6-yl-, 4-Methyl-4,5-dihydro-2H-pyridazin-3-on-6-yl-, 5-Methyl-4,5-dihydro-2H-pyridazin-3-on-6-yl-, 4,4-Dimethyl-4,5-dihydro-2H-pyridazin-3-on-6-yl-, 5,5-Dimethyl-4,5-dihydro-2H-pyridazin-3-on-6-yl-, 4,5-Dimethyl-4,5-dihydro-2H-pyridazin-3-on-6-yl-, 2,4-Dimethyl-4,5-dihydro-2H-pyridazin-3-on-6-yl-, 2,5-Dimethyl-4,5-dihydro-2H-pyridazin-3-on-6-yl-, 2,4,5-Trimethyl-4,5-dihydro-2H-pyridazin-3-on-6-yl-, 2,4,4-Trimethyl-4,5-dihydro-2H-pyridazin-3-on-6-yl-, 2,5,5-Trimethyl-4,5-dihydro-2H-pyridazin-3-on-6-yl-, 2H-Pyridazin-3-on-6-yl-, 2-Methyl-2H-pyridazin-3-on-6-yl-, 2-Ethyl-2H-pyridazin-3-on-6-yl-, 2-n-Propyl-2H-pyridazin-3-on-6-yl-, 2-Isopropyl-2H-pyridazin-3-on-6-yl-, 2-Benzyl-2H-pyridazin-3-on-6-yl-, 2-(2-Phenylethyl)-2H-pyridazin-3-on-6-yl-, 2-(3-Phenylpropyl)-2H-pyridazin-3-on-6-yl-, 4-Methyl-2H-pyridazin-3-on-6-yl-, 5-Methyl-2H-pyridazin-3-on-6-yl-, 4,5-Dimethyl-2H-pyridazin-3-on-6-yl-, 2,4-Dimethyl-2H-pyridazin-3-on-6-yl-, 2,5-Dimethyl-2H-pyridazin-3-on-6-yl-, 2,4,5-Trimethyl-2H-pyridazin-3-on-6-yl-, 3,3-Dimethyl-pyrrolidino-, 3,4-Dimethyl-pyrrolidino-, 3,3-Dimethyl-piperidino-, 3,4-Dimethylpiperidino-, 4,4-Dimethyl-piperidino-, 3,3-Dimethyl-hexamethylenimino-, 3,4-Dimethyl-hexamethylenimino-, 4,4-Dimethylhexamethylenimino-, 3,5-Dimethyl-hexamethylenimino-, Phenylsulfonylamino-, Cyclohexylmethylaminocarbonylamino-, 2-Methylamino-benzoylamino-, 2-Carboxy-cyclohexylmethylcarbonylamino-, 2-tert.Butoxycarbonyl-cyclohexylmethylcarbonylamino-, 2-Carboxy-3,4,5,6-tetrahydrobenzoylamino-, 3-Cyclohexylpropylamino-, N-Propylsulfonyl-methylamino-, N-Phenylsulfonyl-methylamino-, N-(4-Methylphenylsulfonyl)-methylamino-, N-(4-Chlorphenylsulfonyl)-methylamino-, N-Phenylsulfonyl-n-pentylamino-, N-(4-Methoxyphenylsulfonyl)-n-pentylamino-, N-(4-Methylphenylsulfonyl)-n-propylamino-, N-(4-Methoxyphenylsulfonyl)-n-propylamino-, N-Benzoyl-isopropylamino-, N-(4-Chlorphenylsulfonyl)-isopropylamino-, N-Acetyl-cyclohexylmethylamino-, 3,4,5,6-Tetrahydrophthalimido-, Hexahydrophthalimido-, N-Methansulfonyl-2-phenylethylamino-, N-Chlorphenylsulfonyl-benzylamino-, Piperidino-, 4-Methyl-piperidino-, Hexamethylenimino-, Pyrrolidinocarbonylamino-, N-Methylaminocarbonyl-n-pentylamino-, N-Cyclohexylaminocarbonyl-n-pentylamino-, 3,3-Dimethyl-glutarsäureimido-, 4,4-Tetramethylen-glutarsäureimido-, 2-Carboxy-cyclohexylmethylcarbonylamino-, 1-n-Butyl-imidazolin-2-yl-, 1-n-Pentyl-imidazolin-2-yl-, 1-n-Hexyl-imidazolin-2-yl-, 1-Cyclopropyl-imidazolin-2-yl-, 1-Cyclobutyl-imidazolin-2-yl-, 1-Cyclo- hexyl-imidazolin-2-yl-, 1-Cycloheptyl-imidazolin-2-yl-, Imidazol-2-yl-, 1-Methyl-imidazol-2-yl-, 1-Ethyl-imidazol-2-yl-, 1-Propyl-imidazol-2-yl-, 1-n-Butyl-imidazol-2-yl-, 1-n-Pentyl-imidazol-2-yl-, 1-n-Hexyl-imidazol-2-yl-, 1-Cyclopropyl-imidazol-2-yl-, 1-Cyclobutyl-imidazol-2-yl-, 1-Cyclohexyl-imidazol-2-yl- oder 1-Cycloheptylimidazol-2-yl-gruppe und

für $R_2$ die des Wasserstoffatoms, der Methyl-, Ethyl-, n-Propyl-, Isopropyl-, n-Butyl-, Isobutyl-, tert.Butyl-, n-Pentyl-, 1-Methylpropyl-, 1-Methylbutyl-, 2-Methylbutyl-, 3-Methylbutyl-, 1-Ethylpropyl-, 1,1-Diethylethyl-, Methylmercaptomethyl-, 2-Methylmercapto-ethyl-, 3-Methylmercaptopropyl- oder 4-Methylmercaptobutylgruppe in Betracht.

Bevorzugte Verbindungen der obigen allgemeinen Formel sind jedoch diejenigen, in denen

$R_1$ eine Tetrahydrobenzimidazolyl- oder Imidazopyridinylgruppe,

eine gegebenenfalls im Phenylkern durch ein Fluor-, Chlor- oder Bromatom, durch eine Methyl-, Methoxy- oder Trifluormethylgruppe substituierte Benzimidazolylgruppe, wobei die NH-Gruppe der vorstehend erwähnten Imidazolringe zusätzlich durch eine Alkylgruppe mit 1 bis 6 Kohlenstoffatomen oder durch eine Cycloalkylgruppe mit 3 bis 6 Kohlenstoffatomen substituiert sein kann,

eine gegebenenfalls durch eine Methylgruppe substituierte Benzoxazol-2-yl-gruppe,

eine durch eine Bicyclohexylcarbonyl-, Biphenylcarbonyl- oder Benzofuryl-2-carbonylgruppe substituierte Aminogruppe,

eine in 3-Stellung durch eine Bicyclohexyl- oder Biphenylgruppe substituierte Aminocarbonylaminogruppe,

eine gegebenenfalls durch eine oder zwei Methylgruppen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt ist,

eine gegebenenfalls durch eine Methyl- oder Benzylgruppe substituierte 3,4,5,6-Tetrahydro-2(1H)-pyrimidinongruppe,

eine gegebenenfalls durch eine Methylgruppe oder durch eine Phenylgruppe mono- oder disubstituierte Maleinsäureamido- oder Maleinsäureimidogruppe, wobei die Substituenten gleich oder verschieden sein können,

eine in 1-Stellung durch eine Alkylgruppe mit 1 bis 6 Kohlenstoffatomen oder durch eine Cycloalkylgruppe mit 3 bis 7 Kohlenstoffatomen substituierte Imidazolin-2-yl- oder Imidazol-2-yl-gruppe,

eine gegebenenfalls durch eine Methyl-, Benzyl-, Tetramethylen- oder Pentamethylengruppe substituierte Imidazolidindiongruppe,

eine durch eine Butansulfonylgruppe oder durch eine Phenylmethansulfonylgruppe substituierte Methylamino- oder Benzylaminogruppe,

eine durch eine Naphthalinsulfonylgruppe substituierte Amino- oder Methylaminogruppe, welche im Naphthalinring durch eine Dimethylaminogruppe oder durch 2 Methoxygruppen substituiert sein können,

eine gegebenenfalls durch eine Methyl- oder Benzylgruppe substituierte Pyridazin-3-on- oder Dihydro-pyridazin-3-on-gruppe,

eine durch zwei Methylgruppen substituierte Pyrrolidino-, Piperidino- oder Hexamethyleniminogruppe,

eine Heptamethylenimino-, 1H,3H-Chinazolin-2,4-dion-3-yl-, 4,5-Pentamethylen-oxazolin-2-yl-, 7-Nitro-benzofurazan-4-yl-amino- oder 7-Nitro-benzofurazan-4-yl-aminopropionylaminogruppe

oder, wenn $R_3$ eine Carboxygruppe und $R_2$ eine n-Butylgruppe darstellen, $R_1$ in 6-Stellung auch eine durch eine Phenylsulfonyl-, Cyclohexylmethylaminocarbonyl-, 2-Carboxycyclohexylmethylcarbonyl-, 2-tert.Butoxycarbonylcyclohexylmethylcarbonyl-, 2-Carboxy-3,4,5,6-tetrahydrobenzoyl-, N-Methyl-phenylaminocarbonyl- oder 3-Cyclohexylpropylgruppe substituierte Aminogruppe, eine durch eine Propylsulfonyl-, Phenylsulfonyl-, 4-Methylphenylsulfonyl- oder 4-Chlorphenylsulfonylgruppe substituierte Methylaminogruppe, eine durch eine Phenylsulfonyl- oder 4-Methoxyphenylsulfonylgruppe substituierte n-Pentylaminogruppe, eine durch eine 4-Methylphenylsulfonyl- oder 4-Methoxyphenylsulfonylgruppe substituierte n-Propylaminogruppe, eine durch eine Benzoyl- oder 4-Chlorphenylsulfonylgruppe substituierte Isopropylaminogruppe, eine N-Acetyl-cyclohexylmethylamino-, 3,4,5,6-Tetrahydrophthalimido-, Hexahydrohomophthalimido-, N-Methansulfonyl-2-phenylethylamino-, N-(4-Chlorphenylsulfonyl)-benzylamino-, Piperidino-, 4-Methyl-piperidino- oder Hexamethyleniminogruppe

oder, wenn $R_3$ eine Carboxygruppe und $R_2$ eine n-Butylgruppe darstellen, $R_1$ in 5- oder 6-Stellung auch eine 2-Oxo-1,2-dihydro-3,4-tetramethylen-pyrrolidin-1-yl-gruppe

oder, wenn $R_3$ eine Carboxygruppe und $R_2$ eine Methyl-, Ethyl-, n-Propyl-, n-Butyl- oder Methylmercaptogruppe darstellen, $R_1$ in 6-Stellung auch eine Pyrrolidinocarbonylaminogruppe

oder, wenn $R_3$ eine Tetrazolylgruppe und $R_2$ eine n-Butylgruppe darstellen, $R_1$ in 5- oder 6-Stellung auch eine durch eine Methylaminocarbonyl- oder Cyclohexylaminocarbonylgruppe substituierte n-Pentylaminogruppe oder in 6-Stellung eine 3,3-Dimethyl-glutarsäureimido- oder 4,4-Tetramethylen-glutarsäureimidogruppe,

oder, wenn $R_3$ eine Tetrazolylgruppe und $R_2$ eine Ethyl- oder n-Propylgruppe darstellen, $R_1$ in 6-Stellung auch eine N-Benzolsulfonyl-methylaminogruppe

oder, wenn $R_3$ eine tert.Butoxycarbonylgruppe und $R_2$ eine N-Butylgruppe darstellen, $R_1$ in 6-Stellung auch eine 2-Carboxy-cyclohexylmethylcarbonylamino- oder Pyrrolidinocarbonylaminogruppe,

$R_2$ ein Wasserstoffatom oder eine geradkettige oder verzweigte Alkylgruppe mit 1 bis 4 Kohlenstoffatomen, in welcher eine Methylengruppe durch ein Schwefelatom ersetzt sein kann,

$R_3$ eine Carboxy-, Cyano-, 1H-Tetrazolyl- oder 1-Triphenylmethyl-tetrazolylgruppe oder eine Alkoxycarbonylgruppe mit insgesamt 2 bis 5 Kohlenstoffatomen und

$R_4$ ein Wasserstoff-, Fluor-, Chlor- oder Bromatom bedeuten,

deren 1-, 3-Isomerengemische sowie deren physiologisch verträglichen Salze mit anorganischen oder organischen Säuren oder Basen.

Besonders bevorzugte Verbindungen der obigen allgemeinen Formel I sind jedoch diejenigen, in denen

$R_1$ in 6-Stellung eine 1-Methylbenzimidazol-2-yl-, 3,4,5,6-Tetrahydro-phthalimino-, 2,3-Diphenyl-maleinsäureimido-, 2,3-Dimethyl-maleinsäureimido-, N-Phenylmethansulfonyl-methylamino-, 2-Oxo-pyrrolidin-1-yl-, 2-Oxo-piperidin-1-yl-, 2-Oxo-hexamethylenimino-, 2-Oxo-3,4-tetramethylen-pyrrolin-pentylamino-, Propansultam-1-yl- oder Butansultam-1-yl-gruppe,

$R_2$ die Methyl-, Ethyl-, n-Propyl- oder n-Butylgruppe,

$R_3$ eine Carboxy- oder 1H-Tetrazolylgruppe und

$R_4$ ein Wasserstoffatom bedeuten, und deren physiologisch verträglichen Salze mit organischen oder anorganischen Säuren oder Basen.

Erfindungsgemäß erhält man die Verbindungen nach folgenden Verfahren:

a) Cyclisierung einer Verbindung der allgemeinen Formel

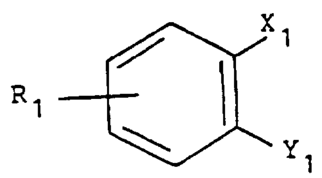

,(II)

in der

$R_1$ wie eingangs definiert ist,
einer der Reste $X_1$ oder $Y_1$ eine Gruppe der allgemeinen Formel

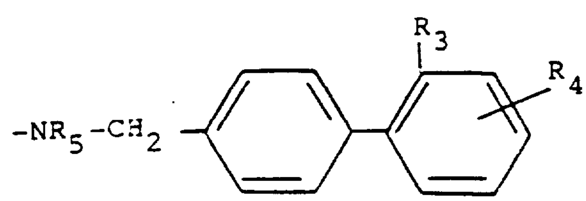

und der andere der Reste $X_1$ oder $Y_1$ eine Gruppe der allgemeinen Formel

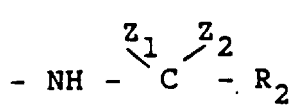

darstellen, wobei
$R_2$ bis $R_4$ wie eingangs definiert sind,
$R_5$ ein Wasserstoffatom oder eine $R_2CO$-Gruppe, wobei $R_2$ wie vorstehend erwähnt definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder
$Z_1$ und $Z_2$, zusammen ein Sauerstoff- oder Schwefelatom,

eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, wobei jedoch einer der Reste $X_1$ oder $Y_1$ eine Gruppe der allgemeinen Formel

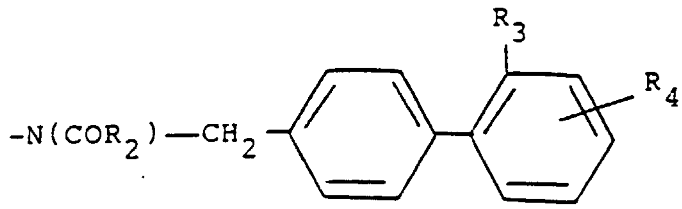

oder

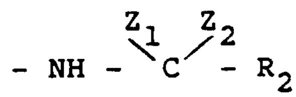

darstellen muß.

Die Cyclisierung wird zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid, Tetralin oder in einem Überschuß des zur Herstellung der Verbindung der allgemeinen Formel II verwendeten Acylierungsmittel, z.B. in dem entsprechenden Nitril, Anhydrid, Säurehalogenid, Ester oder Amid, beispielsweise bei Temperaturen zwischen 0 und 250°C, vorzugsweise jedoch bei der Siedetemperatur des Reaktionsgemisches, gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat durchgeführt. Die Cyclisierung kann jedoch auch ohne Lösungsmittel und/ oder Kondensationsmittel durchgeführt werden.

Besonders vorteilhaft wird die Umsetzung jedoch in der Weise durchgeführt, daß eine Verbindung der allgemeinen Formel II im Reaktionsgemisch durch Reduktion einer entsprechenden o-Nitro-aminoverbindung gegebenenfalls in Gegenwart einer Carbonsäure der allgemeinen Formel $R_2COOH$ oder durch Acylierung einer entsprechenden o-Diaminoverbindung hergestellt wird. Bei Abbruch der Reduktion der Nitrogruppe auf der Hydroxylaminstufe erhält man bei der anschließenden Cyclisierung das N-Oxid einer Verbindung der allgemeinen Formel I. Das so erhaltene N-Oxid wird anschließend mittels Reduktion in eine entsprechende Verbindung der allgemeinen Formel I übergeführt.

Die anschließende Reduktion des erhaltenen N-Oxids der Formel I wird vorzugsweise in einem Lösungsmittel wie Wasser, Wasser/Äthanol, Methanol, Eisessig, Essigsäureäthylester oder Dimethylformamid mit Wasserstoff in Gegenwart eines Hydrierungskatalysators wie Raney-Nickel, Platin oder Palladium/ Kohle, mit Metallen wie Eisen, Zinn oder Zink in Gegenwart einer Säure wie Essigsäure, Salzsäure oder Schwefelsäure, mit Salzen wie Eisen(II)sulfat, Zinn(II)chlorid oder Natriumdithionit, oder mit Hydrazin in Gegenwart von Raney-Nickel bei Temperaturen zwischen 0 und 50°C, vorzugsweise jedoch bei Raumtemperatur durchgeführt.

b) Umsetzung eines Benzimidazols der allgemeinen Formel

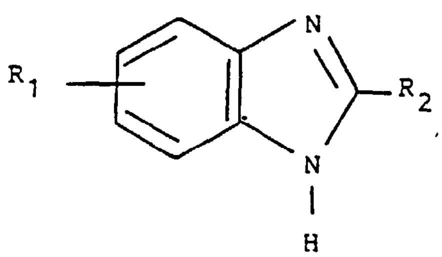
,(III)

in der

$R_1$ und $R_2$ wie eingangs definiert sind, mit einer Biphenylverbindung der allgemeinen Formel

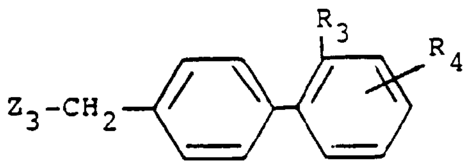

, (IV)

in der

$R_3$ und $R_4$ wie eingangs definiert sind und

$Z_3$ eine nukleophile Austrittsgruppe wie ein Halogenatom, z.B. ein Chlor-, Brom- oder Jodatom, oder eine substituierte Sulfonyloxygruppe, z.B. eine Methansulfonyloxy-, Phenylsulfonyloxy- oder p-Toluolsulfonyloxygruppe, darstellt.

Die Umsetzung wird zweckmäßigerweise in einem Lösungsmittel oder Lösungsmittelgemisch wie Methylenchlorid, Diethylether, Tetrahydrofuran, Dioxan, Dimethylsulfoxid, Dimethylformamid oder Benzol gegebenenfalls in Gegenwart eines säurebindenden Mittels, wie Natriumcarbonat, Kaliumcarbonat, Natriumhydroxid, Kalium-tert. butylat, Triethylamin oder Pyridin, wobei die beiden letzteren gleichzeitig auch als Lösungsmittel verwendet werden können, vorzugsweise bei Temperaturen zwischen 0 und 100°C, z.B. bei Temperaturen zwischen Raumtemperatur und 50°C, durchgeführt.

Bei der Umsetzung erhält man vorzugsweise ein Gemisch der 1-und 3-Isomeren, welches gewünschtenfalls anschließend, vorzugsweise chromatographisch unter Verwendung eines Trägers wie Kieselgel oder Aluminiumoxid, in das entsprechende 1- und 3-Isomere aufgetrennt wird.

c) Zur Herstellung einer Verbindung der allgemeinen Formel I, in der $R_3$ eine Carboxygruppe darstellt: Überführung einer Verbindung der allgemeinen Formel

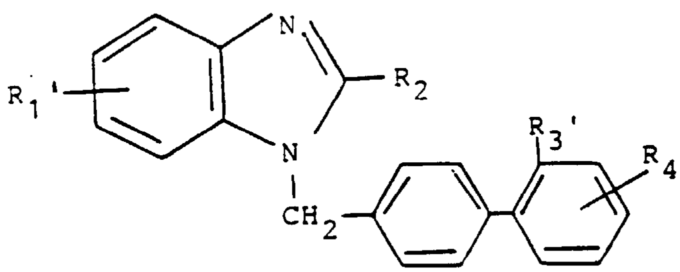

, (V)

in der

$R_2$ und $R_4$ wie eingangs definiert sind,

$R_1$' die für $R_1$ eingangs erwähnten Bedeutungen besitzt und eine 3-Alkoxycarbonylpropionyl- oder 3-Alkoxycarbonyl-2-methyl-propionylgruppe, in der der Alkoxyteil jeweils 1 bis 3 Kohlenstoffatome enthalten kann, und

$R_3$' eine mittels Hydrolyse, Thermolyse oder Hydrogenolyse in eine Carboxygruppe überführbare Gruppe darstellen.

Beispielsweise können funktionelle Derivate der Carboxygruppe wie deren unsubstituierte oder substituierte Amide, Ester, Thiolester, Orthoester, Iminoäther, Amidine oder Anhydride, die Nitrilgruppe oder die Tetrazolylgruppe mittels Hydrolyse in eine Carboxygruppe,

Ester mit tertiären Alkoholen, z.B. der tert. Butylester, mittels Thermolyse in eine Carboxygruppe und

Ester mit Aralkanolen, z.B. der Benzylester, mittels Hydrogenolyse in eine Carboxygruppe übergeführt werden.

Die Hydrolyse wird zweckmäßigerweise entweder in Gegenwart einer Säure wie Salzsäure, Schwefelsäure, Phosphorsäure, Trichloressigsäure oder Trifluoressigsäure in Gegenwart einer Base wie Natriumhydroxid oder Kaliumhydroxid in einem geeigneten Lösungsmittel wie Wasser, Wasser/Methanol, Äthanol, Wasser/Äthanol, Wasser/Isopropanol oder Wasser/Dioxan bei Temperaturen zwischen -10°C und 120°C, z.B. bei Temperaturen zwischen Raumtemperatur und der Siedetemperatur des Reaktionsgemisches, durchgeführt. Bei der Hydrolyse in Gegenwart einer organischen Säuren wie Trichloressigsäure oder Trifluoressigsäure können gegebenenfalls vorhandene alkoholische Hydroxygruppen gleichzeitig in eine entsprechende Acyloxygruppe wie die Trifluoracetoxygruppe übergeführt werden.

Bedeutet $R_3$' in einer Verbindung der allgemeinen Formel V eine Cyano- oder Aminocarbonylgruppe, so können diese Gruppen auch mit einem Nitrit, z.B. Natriumnitrit, in Gegenwart einer Säure wie Schwefelsäure, wobei diese zweckmäßigerweise gleichzeitig als Lösungsmittel verwendet wird, bei Temperaturen zwischen 0 und 50°C in die Carboxygruppe übergeführt werden.

Bedeutet $R_3$' in einer Verbindung der allgemeinen Formel V beispielsweise die tert. Butyloxycarbonylgruppe, so kann die tert. Butylgruppe auch thermisch gegebenenfalls in einem inerten Lösungsmittel wie Methylenchlorid, Chloroform, Benzol, Toluol, Tetrahydrofuran oder Dioxan und vorzugsweise in Gegenwart einer katalytischen Menge einer Säure wie p-Toluolsulfonsäure, Schwefelsäure, Phosphorsäure oder Polyphosphorsäure vorzugsweise bei der Siedetemperatur des verwendeten Lösungsmittels, z.B. bei Temperaturen zwischen 40°C und 100°C, abgespalten werden.

Bedeutet $R_3$' in einer Verbindung der allgemeinen Formel V beispielsweise die Benzyloxycarbonylgruppe, so kann die Benzylgruppe auch hydrogenolytisch in Gegenwart eines Hydrierungskatalysators wie Palladium/Kohle in einem geeigneten Lösungsmittel wie Methanol, Äthanol, Äthanol/Wasser, Eisessig, Essigsäureäthylester, Dioxan oder Dimethylformamid vorzugsweise bei Temperaturen zwischen 0 und 50°C, z.B. bei Raumtemperatur, und einem Wasserstoffdruck von 1 bis 5 bar abgespalten werden. Bei der Hydrogenolyse können gleichzeitig andere Reste, z.B. eine Nitrogruppe zur Aminogruppe, eine Benzyloxygruppe zur Hydroxygruppe, eine Vinylidengruppe zur entsprechenden Alkylidengruppe oder eine Zimtsäuregruppe zur entsprechenden Phenyl-propionsäuregruppe, mitreduziert oder durch Wasserstoffatome, z.B. ein Halogenatom durch ein Wasserstoffatom, ersetzt werden.

Bedeutet $R_1$ in einer Verbindunge der allgemeinen Formel V einen der eingangs erwähnten hydrolysierbaren Reste, so kann dieser während der Umsetzung in eine entsprechende Carboxy- oder Aminoverbindung übergeführt werden.

d) Zur Herstellung einer Verbindung der allgemeinen Formel I, in der $R_3$ eine 1H-Tetrazolylgruppe darstellt: Abspaltung eines Schutzrestes von einer Verbindung der allgemeinen Formel

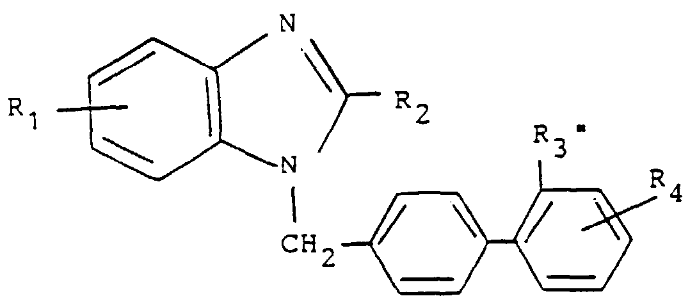

, (VI)

in der

$R_1$, $R_2$ und $R_4$ wie eingangs definiert sind und

$R_3$" eine in 1- oder 3-Stellung durch einen Schutzrest geschützte 1H-Tetrazolylgruppe darstellt.

Als Schutzrest kommt beispielsweise die Triphenylmethyl-, Tributylzinn- oder Triphenylzinngruppe in Betracht.

Die Abspaltung eines verwendeten Schutzrestes erfolgt vorzugsweise in Gegenwart eines Halogenwasserstoffes, vorzugsweise in Gegenwart von Chlorwasserstoff, in Gegenwart einer Base wie Natriumhydroxid oder alkoholischem Ammoniak in einem geeigneten Lösungsmittel wie Methylenchlorid, Methanol, Methanol/Ammoniak, Ethanol oder Isopropanol bei Temperaturen zwischen 0 und 100°C, vorzugsweise jedoch bei Raumtemperatur, oder auch, falls die Umsetzung in Gegenwart von alkoholischem Ammoniak durchgeführt wird, bei erhöhten Temperaturen, z.B. bei Temperaturen zwischen 100 und 150°C, vorzugsweise bei Temperaturen zwischen 120 und 140°C.

e) Zur Herstellung einer Verbindung der allgemeinen Formel I, in der $R_3$ eine 1H-Tetrazolylgruppe darstellt: Umsetzung einer Verbindung der allgemeinen Formel

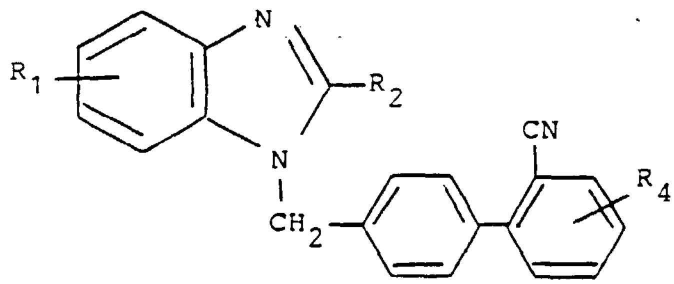

, (VII)

in der
$R_1$, $R_2$ und $R_4$ wie eingangs definiert sind, mit Stickstoffwasserstoffsäure oder deren Salzen.

Die Umsetzung wird vorzugsweise in einem Lösungsmittel wie Benzol, Toluol oder Dimethylformamid bei Temperaturen zwischen 80 und 150°C, vorzugsweise bei 125°C, durchgeführt. Hierbei wird zweckmäßigerweise entweder die Stickstoffwasserstoffsäure während der Umsetzung aus einem Alkaliazid, z.B. aus Natriumazid, in Gegenwart einer schwachen Säure wie Ammoniumchlorid freigesetzt oder das im Reaktionsgemisch bei der Umsetzung mit einem Salz der Stickstoffwassersäure, vorzugsweise mit Aluminiumazid oder Tributylzinnazid, welche außerdem zweckmäßigerweise im Reaktionsgemisch durch Umsetzung von Aluminiumchlorid oder Tributylzinnchlorid mit einem Alkaliazid wie Natriumazid hergestellt werden, erhaltene Tetrazolidsalz anschließend durch Ansäuern mit einer verdünnten Säure wie 2N Salzsäure oder 2N Schwefelsäure freigesetzt.

f) Zur Herstellung von Verbindungen der allgemeinen Formel I, in der $R_1$ eine Pentamethylen-oxazolin-2-yl-gruppe darstellt:
Umsetzung einer Verbindung der allgemeinen Formel

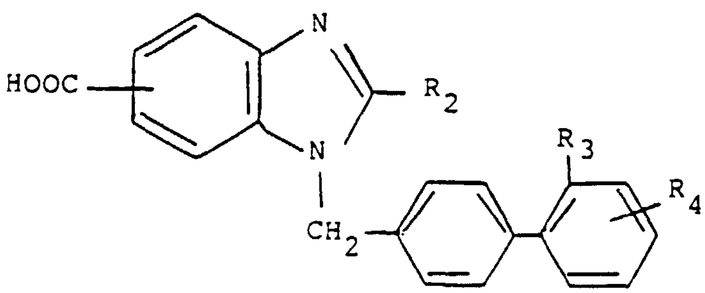

, (VIII)

in der
$R_2$ bis $R_4$ wie eingangs definiert sind, mit 1-Aminomethylcyclohexanol in Gegenwart eines die Säure aktivierenden Mittels.

Die Umsetzung wird vorzugsweise in einem Lösungsmittel wie Tetrahydrofuran oder Dioxan in Gegenwart eines die Säure aktivierenden Mittels wie Carbonylimidazol bei Temperaturen zwischen 0 und 50°C, vorzugsweise bei Raumtemperatur, durchgeführt.

g) Zur Herstellung einer Verbindung der allgemeinen Formel I, in der $R_1$ eine 2-Oxo-3,4-tetramethylen-pyrrolidin-1-yl-gruppe darstellt:
Hydrierung einer Verbindung der allgemeinen Formel

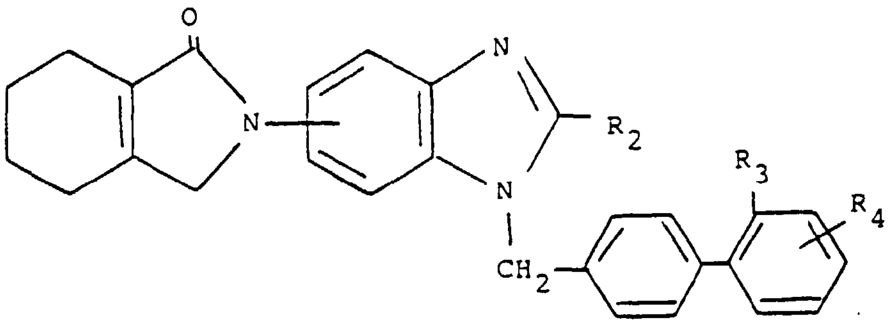

, (IX)

in der

$R_2$, $R_3$ und $R_4$ wie eingangs definiert sind.

Die katalytische Hydrierung wird mit Wasserstoff in Gegenwart eines Katalysators wie Palladium/Kohle in einem Lösungsmittel wie Methanol, Ethanol, Essigsäureethylester oder Eisessig bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar, durchgeführt.

h) Zur Herstellung von Verbindungen der allgemeinen Formel I, in der $R_1$ eine durch eine Bicyclohexylcarbonyl- oder Biphenylcarbonylgruppe substituierte Aminogruppe, die am N-Atom jeweils zusätzlich durch eine Alkylgruppe mit 1 bis 3 Kohlenstoffatomen substituiert sein können, eine durch eine Bicyclohexyl- oder Biphenylgruppe substituierte Aminocarbonylaminogruppe, die zusätzlich durch eine oder zwei Alkylgruppen mit jeweils 1 bis 3 Kohlenstoffatomen am N-Atom substituiert sein kann, eine gegebenenfalls durch eine Alkylgruppe mit 1 bis 3 Kohlenstoffatomen oder durch eine Phenylgruppe. mono- oder disubstituierte Maleinsäureamido- oder Maleinsäureimidogruppe, wobei die Substituenten gleich oder verschieden sein können, eine durch eine Alkylsulfonylgruppe mit 4 bis 6 Kohlenstoffatomen oder durch eine Phenylalkylsulfonylgruppe substituierte Alkylamino- oder Phenylalkylaminogruppe, in denen der Alkylteil jeweils 1 bis 3 Kohlenstoffatome enthalten kann, eine durch eine Naphthalinsulfonylgruppe substituierte Amino- oder Alkylaminogruppe, welche im Naphthalinring durch eine Dialkylaminogruppe, durch eine oder zwei Alkoxygruppen substituiert sein können, wobei der Alkylteil jeweils 1 bis 3 Kohlenstoffatome enthalten kann, eine 7-Nitro-benzofurazan-4-yl-aminoalkanoylaminogruppe, in der der Alkanoylteil 2 oder 3 Kohlenstoffatome enthalten kann, eine Benzofurancarbonylamino- oder 7-Nitro-benzofurazan-4-yl-aminogruppe

oder auch, wenn $R_3$ eine Carboxygruppe und $R_2$ eine n-Butylgruppe darstellen, $R_1$ in 6-Stellung eine durch eine Phenylsulfonyl-, Cyclohexylmethylaminocarbonyl-, 2-Carboxycyclohexylmethylcarbonyl-, 2-tert.Butoxycarbonyl-cyclohexylmethylcarbonyl-, 2-Carboxy-3,4,5,6-tetrahydrobenzoyl-, N-Methyl-phenylaminocarbonyl- oder 3-Cyclohexylpropylgruppe substituierte Aminogruppe, eine durch eine Propylsulfonyl-, Phenylsulfonyl-, Methylphenylsulfonyl- oder Chlorphenylsulfonylgruppe substituierte Methylaminogruppe, eine durch eine Phenylsulfonyl- oder Methoxyphenylsulfonylgruppe substituierte n-Pentylaminogruppe, eine durch eine Methylphenylsulfonyl- oder Methoxyphenylsulfonylgruppe substituierte n-Propylaminogruppe, eine durch eine Benzoyl- oder Chlorphenylsulfonylgruppe substituierte Isopropylaminogruppe, eine N-Acetyl-cyclohexylmethylamino-, 3,4,5,6-Tetrahydrophthalimido-, Hexahydrohomophthalimido-, N-Methansulfonyl-2-phenylethylamino- oder N-Chlorphenylsulfonyl-benzylamino-gruppe

oder, wenn $R_3$ eine Carboxygruppe und $R_2$ eine n-Butylgruppe darstellen, $R_1$ in 5- oder 6-Stellung eine 2-Oxo-1,2-dihydro-3,4-tetramethylen-pyrrolidin-1-yl-gruppe

oder, wenn $R_3$ eine Carboxygruppe und $R_2$ eine Methyl-, Ethyl-, n-Propyl-, n-Butyl- oder Methylmercaptogruppe darstellen, $R_1$ in 6-Stellung eine Pyrrolidinocarbonylaminogruppe

oder auch, wenn $R_3$ eine Tetrazolylgruppe und $R_2$ eine n-Butylgruppe darstellen, $R_1$ in 5- oder 6-Stellung eine durch eine Methylaminocarbonyl- oder Cyclohexylaminocarbonylgruppe substituierte n-Pentylaminogruppe oder in 6-Stellung eine 3,3-Dimethyl-glutarsäureimido- oder 4,4-Tetramethylen-glutarsäureimidogruppe,

oder auch, wenn $R_3$ eine Tetrazolylgruppe und $R_2$ eine Ethyl- oder n-Propylgruppe darstellen, $R_1$ in 6-Stellung eine N-Benzolsulfonyl-methylaminogruppe

oder auch, wenn $R_3$ eine tert.Butoxycarbonylgruppe und $R_2$ eine N-Butylgruppe darstellen, $R_1$ in 6-Stellung eine 2-Carboxy-cyclohexylmethylcarbonylamino- oder Pyrrolidinocarbonylaminogruppe bedeuten:

Umsetzung einer Verbindung der allgemeinen Formel

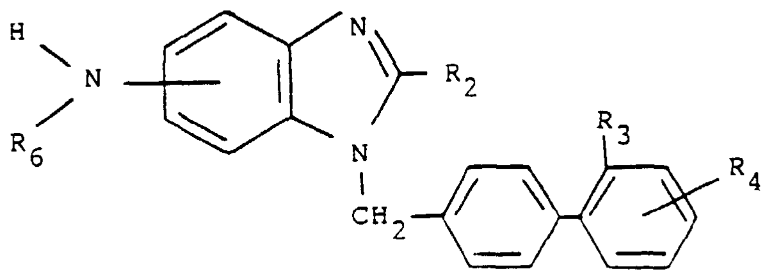

, (X)

in der

$R_2$, $R_3$ und $R_4$ wie eingangs definiert sind und
$R_6$ ein Wasserstoffatom, eine n-Pentyl-, Cyclohexylmethyl-, Alkyl- oder Phenylalklygruppe mit jeweils 1 bis 3 Kohlenstoffatomen im Alkylteil bedeutet, mit einer Verbindung der allgemeinen Formel

$$Z_4 - W - R_7, \qquad (XI)$$

in der
$Z_4$ eine nukleophile Austrittsgruppe,
W eine -CO- oder -$SO_2$-Gruppe und
$R_7$ eine 2-Hydroxycarbonyl-ethenylgruppe, in der der Ethenylteil durch eine Alkylgruppe mit 1 bis 3 Kohlenstoffatomen oder durch eine Phenylgruppe mono- oder disubstituiert ist und die Substituenten gleich oder verschieden sein können, eine Alkylgruppe mit 3 bis 6 Kohlenstoffatomen, eine Phenylalkylgruppe mit 1 bis 3 Kohlenstoffatomen im Alkylteil, eine gegebenenfalls durch eine Dialkylaminogruppe, durch eine oder zwei Alkoxygruppen substituierte Naphthalingruppe, in denen der Alkylteil jeweils 1 bis 3 Kohlenstoffatome enthalten kann, eine Methyl-, Phenyl-, Methylphenyl-,Methoxyphenyl-, Chlorphenyl-, Biphenyl-, Bicyclohexyl-, 2-Carboxy-cyclohexylmethyl-, 2-Carboxy-3,4,5,6-tetrahydrophenyl-, 3-Carboxy-1,1-dimethyl-propyl-, 3-Carboxy-2,2-tetramethylenpropyl-, 7-Nitro-benzofurazan-4-yl-aminomethyl- oder 7-Nitro-benzofurazan-4-yl-aminoethylgruppe,

oder auch, wenn W eine -CO-Gruppe darstellt, eine $R_8NR_9$-Gruppe, in der

$R_8$ ein Wasserstoffatom oder eine Alkylgruppe mit 1 bis 3 Kohlenstoffatomen,

$R_9$ eine Methyl-, Cyclohexyl-, Cyclohexylmethyl-, Phenyl-, Biphenyl- oder Bicyclohexylgruppe oder

$R_8$ und $R_9$ zusammen mit dem dazwischenliegenden Stickstoffatom eine Pyrrolidinogruppe

oder auch $Z_4$ zusammen mit $R_9$ eine weitere Kohlenstoff-Stickstoff-Bindung darstellen,

oder auch $R_7$ zusammen mit W eine 7-Nitro-benzofurazan-4-yl-aminogruppe bedeuten.

Als nukleophile Austrittsgruppe kommt für $Z_4$ beispielsweise das Chlor- oder Bromatom, eine Alkoxy- oder Phenylalkoxygruppe wie die Methoxy-, Ethoxy- oder Benzyloxygruppe oder auch, wenn $R_7$ einen der vorstehend erwähnten Kohlenwasserstoffreste darstellt, eine Hydroxygruppe in Betracht.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären organischen Base wie Triäthylamin oder Pyridin, welche gleichzeitig als Lösungsmittel dienen können, bei Temperaturen zwischen -25 und 150°C, vorzugsweise jedoch bei Temperaturen zwischen -10°C und der Siedetemperatur des verwendeten Lösungsmittels, durchgeführt. Bedeutet $Z_4$ eine Hydroxygruppe, so wird die Umsetzung besonders vorteilhaft jedoch mit den reaktionsfähigen Derivaten einer Carbonsäure der allgemeinen Formel IX, z.B. mit deren Estern, Thioestern, Halogeniden, Anhydriden oder Imidazoliden, durchgeführt.

i) Zur Herstellung von Verbindungen der allgemeinen Formel I, in der $R_1$ eine Tetrahydrobenzimidazolyl- oder Imidazopyridinylgruppe oder eine gegebenenfalls im Phenylkern durch ein Fluor-, Chlor- oder Bromatom, durch eine Alkylgruppe mit 1 bis 3 Kohlenstoffatomen, durch eine Alkoxygruppe mit 1 bis 3 Kohlenstoffatomen oder durch eine Trifluormethylgruppe substituierte Benzimidazolylgruppe, wobei die NH-Gruppe der vorstehend erwähnten Imidazolringe zusätzlich durch eine Alkylgruppe mit 1 bis 6 Kohlenstoffatomen oder durch eine Cycloalkylgruppe mit 3 bis 7 Kohlenstoffatomen substituiert sein kann, eine Hydroxycycloalkylaminocarbonylgruppe mit 5 bis 7 Kohlenstoffatomen im Cycloalkylteil, die am N-Atom zusätzlich durch eine Alkylgruppe mit 1 bis 3 Kohlenstoffatomen substituiert sein kann, oder eine geradkettige oder verzweigte Hydroxyalkylaminocarbonylgruppe mit 4 bis 6 Kohlenstoffatomen im Alkylteil bedeutet:
Umsetzung einer Verbindung der allgemeinen Formel

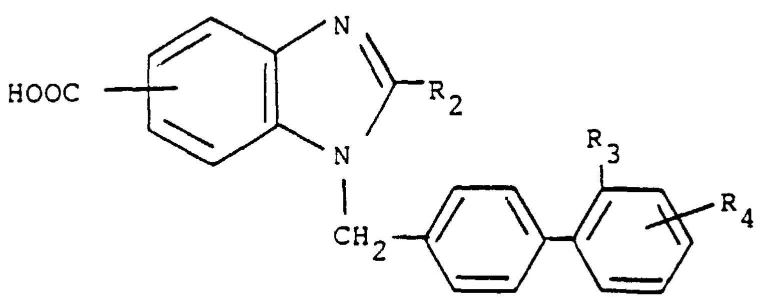

,(XII)

in der

$R_2$ bis $R_4$ wie eingangs definiert sind, oder deren reaktionsfähige Derivate wie deren Säurenhalogenide, Ester, Amide, Anhydride oder Nitrile mit einem Amin der allgemeinen Formel

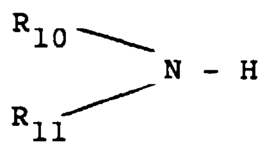

,(XIII)

in der
$R_{10}$ ein Wasserstoffatom, eine Cycloalkylgruppe oder eine Alkylgruppe mit 1 bis 6 Kohlenstoffatomen und
$R_{11}$ eine Hydroxyalkylgruppe mit 4 bis 6 Kohlenstoffatomen, eine Hydroxycycloalkylgruppe mit 5 bis 7 Kohlenstoffatomen oder eine 2-Aminophenylgruppe, die im Phenylkern durch ein Fluor-, Chlor- oder Bromatom, durch eine Alkylgruppe mit 1 bis 3 Kohlenstoffatomen, durch eine Alkoxygruppe mit 1 bis 3 Kohlenstoffatomen oder durch eine Trifluormethylgruppe substituiert sein kann, eine 2-Aminocyclohexyl- oder 2-Aminopyridylgruppe bedeuten, unter gegebenenfalls gleichzeitiger Decarboxylierung.

Die Umsetzung wird zweckmäßigerweise in einem Lösungsmittel wie Methylenchlorid, Chloroform, Tetrachlorkohlenstoff, Äther, Tetrahydrofuran, Dioxan, Benzol, Toluol, Acetonitril oder Dimethylformamid, gegebenenfalls in Gegenwart eines die Säure aktivierenden Mittels oder eines wasserentziehenden Mittels, z.B. in Gegenwart von Chlorameisensäureäthylester, Thionylchlorid, Phosphortrichlorid, Phosphorpentoxid, N,N'-Dicyclohexylcarbodiimid, N,N'-Dicyclohexylcarbodiimid/N-Hydroxysuccinimid, N,N'-Carbonyldiimidazol oder N,N'-Thionyldiimidazol oder Triphenylphosphin/Tetrachlorkohlenstoff, oder eines die Aminogruppe aktivierenden Mittels, z.B. Phosphortrichlorid, und gegebenenfalls in Gegenwart einer anorganischen Base wie Natriumcarbonat oder einer tertiären

organischen Base wie Triäthylamin oder Pyridin, welche gleichzeitig als Lösungsmittel dienen können, bei Temperaturen zwischen -25 und 150°C, vorzugsweise jedoch bei Temperaturen zwischen -10°C und der Siedetemperatur des verwendeten Lösungsmittels, durchgeführt.

Eine gegebenenfalls so erhaltene ortho-Benzamidoverbindung kann erforderlichenfalls anschließend durch Erhitzen zweckmäßigerweise in einem Lösungsmittel oder Lösungsmittelgemisch wie Ethanol, Isopropanol, Eisessig, Benzol, Chlorbenzol, Toluol, Xylol, Glycol, Glycolmonomethylether, Diethylenglycoldimethylether, Sulfolan, Dimethylformamid oder Tetralin gegebenenfalls in Gegenwart eines Kondensationsmittels wie Phosphoroxychlorid, Thionylchlorid, Sulfurylchlorid, Schwefelsäure, p-Toluolsulfonsäure, Methansulfonsäure, Salzsäure, Phosphorsäure, Polyphosphorsäure, Essigsäureanhydrid oder gegebenenfalls auch in Gegenwart einer Base wie Kaliumäthylat oder Kaliumtert.butylat in die gewünschte Benzimidazolverbindung übergeführt werden. Diese Cyclisierung kann jedoch auch ohne Lösungsmittel und/oder Kondensationsmittel durchgeführt werden.

k) Zur Herstellung von Verbindungen der allgemeinen Formel I, in der $R_1$ eine Dihydro-pyridazin-3-on- oder Pyridazin-3-on-gruppe darstellt, die in 2-Stellung durch eine gegebenenfalls Phenylgruppe substituierte Alkylgruppe mit 1 bis 3 Kohlenstoffatomen oder im Kohlenstoffgerüst durch eine oder zwei Alkylgruppen mit jeweils 1 bis 3 Kohlenstoffatomen substituiert sein kann:
Umsetzung einer Carbonsäure der allgemeinen Formel

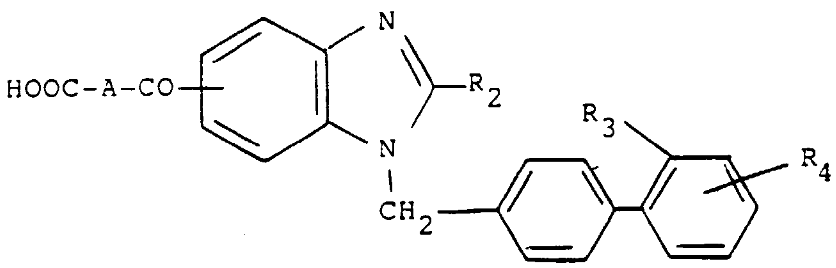

, (XIV)

in der

$R_1$ bis $R_4$ wie eingangs definiert sind und
A eine gegebenenfalls durch eine oder zwei Alkylgruppen mit jeweils 1 bis 3 Kohlenstoffatomen substituierte Ethylen- oder Ethenylengruppe darstellt, oder deren reaktionsfähige Säurederivate wie deren Ester, Amide oder Halogenide mit einem Hydrazin der allgemeinen Formel

$$H_2N - NHR_{12}, \qquad (XV)$$

in der
$R_{12}$ ein Wasserstoffatom oder eine gegebenenfalls durch eine Phenylgruppe substituierte Alkylgruppe mit 1 bis 3 Kohlenstoffatomen darstellt.

Die Umsetzung wird zweckmäßigerweise in einem Lösungsmittel wie Methanol, Äthanol, Isopropanol, Eisessig, Propionsäure und/oder in einem Überschuß des eingesetzten Hydrazins bzw. Hydrazin-hydrats bei Temperaturen zwischen 0 und 200°C, z.B. bei Temperaturen zwischen 20 und 150°C, vorzugsweise jedoch bei der Siedetemperatur des Reaktionsgemisches, und gegebenenfalls in Gegenwart einer Säure wie Schwefelsäure oder p-Toluolsulfonsäure als Kondensationsmittel durchgeführt. Die Umsetzung kann jedoch auch ohne Lösungsmittel durchgeführt werden.

Bei den vorstehend beschriebenen Umsetzungen können gegebenenfalls vorhandene reaktive Gruppen wie Hydroxy-, Amino- oder Alkylaminogruppen während der Umsetzung durch übliche Schutzgruppen geschützt werden, welche nach der Umsetzung wieder abgespalten werden.

Beispielsweise kommt als Schutzrest für eine Hydroxygruppe die Trimethylsilyl-, Acetyl-, Benzoyl-, Methyl-, Ethyl-, tert.Butyl-, Benzyl- oder Tetrahydropyranylgruppe und als Schutzrest für eine Amino-, Alkylamino- oder Iminogruppe die Acetyl-, Benzoyl-, Ethoxycarbonyl- oder Benzylgruppe in Betracht.

Die gegebenenfalls anschließende Abspaltung eines verwendeten Schutzrestes erfolgt vorzugsweise hydrolytisch in einem wässrigen Lösungsmittel, z.B. in Wasser, Isopropanol/Wasser, Tetrahydrofuran/Wasser oder Dioxan/Wasser,

in Gegenwart einer Säure wie Salzsäure oder Schwefelsäure oder in Gegenwart einer Alkalibase wie Natriumhydroxid oder Kaliumhydroxid bei Temperaturen zwischen 0 und 100°C, vorzugsweise bei der Siedetemperatur des Reaktionsgemisches. Die Abspaltung eines Benzylrestes erfolgt jedoch vorzugsweise hydrogenolytisch, z.B. mit Wasserstoff in Gegenwart eines Katalysators wie Palladium/Kohle in einem Lösungsmittel wie Methanol, Ethanol, Essigsäureethylester oder Eisessig gegebenenfalls unter Zusatz einer Säure wie Salzsäure bei Temperaturen zwischen 0 und 50°C, vorzugsweise jedoch bei Raumtemperatur, und einem Wasserstoffdruck von 1 bis 7 bar, vorzugsweise jedoch von 3 bis 5 bar.

Ein so erhaltenes Isomerengemisch einer Verbindung der allgemeinen Formel I kann gewünschtenfalls vorzugsweise chromatographisch unter Verwendung eines Trägers wie Kieselgel oder Aluminiumoxid getrennt werden.

Desweiteren können die erhaltenen Verbindungen der allgemeinen Formel I in ihre Säureadditionssalze, insbesondere für die pharmazeutische Anwendung in ihre physiologisch verträglichen Salze mit anorganischen oder organischen Säuren, übergeführt werden. Als Säuren kommen hierfür beispielsweise Salzsäure, Bromwasserstoffsäure, Schwefelsäure, Phosphorsäure, Fumarsäure, Bernsteinsäure, Milchsäure, Zitronensäure, Weinsäure oder Maleinsäure in Betracht.

Außerdem lassen sich die so erhaltenen neuen Verbindungen der allgemeinen Formel I, falls diese eine Carboxy- oder 1H-Tetrazolylgruppe enthalten, gewünschtenfalls anschließend in ihre Salze mit anorganischen oder organischen Basen, insbesondere für die pharmazeutische Anwendung in ihre physiologisch verträglichen Salze, überführen. Als Basen kommen hierbei beispielsweise Natriumhydroxid, Kaliumhydroxid, Cyclohexylamin, Äthanolamin, Diäthanolamin und Triäthanolamin in Betracht.

Die als Ausgangsstoffe verwendeten Verbindungen der allgemeinen Formeln II bis XV sind teilweise literaturbekannt oder erhält diese nach literaturbekannten Verfahren.

So erhält man beispielsweise eine Verbindung der allgemeinen Formel II durch Alkylierung einer entsprechenden o-Amino-nitroverbindung und anschließende Reduktion der Nitrogruppe.

Eine als Ausgangsstoff verwendete Verbindung der allgemeinen Formeln III, V, VI, VII, VIII, IX, X, XII oder XIV erhält man durch Alkylierung eines entsprechenden o-Phenylendiamins oder einer entsprechenden o-Amino-nitroverbindung, anschließender Reduktion der Nitrogruppe und anschließender Cyclisierung einer so erhaltenen o-Diaminophenylverbindung und gegebenenfalls anschließender Abspaltung eines verwendeten Schutzrestes oder durch NH-Alkylierung eines entsprechenden 1H-Benzimidazols, wobei das so erhaltene Isomerengemisch anschließend mittels üblicher Methoden, z.B. mittels Chromatographie, aufgetrennt werden kann. Die vorstehend erwähnten Ausgangsverbindungen werden teilweise in der nicht vorveröffentlichten EP-A-0 392 317 beschrieben.

Die neuen Verbindungen der allgemeinen Formel I und deren physiologisch verträgliche Salze weisen wertvolle pharmakologische Eigenschaften auf. Sie stellen Angiotensin-Antagonisten, insbesondere Angiotensin-II-Antagonisten, dar.

Beispielsweise wurden die Verbindungen

A = 4'-[[2-n-Propyl-6-(1-methylbenzimidazol-2-yl)-benzimidazol-1-yl]methyl]biphenyl-2-carbonsäure,

B = 4'-[[2-n-Butyl-6-(3,4,5,6-tetrahydro-phthalimino)-benzimidazol-1-yl]methyl]biphenyl-2-carbonsäure-dihydrat,

C = 4'-[[2-n-Butyl-6-(2,3-diphenyl-maleinsäureimido)-benzimidazol-1-yl]methyl]biphenyl-2-carbonsäure,

D = 4'-[[2-n-Butyl-6-(2,3-dimethyl-maleinsäureimido)-benzimidazol-1-yl]methyl]biphenyl-2-carbonsäure,

E = 4'-[[2-n-Butyl-6-(N-phenylmethansulfonyl-methylamino)-benzimidazol-1-yl]methyl]biphenyl-2-carbonsäure,

F = 4'-[[2-n-Butyl-6-(2-oxo-piperidin-1-yl)-benzimidazol-1-yl]methyl]-2-(1H-tetrazol-5-yl)-biphenyl,

G = 4'-[[2-n-Butyl-6-(2-oxo-pyrrolidin-1-yl)-benzimidazol-1-yl]methyl]-2-(1H-tetrazol-5-yl)-biphenyl,

H = 4'-[[2-n-Butyl-6-(2-oxo-hexamethylenimino)-benzimidazol-1-yl]methyl]-2-(1H-tetrazol-5-yl)-biphenyl,

I = 4'-[[2-n-Butyl-6-(3,3-dimethylglutarimido)-benzimidazol-1-yl]methyl]-2-(1H-tetrazol-5-yl)-biphenyl,

J = 4'-[[2-n-Butyl-6-(N-methylaminocarbonyl-n-pentylamino)-benzimidazol-1-yl]methyl]-2-(1H-tetrazol-5-yl)-biphenyl,

K = 4'-[[2-n-Butyl-6-(Cyclohexylaminocarbonyl-n-pentylamino)-benzimidazol-1-yl]methyl]-2-(1H-tetrazol-5-yl)-biphenylhydrat und

L = 4'-[[2-n-Butyl-6-(2-oxo-3,4-tetramethylen-pyrrolidin-1-yl)-benzimidazol-1-yl]methyl]biphenyl-2-carbonsäure

auf ihre biologischen Wirkungen wie folgt untersucht:

Ratten (männlich, 180-220 g) werden mit Hexobarbital-Natrium (150 mg/kg i.p.) narkotisiert. Nach Eintreten der Narkose wird eine Trachealkanüle gelegt, die Tiere werden despinalisiert und dann sofort mit einer Atempumpe künstlich beatmet. Der arterielle Blutdruck wird über eine Kanüle in der Arteria Carotis mittels eines Bell & Howell Druckaufnehmers registriert. Substanzen werden über eine Kanüle in die Vena Jugularis appliziert.

Test-Substanzen werden mit drei Dosierungen (10, 20 und 30 mg/kg i.v.) appliziert, wobei pro Tier eine Substanzdosis getestet wird. Drei Minuten nach der intravenösen Applikation der Testsubstanz wird Angiotensin-II in steigender Dosierung intravenös appliziert und so eine kumulative Dosis-Wirkungsbeziehung für Angiotensin-II in Gegenwart der Testsubstanzen erreicht. Der Meßparameter ist die Steigerung des arteriellen Blutdruckes.

Diese Dosis-Wirkungskurven werden mit Standardkurven für Angiotensin-II ohne Testsubstanzen verglichen. Mittels eines Computerprogramms werden die Rechtsverschiebungen der Dosis-Wirkungskurven von Angiotensin-II durch Testsubstanzen ermittelt und entsprechende $pA_2$-Werte für die Testsubstanzen berechnet.

Die $pA_2$-Werte der genannten Testverbindungen A bis L liegen zwischen 6.0 und 7.5.

Desweiteren konnten bei der Applikation der vorstehenden Verbindungen bis zu einer Dosis von 30 mg/kg i.v. keine toxischen Nebenwirkungen, z.B. keine negativ inotrope Wirkung und keine Herzrhythmusstörungen, beobachtet werden. Die Verbindungen sind demnach gut verträglich.

Aufgrund ihrer pharmakologischen Eigenschaften eignen sich die neuen Verbindungen und deren physiologisch verträgliche Salze zur Behandlung der Hypertonie und Herzinsuffizienz, ferner zur Behandlung ischämischer peripherer Durchblutungsstörungen, der myokardialen Ischämie (Angina), zur Prävention der Herzinsuffizienzprogression nach Myokard-Infarkt, zur Behandlung der diabetischen Nephropathie, des Glaukoms, von gastrointestinalen Erkrankungen und Blasenerkrankungen.

Weiterhin eignen sich die neuen Verbindungen und deren physiologisch verträgliche Salze zur Behandlung pulmonarer Erkrankungen, z.B. von Lungenödemen und der chronischen Bronchitis, zur Prävention von arterieller Re-Stenosis nach Angioplastie, von Verdickungen der Gefäßwand nach Gefäßoperationen, der Arteriosklerose und der diabetischen Angiopathie. Auf Grund der Beeinflußung der Acetylcholin- und Dopamin-Freisetzung durch Angiotensin im Gehirn eignen sich die neuen Angiotensin-Antagonisten auch zur Behebung zentralnervöser Störungen, z.B. von Depressionen, der Alzheimer'schen Krankheit, des Parkinson-Syndroms, der Bulimie, sowie von Störungen kognitiver Funktionen.

Die zur Erzielung einer entsprechenden Wirkung erforderliche Dosierung beträgt zweckmäßigerweise bei intravenöser Gabe 20 bis 100 mg, vorzugsweise 30 bis 70 mg, und bei oraler Gabe 50 bis 200 mg, vorzugsweise 75 bis 150 mg, jeweils 1 bis 3 x täglich. Hierzu lassen sich die erfindungsgemäß hergestellten Verbindungen der allgemeinen Formel I, gegebenenfalls in Kombination mit anderen Wirksubstanzen wie z.B. Blutdrucksenker, Diuretika und/oder Kalzium-Antagonisten, zusammen mit einem oder mehreren inerten üblichen Trägerstoffen und/oder Verdünnungsmitteln, z.B. mit Maisstärke, Milchzucker, Rohrzucker, mikrokristalliner Zellulose, Magnesiumstearat, Polyvinylpyrrolidon, Zitronensäure, Weinsäure, Wasser, Wasser/Äthanol, Wasser/Glycerin, Wasser/Sorbit, Wasser/Polyäthylenglykol, Propylenglykol, Cetylstearylalkohol, Carboxymethylcellulose oder fetthaltigen Substanzen wie Hartfett oder deren geeigneten Gemischen, in übliche galenische Zubereitungen wie Tabletten, Dragees, Kapseln, Pulver, Suspensionen oder Zäpfchen einarbeiten.

Für die oben erwähnten Kombinationen kommen somit als weitere Wirksubstanzen beispielsweise Bendroflumethiazid, Chlorthiazid, Hydrochlorthiazid, Spironolacton, Benzthiazid, Cyclothiazid, Ethacrinsäure, Furosemid, Metoprolol, Prazosin, Atenolol, Propranolol, (Di)hydralazin-hydrochlorid, Diltiazem, Felodipin, Nicardipin, Nifedipin, Nisoldipin und Nitrendipin in Betracht. Die Dosis für diese Wirksubstanzen beträgt hierbei zweckmäßigerweise 1/5 der üblicherweise empfohlenen niedrigsten Dosierung bis zu 1/1 der normalerweise empfohlenen Dosierung, also beispielsweise 15 bis 200 mg Hydrochlorthiazid, 125 bis 2000 mg Chlorthiazid, 15 bis 200 mg Ethacrinsäure, 5 bis 80 mg Furosemid, 20 bis 480 mg Propranolol, 5 bis 60 mg Felodipin, 5 bis 60 mg Nifedipin oder 5 bis 60 mg Nitrendipin.

Die nachfolgenden Beispiele sollen die Erfindung näher erläutern:

## Beispiel 1

4'-[[2-n-Propyl-5-(1-methylbenzimidazol-1-yl]methyl]biphenyl-2-carbonsäure und 4'-[[2-n-Propyl-6-(1-methylbenzimidazol-2-yl)-benzimidazol-1-yl]methyl]biphenyl-2-carbonsäure

a) 2-n-Propyl-benzimidazol-5-carbonsäure-methylester

Eine Lösung von 23,9 g (100 mMol) 3,4-Diaminobenzoesäuremethylester-dihydrochlorid und 11,7 g (110 mMol) Buttersäurechlorid in 100 ml Phosphoroxychlorid wird 2 Stunden lang unter Rückfluß erhitzt. Anschließend werden ca. 80 ml Phosphoroxychlorid abdestilliert und der Rückstand mit ca. 150 ml Wasser zersetzt. Das ausgefallene ölige Rohprodukt wird mit 3 x 50 ml Essigester extrahiert und nach dem Einengen durch Säulenchromatographie (600 g Kieselgel; Laufmittel: Methylenchlorid/Methanol (30:1)) gereinigt.
Ausbeute: 15,0 g Öl (69 % der Theorie)

b) 2-n-Propyl-benzimidazol-5-carbonsäure-hemisulfat

Eine Lösung von 15,0 g (73 mMol) 2-n-Propyl-benzimidazol-5-carbonsäuremethylester und 8 g (200 mMol) Natriumhydroxid in 200 ml Wasser und 400 ml Ethanol wird 2 Stunden lang unter Rückfluß erhitzt. Anschließend wird der Alkohol abdestilliert, die wäßrige Lösung mit verdünnter Schwefelsäure angesäuert (pH 4-5) und am Rotationsverdampfer eingeengt. Das dabei auskristallisierende Produkt wird abgesaugt, mit je 50 ml Aceton und Diethylether gewaschen und getrocknet. Ausbeute: 9,1 g (61 % der Theorie),
Schmelzpunkt: > 220°C.

| $C_{11}H_{12}N_2O_2$ x 1/2 $H_2SO_4$ (253,26) | | | | |
|---|---|---|---|---|
| Ber.: | C 52,17 | H 5,17 | N 11,06 | S 6,33 |
| Gef.: | 51,87 | 5,23 | 11,11 | 6,41 |

c) 2-n-Propyl-5-(1-methylbenzimidazol-2-yl)-benzimidazol

Eine Lösung von 6,7 g (25 mMol) 2-n-Propyl-benzimidazol-5-carbonsäure-Hemisulfat und 4,9 g (25 mMol) 2-Methylaminoanilin-dihydrochlorid in 200 g Polyphosphorsäure wird 5 Stunden lang bei 150°C gerührt, anschließend auf 600 ml Wasser gegossen und unter Eiskühlung mit konzentriertem Ammoniak alkalisch gestellt. Die erhaltene Lösung wird dreimal mit je 200 ml Essigester extrahiert, das so erhaltene Rohprodukt durch Säulenchromatographie (300 g Kieselgel; Laufmittel: Methylenchlorid/Methanol = 15:1) gereinigt.
Ausbeute: 2,8 g Öl (39 % der Theorie),

| $C_{18}H_{18}N_4$ (290,37) | | | |
|---|---|---|---|
| Ber.: | C 74,46 | H 6,25 | N 19,29 |
| Gef.: | 73,92 | 6,32 | 18,96 |

d) 4'-[[2-n-Propyl-5-(1-methylbenzimidazol-2-yl)-benzimidazol-1-yl]methyl]biphenyl-2-carbonsäure-tert.butylester und 4'-[[2-n-Propyl-6-(1-methylbenzimidazol-2-yl)-benzimidazol-1-yl]methyl]biphenyl-2-carbonsäure-tert.butylester

Eine Lösung von 2,0 g (6,9 mMol) 2-n-Propyl-5-(1-methylbenzimidazol-2-yl)-benzimidazol und 0,91 g (7,5 mMol) Kaliumtert.butylat in 50 ml Dimethylsulfoxid wird 90 Minuten lang bei Raumtemperatur gerührt, anschließend 2,6 g (7,5 mMol) 4'-Brommethyl-biphenyl-2-carbonsäure-tert.butylester hinzugegeben und weitere 15 Stunden bei Raumtemperatur gerührt. Das Gemisch wird dann auf 300 ml Wasser gegossen und dreimal mit je 50 ml Essigester extrahiert. Das nach dem Eindampfen der organischen Phase erhaltene Rohprodukt wird durch Säulenchromatographie (300 g Kieselgel; Laufmittel: Methylenchlorid/Methanol = 30:1) gereinigt. Man erhält so 2,7 g (70 % der Theorie) eines Isomerengemisches, das, anhand NMR-spektroskopischer Daten geschätzt, ca. 1,18 g 4'-[(2-n-Propyl-5-(1-methylbenzimidazol-2-yl)-benzimidazol-1-yl)-methyl]biphenyl-2-carbonsäure-tert.butylester und ca. 1,52 g 4'-[(2-n-Propyl-6-(1-methylbenzimidazol-2-yl)-benzimidazol-1-yl)-methyl]biphenyl-2-carbonsäure-tert.butylester enthält. $R_f$-Wert: 0,43 (Methylenchlorid/Methanol = 19:1)

e) 4'-[[2-n-Propyl-5-(1-methylbenzimidazol-2-yl)-benzimidazol-1-yl]methyl]biphenyl-2-carbonsäure und 4'-[[2-n-Propyl-6-(1-methylbenzimidazol-2-yl)-benzimidazol-1-yl]methyl]biphenyl-2-carbonsäure

2,70 g des gemäß Beispiel 1d erhaltenen Isomerengemisches werden in 100 ml Methylenchlorid gelöst, mit 40 ml Trifluoressigsäure versetzt und vier Stunden bei Raumtemperatur gerührt. Anschließend wird im Vakuum zur Trockne eingeengt, der Rückstand in 100 ml 2N Natronlauge gelöst, die Lösung mit 50 ml Diethylether gewaschen und das Produktgemisch durch Ansäuern der wäßrigen Phase mit Essigsäure ausgefällt. Durch Säulenchromatographie (400 g Kieselgel, Laufmittel: Methylenchlorid/Methanol = 15:1) des so erhaltenen Feststoffes erhält man
0,7 g (58 % der Theorie) 4'-[[2-n-Propyl-5-(1-methylbenzimidazol-2-yl)-benzimidazol-1-yl]methyl]biphenyl-2-Carbonsäure vom Schmelzpunkt 219-220°C

| $C_{32}H_{28}N_4O_2$ (500,60) | | | |
|---|---|---|---|
| Ber.: | C 76,78 | H 5,64 | N 11,19 |

(fortgesetzt)

| $C_{32}H_{28}N_4O_2$ (500,60) | | | |
|---|---|---|---|
| Gef.: | 76,54 | 5,57 | 11,01 |

$R_f$-Wert: 0,15 (Methylenchlorid/Methanol = 9:1)
und 0,9 g (74 % der Theorie) 4'-[[2-n-Propyl-6-(1-methylbenzimidazol-2-yl)-benzimidazol-1-yl]methyl]biphenyl-2-carbonsäure vom Schmelzpunkt 217-218°C

| $C_{32}H_{28}N_4O_2$ (500,60) | | | |
|---|---|---|---|
| Ber.: | C 76,78 | H 5,64 | N 11,19 |
| Gef.: | 76,63 | 5,55 | 11,29 |

$R_f$-Wert: 0,40 (Methylenchlorid/Methanol = 9:1)

Analog werden folgenden Verbindungen erhalten:

4'-[[2-n-Propyl-6-(1,6-dimethyl-benzimidazol-2-yl)-benzimidazol-1-yl]methyl]biphenyl-2-carbonsäure

4'-[[2-n-Butyl-6-(1-methyl-5-brom-benzimidazol-2-yl)-benzimidazol-1-yl]methyl]biphenyl-2-carbonsäure

4'-[[2-n-Butyl-6-(1-methyl-5-methoxy-benzimidazol-2-yl)-benzimidazol-1-yl]methyl]biphenyl-2-carbonsäure

4'-[[2-n-Butyl-6-(1-n-butyl-5-trifluormethyl-benzimidazol-2-yl)-benzimidazol-1-yl]methyl]biphenyl-2-carbonsäure

4'-[[2-n-Butyl-6-(1-n-hexyl-5-methyl-benzimidazol-2-yl)-benzimidazol-1-yl]methyl]biphenyl-2-carbonsäure

4'-[[2-n-Propyl-6-(1-methyl-5-fluor-benzimidazol-2-yl)-benzimidazol-1-yl]methyl]biphenyl-2-carbonsäure

4'-[[2-n-Propyl-6-(1-methyl-5-chlor-benzimidazol-2-yl)-benzimidazol-1-yl]methyl]biphenyl-2-carbonsäure

Beispiel 2

4'-[[2-n-Butyl-6-(1-methylbenzimidazol-2-yl)-benzimidazol-1-yl]methyl]biphenyl-2-carbonsäure

Hergestellt analog Beispiel 1 aus 4'-[[2-n-Butyl-6-(1-methylbenzimidazol-2-yl)-benzimidazol-1-yl]methyl]biphenyl-2-carbonsäure-tert.butylester und Trifluoressigsäure in Methylenchlorid.
Ausbeute: 43 % der Theorie,
Schmelzpunkt: amorph

| $C_{33}H_{30}N_4O_2$ (514,60) | | | |
|---|---|---|---|
| Ber.: | C 77,02 | H 5,88 | N 10,89 |
| Gef.: | 76,88 | 5,83 | 10,55 |

$R_f$-Wert: 0,42 (Kieselgel; Laufmittel: Methylenchlorid/Ethanol = 9:1)
Massenspektrum: $(M + H)^+ = 515$

Beispiel 3

4'-[[6-(Biphenyl-4-carbonylamino)-2-n-butyl-benzimidazol-1-yl]methyl]biphenyl-2-carbonsäure x 0,25 $H_2O$

Hergestellt analog Beispiel 1 aus 4'-[[6-(Biphenyl-4-carbonylamino)-2-n-butyl-benzimidazol-1-yl]methyl]biphenyl-2-carbonsäure-tert.butylester und Trifluoressigsäure in Methylenchlorid.
Ausbeute: 70,6 % der Theorie,
Schmelzpunkt: 316-317°C

| $C_{38}H_{33}N_3O_3$ x 0,25 $H_2O$ (584,20) | | | |
|---|---|---|---|
| Ber.: | C 78,13 | H 5,78 | N 7,19 |
| Gef.: | 78,12 | 5,79 | 7,08 |

$R_f$-Wert: 0,25 (Kieselgel; Laufmittel: Essigester/Ethanol/ Ammoniak = 80:40:2)

Beispiel 4

4'-[[6-(Biphenylyl-4-aminocarbonylamino)-2-n-butyl-benzimidazol-1-yl]methyl]biphenyl-2-carbonsäure-trifluoracetat-semihydrat

Hergestellt analog Beispiel 1 aus 4'-[[6-(Biphenylyl-4-aminocarbonylamino)-2-n-butyl-benzimidazol-1-yl]methyl]biphenyl-2-carbonsäure-tert.butylester und Trifluoressigsäure in Methylenchlorid.
Ausbeute: 97,0 % der Theorie,
Schmelzpunkt: 171-172°C

| $C_{38}H_{34}N_4O_3$ x $CF_3COOH$ x 1/2 $H_2O$ (717,74) | | | |
|---|---|---|---|
| Ber.: | C 66,94 | H 5,06 | N 7,81 |
| Gef.: | 67,13 | 4,99 | 7,76 |

$R_f$-Wert: 0,25 (Kieselgel; Laufmittel: Essigester/Ethanol/ Ammoniak = 80:40:2)

Beispiel 5

4'-[(6-Benzolsulfonamido-2-n-butyl-benzimidazol-1-yl)-methyl]biphenyl-2-carbonsäure

Hergestellt analog Beispiel 1 aus 4'-[(6-Benzlsulfonamido-2-n-butyl-benzimidazol-1-yl)-methyl]biphenyl-2-carbonsäure-tert.butylester und Trifluoressigsäure in Methylenchlorid. Ausbeute: 75,0 % der Theorie,
Schmelzpunkt: 251-252°C

| $C_{31}H_{29}N_3O_4S$ (539,65) | | | | |
|---|---|---|---|---|
| Ber.: | C 69,00 | H 5,42 | N 7,79 | S 5,94 |
| Gef.: | 68,96 | 5,52 | 7,82 | 5,86 |

$R_f$-Wert: 0,50 (Kieselgel; Laufmittel: Essigester/Ethanol/ Ammoniak = 50:45:5)

Beispiel 6

4'-[[6-(N-Benzolsulfonyl-methylamino)-2-n-butyl-benzimidazol-1-yl]methyl]biphenyl-2-carbonsäure

Hergestellt analog Beispiel 1 aus 4'-[[6-(N-Benzolsulfonylmethylamino)-2-n-butyl-benzimidazol-1-yl]methyl]biphenyl-2-carbonsäure-tert.butylester und Trifluoressigsäure in Methylenchlorid.
Ausbeute: 70,0 % der Theorie,
Schmelzpunkt: 211-212°C

| $C_{32}H_{31}N_3O_4S$ (553,68) | | | | |
|---|---|---|---|---|
| Ber.: | C 69,42 | H 5,64 | N 7,59 | S 5,79 |
| Gef.: | 69,24 | 5,66 | 7,53 | 6,02 |

$R_f$-Wert: 0,55 (Kieselgel; Laufmittel: Essigester/Ethanol/ Ammoniak = 50:45:5)

Beispiel 7

4'-[[2-n-Butyl-6-(cyclohexylmethylaminocarbonylamino)-benzimidazol-1-yl]methyl]biphenyl-2-carbonsäure-trifluoracetat

Hergestellt analog Beispiel 1 aus 4'-[[2-n-Butyl-6-(cyclohexylmethylaminocarbonylamino)-benzimidazol-1-yl]methyl]biphenyl-2-carbonsäure-tert.butylester und Trifluoressigsäure in Methylenchlorid.
Ausbeute: 91,1 % der Theorie,
Schmelzpunkt: 149-150°C

| $C_{33}H_{38}N_4O_3$ x $CF_3$ COOH (652,71) | | | |
|---|---|---|---|
| Ber.: | C 64,41 | H 6,02 | N 8,58 |
| Gef.: | 64,23 | 6,09 | 8,73 |

$R_f$-Wert: 0,25 (Kieselgel; Laufmittel: Essigester/Ethanol/ Ammoniak = 80:40:2)

Beispiel 8

4'-[[2-n-Butyl-6-(N-cyclohexylmethyl)-acetamido-benzimidazol-1-yl]methyl]biphenyl-2-carbonsäure

Hergestellt analog Beispiel 1 aus 4'-[[2-n-Butyl-6-(N-cyclohexylmethyl)-acetamido-benzimidazol-1-yl]methyl]biphenyl-2-carbonsäure-tert.butylester und Trifluoressigsäure in Methylenchlorid.
Ausbeute: 78,6 % der Theorie,
Schmelzpunkt: 185-187°C

| $C_{34}H_{39}N_3O_3$ (537,70) | | | |
|---|---|---|---|
| Ber.: | C 75,95 | H 7,31 | N 7,81 |
| Gef.: | 75,75 | 7,40 | 7,65 |

$R_f$-Wert: 0,45 (Kieselgel; Laufmittel: Essigester/Ethanol/ Ammoniak = 50:45:5)

Beispiel 9

4'-[[6-(Bicyclohexyl-4-carbonylamino)-2-n-butyl-benzimidazol-1-yl]methyl]biphenyl-2-carbonsäure-trifluoracetat

Hergestellt analog Beispiel 1 aus 4'-[[6-(Bicyclohexyl-4-carbonylamino)-2-n-butyl-benzimidazol-1-yl]methyl]biphenyl-2-carbonsäure-tert.butylester und Trifluoressigsäure in Methylenchlorid.
Ausbeute: 93,3 % der Theorie,
Schmelzpunkt: 104-106°C

| $C_{38}H_{45}N_3O_3$ x $CF_3COOH$ (705,82) | | | |
|---|---|---|---|
| Ber.: | C 68,07 | H 6,57 | N 5,95 |
| Gef.: | 68,38 | 6,64 | 5,80 |

$R_f$-Wert: 0,30 (Kieselgel; Laufmittel: Essigester/Ethanol/ Ammoniak = 80:40:2)

Beispiel 10

4'-[[6-(Bicyclohexyl-4-aminocarbonylamino)-2-n-butyl-benzimidazol-1-yl]methyl]biphenyl-2-carbonsäure-semitrifluoracetat-monohydrat

Hergestellt analog Beispiel 1 aus 4'-[[6-(Bicyclohexyl-4-aminocarbonylamino)-2-n-butyl-benzimidazol-1-yl]methyl] biphenyl-2-carbonsäure-tert.butylester und Trifluoressigsäure in Methylenchlorid.
Ausbeute: 94,9 % der Theorie,
Schmelzpunkt: 119-120°C

| $C_{38}H_{46}N_4O_3$ x 1/2 $CF_3$ COOH x $H_2O$ (681,83) | | | |
|---|---|---|---|
| Ber.: | C 68,70 | H 7,17 | N 8,22 |
| Gef.: | 68,32 | 6,91 | 7,81 |

$R_f$-Wert: 0,30 (Kieselgel; Laufmittel: Essigester/Ethanol/ Ammoniak = 80:40:2)

Beispiel 11

4'-[[2-n-Butyl-6-(3,4,5,6-tetrahydro-phthalimino)-benzimidazol-1-yl]methyl]biphenyl-2-carbonsäure-dihydrat

Hergestellt analog Beispiel 1 aus 4'-[[2-n-Butyl-6-(3,4,5,6-tetrahydro-phthalimino)-benzimidazol-1-yl]methyl]biphenyl-2-carbonsäure-tert.butylester und Trifluoressigsäure in Methylenchlorid.
Ausbeute: 14,7 % der Theorie,
Schmelzpunkt: 119-122°C

| $C_{33}H_{31}N_3O_4$ x 2 $H_2O$ (533,63) | | | |
|---|---|---|---|
| Ber.: | C 69,58 | H 6,19 | N 7,38 |
| Gef.: | 69,77 | 6,34 | 7,65 |

$R_f$-Wert: 0,45 (Kieselgel; Laufmittel: Essigester/Ethanol/ Ammoniak = 80:40:2)

Beispiel 12

4'-[[2-n-Butyl-6-(5-dimethylamino-naphthalin-1-sulfonamino)benzimidazol-1-yl]methyl]biphenyl-2-carbonsäure-semitrifluoracetat

Hergestellt analog Beispiel 1 aus 4'-[[2-n-Butyl-6-(5-dimethylamino-naphthalin-1-sulfonamino)-benzimidazol-1-yl]-methyl]biphenyl-2-carbonsäure-tert.butylester und Trifluoressigsäure in Methylenchlorid.
Ausbeute: 92,3 % der Theorie,
Schmelzpunkt: 148-150°C

| $C_{37}H_{36}N_4O_4S$ x 1/2 $CF_3COOH$ (689,78) | | | | |
|---|---|---|---|---|
| Ber.: | C 66,17 | H 5,33 | N 8,12 | S 4,64 |
| Gef.: | 65,40 | 5,33 | 7,92 | 5,19 |

Beispiel 13

4'-[[2-n-Butyl-6-(2,3-diphenyl-maleinsäureimido)-benzimidazol-1-yl]methyl]biphenyl-2-carbonsäure

Hergestellt analog Beispiel 1 aus 4'-[[2-n-Butyl-6-(2,3-diphenyl-maleinsäureimido)-benzimidazol-1-yl]methyl]biphenyl-2-Carbonsäure-tert.butylester und Trifluoressigsäure in Methylenchlorid.
Ausbeute: 82,6 % der Theorie,
Schmelzpunkt: 236-237°C

| $C_{41}H_{33}N_3O_4$ (631,73) | | | |
|---|---|---|---|
| Ber.: | C 77,95 | H 5,27 | N 6,65 |
| Gef.: | 77,66 | 5,24 | 6,56 |

$R_f$-Wert: 0,65 (Kieselgel; Laufmittel: Essigester/Ethanol/ Ammoniak = 50:45:5)

Beispiel 14

4'-[[2-n-Butyl-6-(N-methansulfonyl-2-phenylethylamino)-benzimidazol-1-yl]methyl]biphenyl-2-carbonsäure

Hergestellt analog Beispiel 1 aus 4'-[[2-n-Butyl-6-(N-methansulfonyl-2-phenylethylamino)-benzimidazol-1-yl]methyl]biphenyl-2-carbonsäure-tert.butylester und Trifluoressigsäure in Methylenchlorid.
Ausbeute: 71,4 % der Theorie,
Schmelzpunkt: 215-216°C

| $C_{34}H_{35}N_3O_4$ (581,73) | | | | |
|---|---|---|---|---|
| Ber.: | C 70,20 | H 6,06 | N 7,22 | S 5,51 |
| Gef.: | 69,99 | 6,14 | 7,23 | 5,55 |

$R_f$-Wert: 0,25 (Kieselgel; Laufmittel: Essigester/Ethanol/ Ammoniak = 80:40:2)

Beispiel 15

4'-[[2-n-Butyl-6-(2,3-dimethyl-maleinsäureimido)-benzimidazol-1-yl]methyl]biphenyl-2-carbonsäure

Hergestellt analog Beispiel 1 aus 4'-[[2-n-Butyl-6-(2,3-dimethyl-maleinsäureimido)benzimidazol-1-yl]methyl]biphenyl-2-carbonsäure-tert.butylester und Trifluoressigsäure in Methylenchlorid.
Ausbeute: 69,6 % der Theorie,
Schmelzpunkt: 289-290°C

| $C_{31}H_{29}H_3O_4$ (507,59) | | | |
|---|---|---|---|
| Ber.: | C 73,35 | H 5,76 | N 8,28 |
| Gef.: | 73,14 | 5,90 | 8,20 |

$R_f$-Wert: 0,55 (Kieselgel; Laufmittel: Essigester/Ethanol/ Ammoniak = 50:45:5)

Beispiel 16

4'-[[6-(N-Benzolsulfonyl-n-pentylamino)-2-n-butyl-benzimidazol-1-yl]methyl]biphenyl-2-carbonsäure

Hergestellt analog Beispiel 1 aus 4'-[[6-(N-Benzolsulfonyl-n-pentylamino)-2-n-butyl-benzimidazol-1-yl]methyl]biphenyl-2-carbonsäure-tert.butylester und Trifluoressigsäure in Methylenchlorid.
Ausbeute: 83,9 % der Theorie,
Schmelzpunkt: 243-244°C

| $C_{36}H_{39}N_3O_4S$ (609,78) | | | | |
|---|---|---|---|---|
| Ber.: | C 70,91 | H 6,45 | N 6,89 | S 5,26 |
| Gef.: | 70,92 | 6,21 | 6,98 | 5,19 |

$R_f$-Wert: 0,45 (Kieselgel; Laufmittel: Essigester/Ethanol/ Ammoniak = 80:40:2)

Beispiel 17

4'-[[2-n-Butyl-6-(N-4-methoxybenzolsulfonyl-n-pentylamino)benzimidazol-1-yl]methyl]biphenyl-2-carbonsäure

Hergestellt analog Beispiel 1 aus 4'-[[2-n-Butyl-6-(N-4-methoxybenzolsulfonyl-n-pentylamino)-benzimidazol-1-yl]methyl]biphenyl-2-carbonsäure-tert.butylester und Trifluoressigsäure in Methylenchlorid.
Ausbeute: 84,6 % der Theorie,
Schmelzpunkt: 207-208°C

| $C_{37}H_{41}N_3O_5S$ (639,81) | | | | |
|---|---|---|---|---|
| Ber.: | C 69,46 | H 6,46 | N 6,57 | S 5,01 |
| Gef.: | 69,31 | 6,50 | 6,77 | 5,21 |

$R_f$-Wert: 0,50 (Kieselgel; Laufmittel: Essigester/Ethanol/ Ammoniak = 80:40:2)

Beispiel 18

4'-[[2-n-Butyl-6-(N-4-chlorbenzolsulfonyl-methylamino)benzimidazol-1-yl]methyl]biphenyl-2-carbonsäure

Hergestellt analog Beispiel 1 aus 4'-[[2-n-Butyl-6-(N-4-chlorbenzolsulfonyl-methylamino)-benzimidazol-1-yl]methyl]biphenyl-2-carbonsäure-tert.butylester und Trifluoressigsäure in Methylenchlorid.
Ausbeute: 84,8 % der Theorie,
Schmelzpunkt: 240-241°C

| $C_{32}H_{30}ClN_3O_4S$ (588,12) | | | | | |
|---|---|---|---|---|---|
| Ber.: | C 65,35 | H 5,14 | N 7,14 | Cl 6,03 | S 5,45 |
| Gef.: | 65,02 | 5,30 | 7,17 | 6,21 | 5,46 |

Beispiel 19

4'-[[2-n-Butyl-6-(N-phenylmethansulfonyl-methylamino)-benzimidazol-1-yl]methyl]biphenyl-2-carbonsäure

Hergestellt analog Beispiel 1 aus 4'-[[2-n-Butyl-6-(N-phenylmethansulfonyl-methylamino)-benzimidazol-1-yl]methyl]biphenyl-2-carbonsäure-tert.butylester und Trifluoressigsäure in Methylenchlorid.
Ausbeute: 54,9 % der Theorie,
Schmelzpunkt: 208-209°C

| $C_{33}H_{33}N_3O_4S$ (567,70) | | | | |
|---|---|---|---|---|
| Ber.: | C 69,82 | H 5,86 | N 7,40 | S 5,65 |
| Gef.: | 69,54 | 5,79 | 7,47 | 5,59 |

Beispiel 20

4'-[[2-n-Butyl-6-(N-4-methylbenzolsulfonyl-methylamino)-benzimidazol-1-yl]methyl]biphenyl-2-carbonsäure

Hergestellt analog Beispiel 1 aus 4'-[[2-n-Butyl-6-(N-4-methylbenzolsulfonyl-methylamino)-benzimidazol-1-yl]methyl]biphenyl-2-carbonsäure-tert.butylester und Trifluoressigsäure in Methylenchlorid.
Ausbeute: 92,5 % der Theorie,
Schmelzpunkt: 259-260°C

| $C_{33}H_{33}N_3O_4S$ (567,70) | | | | |
|---|---|---|---|---|
| Ber.: | C 69,82 | H 5,86 | N 7,40 | S 5,65 |
| Gef.: | 69,70 | 5,90 | 7,44 | 5,68 |

$R_f$-Wert: 0,25 (Kieselgel; Laufmittel: Essigester/Ethanol/ Ammoniak = 80:40:2)

Beispiel 21

4'-[[2-n-Butyl-6-(N-n-propylsulfonyl-methylamino)-benzimidazol-1-yl]methyl]biphenyl-2-carbonsäure

Hergestellt analog Beispiel 1 aus 4'-[[2-n-Butyl-6-(N-n-propylsulfonyl-methylamino)-benzimidazol-1-yl]methyl]biphenyl-2-carbonsäure-tert.butylester und Trifluoressigsäure in Methylenchlorid.

Ausbeute: 67,3 % der Theorie,
Schmelzpunkt: 222-223°C

| $C_{29}H_{33}N_3O_4S$ (519,66) | | | | |
|---|---|---|---|---|
| Ber.: | C 67,03 | H 6,40 | N 8,09 | S 6,17 |
| Gef.: | 67,02 | 6,49 | 8,04 | 6,18 |

$R_f$-Wert: 0,20 (Kieselgel; Laufmittel: Essigester/Ethanol/ Ammoniak = 80:40:2)

Beispiel 22

4'-[[2-n-Butyl-6-(N-4-methoxybenzolsulfonyl-n-propylamino)benzimidazol-1-yl]methyl]biphenyl-2-carbonsäure

Hergestellt analog Beispiel 1 aus 4'-[[2-n-Butyl-6-(N-4-methoxybenzolsulfonyl-n-propylamino)-benzimidazol-1-yl] methyl]biphenyl-2-carbonsäure-tert.butylester und Trifluoressigsäure in Methylenchlorid.
Ausbeute: 86,4 % der Theorie,
Schmelzpunkt: 227-228°C

| $C_{35}H_{37}N_3O_5S$ (611,75) | | | | |
|---|---|---|---|---|
| Ber.: | C 68,72 | H 6,10 | N 6,87 | S 5,24 |
| Gef.: | 68,54 | 6,20 | 6,88 | 5,25 |

$R_f$-Wert: 0,25 (Kieselgel; Laufmittel: Essigester/Ethanol/ Ammoniak = 80:40:2)

Beispiel 23

4'-[[2-n-Butyl-6-(N-4-methylbenzolsulfonyl-n-propylamino)benzimidazol-1-yl]methyl]biphenyl-2-carbonsäure

Hergestellt analog Beispiel 1 aus 4'-[[2-n-Butyl-6-(N-4-methylbenzolsulfonyl-n-propylamino)-benzimidazol-1-yl] methyl]biphenyl-2-carbonsäure-tert.butylester und Trifluoressigsäure in Methylenchlorid.
Ausbeute: 82,8 % der Theorie,
Schmelzpunkt: 223-224°C

| $C_{35}H_{37}N_3O_4S$ (595,76) | | | | |
|---|---|---|---|---|
| Ber.: | C 70,56 | H 6,26 | N 7,05 | S 5,38 |
| Gef.: | 70,25 | 6,20 | 7,24 | 5,61 |

$R_f$-Wert: 0,28 (Kieselgel; Laufmittel: Essigester/Ethanol/ Ammoniak = 80:40:2)

Beispiel 24

4'-[[2-n-Butyl-6-(N-4-chlorbenzolsulfonyl-isopropylamino)benzimidazol-1-yl]methyl]biphenyl-2-carbonsäure

Hergestellt analog Beispiel 1 aus 4'-[[2-n-Butyl-6-(N-4-chlorbenzolsulfonyl-isopropylamino)-benzimidazol-1-yl]methyl]biphenyl-2-carbonsäure-tert.butylester und Trifluoressigsäure in Methylenchlorid.
Ausbeute: 82,1 % der Theorie,
Schmelzpunkt: 260-261°C

| $C_{34}H_{34}ClN_3O_4S$ (616,17) | | | | | |
|---|---|---|---|---|---|
| Ber.: | C 66,28 | H 5,56 | N 6,82 | Cl 5,75 | S 5,20 |
| Gef.: | 66,05 | 5,77 | 7,05 | 5,87 | 5,34 |

$R_f$-Wert: 0,30 (Kieselgel; Laufmittel: Essigester/Ethanol/ Ammoniak = 80:40:2)

Beispiel 25

4'-[[6-(N-Benzoyl-isopropylamino)-2-n-butyl-benzimidazol-1-yl]methyl]biphenyl-2-carbonsäure

Hergestellt analog Beispiel 1 aus 4'-[[6-(N-Benzoyl-isopropylamino)-2-n-butyl-benzimidazol-1-yl]methyl]biphenyl-2-carbonsäure-tert.butylester und Trifluoressigsäure in Methylenchlorid.
Ausbeute: 58,3 % der Theorie,
Schmelzpunkt: 209-210°C

| $C_{35}H_{35}N_3O_3$ (545,68) | | | |
|---|---|---|---|
| Ber.: | C 77,04 | H 6,46 | N 7,70 |
| Gef.: | 76,66 | 6,57 | 7,65 |

$R_f$-Wert: 0,20 (Kieselgel; Laufmittel: Essigester/Ethanol/ Ammoniak = 80:40:2)

Beispiel 26

4'-[[2-n-Butyl-6-(1H,3H-chinazolin-2,4-dion-3-yl)-benzimidazol-1-yl]methyl]biphenyl-2-carbonsäure-hemihydrat

Hergestellt analog Beispiel 1 aus 4'-[[2-n-Butyl-6-(1H,3H-chinazolin-2,4-dion-3-yl)-benzimidazol-1-yl]methyl]biphenyl-2-carbonsäure-tert.butylester und Trifluoressigsäure in Methylenchlorid.
Ausbeute: 53,1 % der Theorie,
Schmelzpunkt: 338-340°C

| $C_{33}H_{28}N_4O_4$ x 1/2 $H_20$ (553,61) | | | |
|---|---|---|---|
| Ber.: | C 71,59 | H 5,28 | N 10,12 |
| Gef.: | 71,19 | 5,33 | 10,22 |

Beispiel 27

4'-[[2-n-Butyl-6-(N-4-chlorbenzolsulfonyl-benzylamino)benzimidazol-1-yl]methyl]biphenyl-2-carbonsäure

Hergestellt analog Beispiel 1 aus 4'-[[2-n-Butyl-6-(N-4-chlorbenzolsulfonyl-benzylamino)-benzimidazol-1-yl]methyl]biphenyl-2-carbonsäure-tert.butylester und Trifluoressigsäure in Methylenchlorid.
Ausbeute: 64,5 % der Theorie,
Schmelzpunkt: 212-213°C

| $C_{38}H_{34}ClN_3O_4S$ (664,22) | | | | | |
|---|---|---|---|---|---|
| Ber.: | C 68,72 | H 5,16 | N 6,33 | Cl 5,34 | S 4,83 |
| Gef.: | 68,76 | 5,27 | 6,39 | 5,62 | 4,81 |

$R_f$-Wert: 0,28 (Kieselgel; Laufmittel: Essigester/Ethanol/ Ammoniak = 80:40:2)

Beispiel 28

4'-[[2-n-Butyl-6-(N-n-butansulfonyl-benzylamino)-benzimidazol-1-yl]methyl]biphenyl-2-carbonsäure

Hergestellt analog Beispiel 1 aus 4'-[[2-n-Butyl-6-(N-n-butansulfonyl-benzylamino)-benzimidazol-1-yl]methyl]biphenyl-2-carbonsäure-tert.butylester und Trifluoressigsäure in Methylenchlorid.
Ausbeute: 66,4 % der Theorie,
Schmelzpunkt: 193-194°C

| $C_{36}H_{39}N_3O_4S$ (609,78) | | | | |
|---|---|---|---|---|
| Ber.: | C 70,91 | H 6,45 | N 6,89 | S 5,26 |

(fortgesetzt)

| $C_{36}H_{39}N_3O_4S$ (609,78) | | | | |
|---|---|---|---|---|
| Gef.: | 70,76 | 6,54 | 6,94 | 5,40 |

$R_f$-Wert: 0,25 (Kieselgel; Laufmittel: Essigester/Ethanol/ Ammoniak = 80:40:2)

Beispiel 29

4'-[[2-n-Butyl-6-(N-6,7-dimethoxynaphthalin-2-sulfonyl-methylamino]benzimidazol-1-yl]methyl]biphenyl-2-carbonsäure

Hergestellt analog Beispiel 1 aus 4'-[[2-n-Butyl-6-(N-6,7-dimethoxynaphthalin-2-sulfonyl-methylamino)-benzimidazol-1-yl]methyl]biphenyl-2-carbonsäure-tert.butylester und Trifluoressigsäure in Methylenchlorid.
Ausbeute: 87,0 % der Theorie,
Schmelzpunkt: 261-262°C

| $C_{38}H_{37}N_3O_6S$ (663,79) | | | | |
|---|---|---|---|---|
| Ber.: | C 68,76 | H 5,62 | N 6,33 | S 4,83 |
| Gef.: | 69,00 | 6,00 | 6,15 | 5,07 |

$R_f$-Wert: 0,23 (Kieselgel; Laufmittel: Essigester/Ethanol/ Ammoniak = 80:40:2)

Beispiel 30

4'-[[2-n-Butyl-6-(2-oxo-3,4-tetramethylen-pyrrolidin-1-yl)benzimidazol-1-yl]methyl]biphenyl-2-carbonsäure

Hergestellt analog Beispiel 1 aus 4'-[(2-n-Butyl-6-(2-oxo-3,4-tetramethylen-pyrrolidin-1-yl)-benzimidazol-1-yl]methyl]biphenyl-2-carbonsäure-tert.butylester und Trifluoressigsäure in Methylenchlorid.
Ausbeute: 38,0 % der Theorie,
Schmelzpunkt: 146-148°C
$C_{33}H_{33}N_3O_3$ (519,65)
$R_f$-Wert: 0,30 (Kieselgel; Laufmittel: Methylenchlorid/ Ethanol = 9:1)

Beispiel 31

4'-[[2-n-Butyl-5-(2-oxo-3,4-tetramethylen-pyrrolidin-1-yl)benzimidazol-1-yl]methyl]biphenyl-2-carbonsäure

Hergestellt analog Beispiel 1 aus 4'-[[2-n-Butyl-5-(2-oxo-3,4-tetramethylen-pyrrolidin-1-yl)-benzimidazol-1-yl]methyl]biphenyl-2-carbonsäure-tert.butylester und Trifluoressigsäure in Methylenchlorid.
Ausbeute: 15,5 % der Theorie,
Schmelzpunkt: amorph
$C_{33}H_{33}N_3O_3$ (519,65)
$R_f$-Wert: 0,20 (Kieselgel; Laufmittel: Methylenchlorid/ Ethanol = 9:1)

Beispiel 32

4'-[[2-n-Butyl-6-(3,3-dimethylpiperidin-1-yl)-benzimidazol-1-yl]methyl]biphenyl-2-carbonsäure

Hergestellt analog Beispiel 1 aus 4'-[[2-n-Butyl-6-(3,3-dimethylpiperidin-1-yl)-benzimidazol-1-yl]methyl]biphenyl-2-carbonsäure-tert.butylester und Trifluoressigsäure in Methylenchlorid.
Ausbeute: 86 % der Theorie,
Schmelzpunkt: ab 120°C (sintern)

| $C_{32}H_{37}N_3O_2$ (495,70) | | | |
|---|---|---|---|
| Ber.: | C 77,54 | H 7,52 | N 8,48 |
| Gef.: | 77,54 | 7,24 | 8,19 |

$R_f$-Wert: 0,35 (Kieselgel; Laufmittel: Methylenchlorid/ Ethanol = 9:1)

Beispiel 33

4'-[[2-n-Butyl-6-heptamethylenimino-benzimidazol-1-yl)-methyl]biphenyl-2-carbonsäure

Hergestellt analog Beispiel 1 aus 4'-[(2-n-Butyl-6-heptamethylenimino-benzimidazol-1-yl]methyl]biphenyl-2-carbonsäure-tert.butylester und Trifluoressigsäure.
Ausbeute: 71 % der Theorie,
Schmelzpunkt: 195-198°C

| $C_{32}H_{37}N_3O_2$ (495,60) | | | |
|---|---|---|---|
| Ber.: | C 77,55 | H 7,52 | N 8,48 |
| Gef.: | 77,40 | 7,66 | 8,23 |

$R_f$-Wert: 0,40 (Kieselgel; Laufmittel: Methylenchlorid/ Ethanol = 9:1)

Beispiel 34

4'-[[2-n-Butyl-6-(piperidin-1-yl)-benzimidazol-1-yl]methyl]biphenyl-2-carbonsäure

Hergestellt analog Beispiel 1 aus 4'-[[2-n-Butyl-6-(piperidin-1-yl)-benzimidazol-1-yl]methyl]-biphenyl-2-carbonsäure-tert.butylester und Trifluoressigsäure.
Ausbeute: 84 % der Theorie,
Schmelzpunkt: 199-200°C

| $C_{30}H_{33}N_3O_2$ (467,60) | | | |
|---|---|---|---|
| Ber.: | C 77,06 | H 7,11 | N 8,99 |
| Gef.: | 76,85 | 7,28 | 9,02 |

$R_f$-Wert: 0,40 (Kieselgel; Laufmittel: Methylenchlorid/ Ethanol = 9:1)

Beispiel 35

4'-[[2-n-Butyl-6-(4-methylpiperidin-1-yl)-benzimidazol-1-yl]methyl]-biphenyl-2-carbonsäure

Hergestellt analog Beispiel 1 aus 4'-[[2-n-Butyl-6-(4-methylpiperidin-1-yl)-benzimidazol-1-yl]methyl]-biphenyl-2-carbonsäure-tert.butylester und Trifluoressigsäure.
Ausbeute: 82 % der Theorie,
Schmelzpunkt: 162-165°C

| $C_{31}H_{35}N_3O_2$ (481,60) | | | |
|---|---|---|---|
| Ber.: | C 77,31 | H 7,33 | N 8,73 |
| Gef.: | 77,20 | 7,19 | 8,63 |

$R_f$-Wert: 0,40 (Kieselgel; Laufmittel: Methylenchlorid/ Ethanol = 9:1)

Beispiel 36

4'-[(2-n-Butyl-6-hexamethylenimino-benzimidazol-1-yl]methyl]biphenyl-2-carbonsäure

Hergestellt analog Beispiel 1 aus 4'-[(2-n-Butyl-6-hexamethylenimino-benzimidazol-1-yl)-methyl]-biphenyl-2-carbonsäure-tert.butylester und Trifluoressigsäure.
Ausbeute: 34 % der Theorie,
Schmelzpunkt: 197-199°C

| $C_{31}H_{35}N_3O_2$ (481,60) | | | |
|---|---|---|---|
| Ber.: | C 77,31 | H 7,33 | N 8,73 |
| Gef.: | 76,99 | 7,35 | 8,62 |

$R_f$-Wert: 0,40 (Kieselgel; Laufmittel: Methylenchlorid/ Ethanol = 9:1)

Beispiel 37

4'-[[2-n-Propyl-6-(2-oxo-piperidin-1-yl)-benzimidazol-1-yl]methyl]biphenyl-2-carbonsäure

Hergestellt analog Beispiel 1 aus 4'-[[2-n-Propyl-6-(2-oxo-piperidin-1-yl)-benzimidazol-1-yl]methyl]biphenyl-2-carbonsäure-tert.butylester und Trifluoressigsäure.
Ausbeute: 60 % der Theorie,
Schmelzpunkt: 208-210°C

| $C_{29}H_{29}N_3O_3$ (467,60) | | | |
|---|---|---|---|
| Ber.: | C 74,49 | H 6,25 | N 8,99 |
| Gef.: | 74,00 | 6,29 | 8,90 |

$R_f$-Wert: 0,50 (Kieselgel; Laufmittel: Methylenchlorid/ Ethanol = 9:1)

Beispiel 38

4'-[[2-n-Propyl-6-(propansultam-1-yl)-benzimidazol-1-yl]methyl]biphenyl-2-carbonsäure

Hergestellt analog Beispiel 1 aus 4'-[[2-n-Propyl-6-(propansultam-1-yl)-benzimidazol-1-yl]methyl]biphenyl-2-carbonsäure-tert.butylester und Trifluoressigsäure.
Ausbeute: 49 % der Theorie,
Schmelzpunkt: amorph

| $C_{27}H_{27}N_3O_4S$ (489,58) | | | | |
|---|---|---|---|---|
| Ber.: | C 66,23 | H 5,56 | N 8,56 | S 6,55 |
| Gef.: | 66,08 | 5,50 | 8,37 | 6,51 |

$R_f$-Wert: 0,47 (Kieselgel; Laufmittel: Methylenchlorid/ Ethanol = 9:1)
Massenspektrum: $(M + H)^+ = 490$

Beispiel 39

4'-[[2-n-Propyl-6-(butansultam-1-yl)-benzimidazol-1-yl]methyl]biphenyl-2-carbonsäure

Hergestellt analog Beispiel 1 aus 4'-[[2-n-Propyl-6-(butansultam-1-yl)-benzimidazol-1-yl]methyl]biphenyl-2-carbonsäure-tert.butylester und Trifluoressigsäure.
Ausbeute: 57 % der Theorie,
Schmelzpunkt: amorph

| $C_{28}H_{29}N_3O_4S$ (503,63) | | | | |
|---|---|---|---|---|
| Ber.: | C 66,77 | H 5,80 | N 8,34 | S 6,37 |
| Gef.: | 66,59 | 5,77 | 8,18 | 6,33 |

$R_f$-Wert: 0,51 (Kieselgel; Laufmittel: Methylenchlorid/ Ethanol = 9:1)
Massenspektrum: $(M + H)^+ = 504$

Beispiel 40

4'-[[2-n-Butyl-6-(butansultam-1-yl)-benzimidazol-1-yl]methyl]biphenyl-2-carbonsäure

Hergestellt analog Beispiel 1 aus 4'-[[2-n-Butyl-6-(butansultam-1-yl)-benzimidazol-1-yl]methyl]biphenyl-2-carbonsäure-tert.butylester und Trifluoressigsäure.
Ausbeute: 51 % der Theorie,
Schmelzpunkt: 203-205°C

| $C_{29}H_{31}N_3O_4S$ (517,63) | | | | |
|---|---|---|---|---|
| Ber.: | C 67,29 | H 6,04 | N 8,12 | S 6,19 |
| Gef.: | 67,22 | 5,97 | 7,97 | 6,10 |

$R_f$-Wert: 0,52 (Kieselgel; Laufmittel: Methylenchlorid/ Ethanol = 9:1)
Massenspektrum: $(M + H)^+ = 518$

Beispiel 41

4'-[[2-n-Butyl-6-(benzoxazol-2-yl)-benzimidazol-1-yl]methyl]-2-(1H-tetrazol-5-yl)-biphenyl

a) 2-n-Butyl-5-(benzoxazol-2-yl)-benzimidazol

Zu einer Suspension von 2,52 g (10 mMol) 2-n-Butyl-benzimidazol-5-carbonsäure in 15 ml N-Methylpyrrolidinon werden bei 10°C unter Rühren 1,43 g (12 mMol) Thionylchlorid zugetropft. Bei Raumtemperatur wird weitere 15 Minuten nachgerührt, dann 1,31 g (11 mMol) 2-Aminophenol hinzugefügt und 2 Stunden lang auf 140°C erhitzt. Das Gemisch wird dann auf ca. 50 g Eis gegossen und unter Rühren 5 ml 30%ige Natronlauge hinzugefügt. Das ausgefallene Rohprodukt wird abgesaugt und durch Säulenchromatographie (300 g Kieselgel; Laufmittel: Methylenchlorid + 3 % Ethanol) gereinigt.
Ausbeute: 1,2 g (41 % der Theorie),
Schmelzpunkt: 118-120°C

| $C_{18}H_{17}N_3O$ (291,36) | | | |
|---|---|---|---|
| Ber.: | C 74,20 | H 5,88 | N 14,42 |
| Gef.: | 73,98 | 5,97 | 14,20 |

b) Isomerengemisch aus 4'-[[2-n-Butyl-6-(benzoxazol-2-yl)-benzimidazol-1-yl]methyl]biphenyl-2-carbonsäurenitril und 4'-[[2-n-Butyl-5-(benzoxazol-2-yl)-benzimidazol-1-yl]methyl]biphenyl-2-carbonsäurenitril

Eine Lösung aus 1 g (3,43 mMol) 2-n-Butyl-5-(benzoxazol-2-yl)-benzimidazol und 0,98 g (3,60 mMol) 4'-Brommethyl-biphenyl-2-carbonsäurenitril in 20 ml Dimethylsulfoxid wird mit 0,41 g (3,6 mMol) Kalium-tert.butylat versetzt und 48 Stunden lang bei Raumtemperatur gerührt. Das Gemisch wird dann auf 100 ml Wasser gegossen, mit Natriumchlorid gesättigt und dreimal mit je 30 ml Essigester extrahiert. Durch Säulenchromatographie (200 g Kieselgel; Laufmittel: Essigester/Petrolether (1:1)) erhält man 1,4 g (85 % der Theorie) eines Gemisches der Isomeren im Verhältnis 1:1, das ab 130°C zu sintern beginnt.

| $C_{32}H_{26}N_4O$ (482,59) | | | |
|---|---|---|---|
| Ber.: | C 79,64 | H 5,43 | N 11,61 |
| Gef.: | 79,64 | 5,36 | 11,59 |

c) 4'-[[2-n-Butyl-6-(benzoxazol-2-yl)-benzimidazol-1-yl]methyl]-2-(1H-tetrazol-5-yl)-biphenyl

Eine Lösung des (1:1)-Isomerengemisches von 4'-[[2-n-Butyl-6-(benzoxazol-2-yl)-benzimidazol-1-yl]methyl] biphenyl-2-Carbonsäurenitril und 4'-[[2-n-Butyl-5-(benzoxazol-2-yl)-benzimidazol-1-yl]methyl]biphenyl-2-carbonsäurenitril in 20 ml Dimethylformamid wird mit 2 g Ammoniumchlorid und 2 g Natriumazid versetzt und 4 Stunden lang auf 120-130°C erhitzt. Nach nochmaliger Zugabe von 2 g Ammoniumchlorid und 2 g Natriumazid und weiteren 18 Stunden bei 120-130°C wird das Gemisch auf 100 ml Wasser gegossen. Das ausgefallene Produktgemisch wird abgesaugt und durch Säulenchromatographie (300 g Kieselgel, Laufmittel: Methylenchlorid + 3 % Ethanol) getrennt.

Ausbeute: 100 mg (20 % der Theorie) in amorpher Form.

| $C_{32}H_{27}N_7O$ (525,62) | | | |
|---|---|---|---|
| Ber.: | C 73,12 | H 5,18 | N 18,66 |
| Gef.: | 73,10 | 5,50 | 18,42 |

$R_f$-Wert: 0,75 (Kieselgel; Laufmittel: Methylenchlorid/ Ethanol = 9:1)

Analog Beispiel 41 werden folgende Verbindungen erhalten:

4'-[[2-n-Butyl-6-(4,5-dihydro-2H-pyridazin-3-on-6-yl)-benzimidazol-1-yl]methyl]-2-(1H-tetrazol-5-yl)-biphenyl

4'-[[2-n-Propyl-6-(4,5-dihydro-2H-pyridazin-3-on-6-yl)-benzimidazol-1-yl]methyl]-2-(1H-tetrazol-5-yl)-biphenyl

4'-[[2-Ethyl-6-(4,5-dihydro-2H-pyridazin-3-on-6-yl)-benzimidazol-1-yl]methyl]-2-(1H-tetrazol-5-yl)-biphenyl

4'-[[2-n-Butyl-6-(2H-pyridazin-3-on-6-yl)-benzimidazol-1-yl]-methyl]-2-(1H-tetrazol-5-yl)-biphenyl

4'-[[2-n-Propyl-6-(2H-pyridazin-3-on-6-yl)-benzimidazol-1-yl]-methyl]-2-(1H-tetrazol-5-yl)-biphenyl

4'-[[2-Ethyl-6-(2H-pyridazin-3-on-6-yl)-benzimidazol-1-yl]-methyl]-2-(1H-tetrazol-5-yl)-biphenyl

4'-[[2-n-Propyl-6-(2-methyl-4,5-dihydro-pyridazin-3-on-6-yl)-benzimidazol-1-yl]methyl]-2-(1H-tetrazol-5-yl)-biphenyl

4'-[[2-n-Propyl-6-(2-benzyl-4,5-dihydro-pyridazin-3-on-6-yl)-benzimidazol-1-yl]methyl]-2-(1H-tetrazol-5-yl)-biphenyl

4'-[[2-n-Butyl-6-(1-methyl-imidazolin-2-yl)-benzimidazol-1-yl]methyl]-2-(1H-tetrazol-5-yl)-biphenyl

4'-[[2-n-Propyl-6-(1-n-hexyl-imidazolin-2-yl)-benzimidazol-1-yl]methyl]-2-(1H-tetrazol-5-yl)-biphenyl

4'-[[2-n-Butyl-6-(1-n-butyl-imidazolin-2-yl)-benzimidazol-1-yl]methyl]-2-(1H-tetrazol-5-yl)-biphenyl

4'-[[2-n-Propyl-6-(1-cyclopropyl-imidazolin-2-yl)-benzimidazol-1-yl]methyl]-2-(1H-tetrazol-5-yl)-biphenyl

4'-[[2-n-Propyl-6-(1-cyclohexyl-imidazolin-2-yl)-benzimidazol-1-yl]methyl]-2-(1H-tetrazol-5-yl)-biphenyl

4'-[[2-n-Propyl-6-(1-methyl-imidazol-2-yl)-benzimidazol-1-yl]methyl]-2-(1H-tetrazol-5-yl)-biphenyl

4'-[[2-n-Butyl-6-(1-methyl-imidazol-2-yl)-benzimidazol-1-yl]-methyl]-2-(1H-tetrazol-5-yl)-biphenyl

4'-[[2-n-Propyl-6-(1-n-propyl-imidazol-2-yl)-benzimidazol-1-yl]methyl]-2-(1H-tetrazol-5-yl)-biphenyl

4'-[[2-n-Propyl-6-(1-n-hesyl-imidazol-2-yl)-benzimidazol-1-yl]methyl]-2-(1H-tetrazol-5-yl)-biphenyl

4'-[[2-n-Butyl-6-(1-n-butyl-imidazol-2-yl)-benzimidazol-1-yl]methyl]-2-(1H-tetrazol-5-yl)-biphenyl

4'-[[2-n-Propyl-6-(1-cyclopropyl-imidazol-2-yl)-benzimidazol-1-yl]methyl]-2-(1H-tetrazol-5-yl)-biphenyl

4'-[[2-n-Propyl-6-(1-cyclohexyl-imidazol-2-yl)-benzimidazol-1-yl]methyl]-2-(1H-tetrazol-5-yl)-biphenyl

Beispiel 42

4'-[[2-n-Propyl-5-(1-methylbenzimidazol-2-yl)-benzimidazol-1-yl]methyl]-2-(1H-tetrazol-5-yl)-biphenyl und 4'-[[2-n-Propyl-6-(1-methylbenzimidazol-2-yl)-benzimidazol-1-yl]methyl]-2-(1H-tetrazol-5-yl)-biphenyl

Hergestellt analog Beispiel 41 aus einem Gemisch aus 4'-[[2-n-Propyl-5-(1-methylbenzimidazol-2-yl)-benzimidazol-1-yl]-methyl]biphenyl-2-carbonsäurenitril und 4'-[[2-n-Propyl-6-(1-methylbenzimidazol-2-yl)-benzimidazol-1-yl]-methyl]-biphenyl-2-carbonsäurenitril und Natriumazid in Dimethylformamid.
5-Isomer:
Ausbeute: 29 % der Theorie,
Schmelzpunkt: amorph

| $C_{32}H_{28}N_8$ (524,61) | | | |
|---|---|---|---|
| Ber.: | C 73,26 | H 5,38 | N 21,36 |
| Gef.: | 73,03 | 5,22 | 21,26 |

Massenspektrum: $(M + H)^+ = 525$
6-Isomer:
Ausbeute: 34 % der Theorie,
Schmelzpunkt: 198-200°C

| $C_{32}H_{28}N_8$ (524,61) | | | |
|---|---|---|---|
| Ber.: | C 73,26 | H 5,38 | N 21,36 |
| Gef.: | 73,11 | 5,27 | 21,19 |

Massenspektrum: $(M + H)^+ = 525$

Beispiel 43

4'-[[2-n-Butyl-6-(1-methylbenzimidazol-2-yl)-benzimidazol-1-yl]methyl]-2-(1H-tetrazol-5-yl)-biphenyl

Hergestellt analog Beispiel 41 aus 4'-[[2-n-Butyl-6-(1-methylbenzimidazol-2-yl)-benzimidazol-1-yl]-methyl]biphenyl-2-carbonsäurenitril und Natriumazid in Dimethylformamid. Ausbeute: 28 % der Theorie,
Schmelzpunkt: 224-226°C

| $C_{33}H_{30}N_8$ (538,63) | | | |
|---|---|---|---|
| Ber.: | C 73,58 | H 5,61 | N 20,81 |
| Gef.: | 73,31 | 5,73 | 19,99 |

$R_f$-Wert: 0,76 (Kieselgel; Laufmittel: Methylenchlorid/ Ethanol = 9:1)
Massenspektrum: $(M + H)^+ = 539$

Beispiel 44

4'-[[2-n-Butyl-6-(2-oxo-piperidin-1-yl)-benzimidazol-1-yl]-methyl]-2-(1H-tetrazol-5-yl)-biphenyl

Hergestellt analog Beispiel 41 aus 4'-[[2-n-Butyl-6-(2-oxo-piperidin-1-yl)-benzimidazol-1-yl]-methyl]biphenyl-2-carbonsäurenitril und Natriumazid in Dimethylformamid. Ausbeute: 20 % der Theorie,
Schmelzpunkt: amorph

$C_{30}H_{31}N_7O$ (505,63)

| | | | | | | |
|---|---|---|---|---|---|---|
| Ber.: | C | 67,94 | H | 6,23 | N | 17,33 |
| Gef.: | | 67,67 | | 6,13 | | 17,52 |

$R_f$-Wert: 0,30 (Kieselgel; Laufmittel: Methylenchlorid/ Ethanol = 9:1)

Beispiel 45

4'-[[2-n-Butyl-6-(3,3-dimethylpiperidin-1-yl)-benzimidazol-1-yl]-methyl]-2-(1H-tetrazol-5-yl)-biphenyl-hydrochlorid

Hergestellt analog Beispiel 41 aus 4'-[[2-n-Butyl-6-(3,3-dimethylpiperidin-1-yl)-benzimidazol-1-yl]-methyl]-2-carbonsäurenitril und Natriumazid in Dimethylformamid.
Ausbeute: 8 % der Theorie,
Schmelzpunkt: ab 148°C sintern
$C_{32}H_{37}N_7$ x HCl (519,70)
Massenspektrum: $(M+H)^+ = 520$

Beispiel 46

4'-[[2-n-Butyl-6-(4,4-tetramethylenglutarimido)-benzimidazol-1-yl]-methyl]-4-chlor-2-(1H-tetrazol-5-yl)-biphenyl

Hergestellt analog Beispiel 41 aus 4'-[[2-n-Butyl-6-(4,4-tetramethylenglutarimido)-benzimidazol-1-yl]-methyl]-4-chlorbiphenyl-2-carbonsäurenitril und Natriumazid in Dimethylformamid.
Ausbeute: 40 % der Theorie,
Schmelzpunkt: ab 160°C sintern

| $C_{34}H_{34}N_7O_2Cl$ (608,16) | | | |
|---|---|---|---|
| Ber.:<br>Gef.: | C 67,15<br>66,90 | H 5,64<br>5,86 | N 16,12<br>15,86 |

$R_f$-Wert: 0,50 (Kieselgel; Laufmittel: Methylenchlorid/Ethanol = 9:1)

Beispiel 47

4'-[[2-n-Butyl-6-(propansultam-1-yl)-benzimidazol-1-yl]-methyl]-2-(1H-tetrazol-5-yl)-biphenyl

Hergestellt analog Beispiel 41 aus 4'-[[2-n-Butyl-6-(propansultam-1-yl)-benzimidazol-1-yl]-methyl]biphenyl-2-carbonsäurenitril und Natriumazid in Dimethylformamid.
Ausbeute: 46 % der Theorie,
Schmelzpunkt: 203-205°C

| $C_{28}H_{29}N_7O_2S$ (527,70) | | | | |
|---|---|---|---|---|
| Ber.:<br>Gef.: | C 63,73<br>62,52 | H 5,54<br>5,56 | N 18,58<br>18,40 | S 6,08<br>6,00 |

$R_f$-Wert: 0,35 (Kieselgel; Laufmittel: Methylenchlorid/ Ethanol = 9:1)

Beispiel 48

4'-[[2-n-Butyl-6-(butansultam-1-yl)-benzimidazol-1-yl]-methyl]-2-(1H-tetrazol-5-yl)-biphenyl

Hergestellt analog Beispiel 41 aus 4'-[[2-n-Butyl-6-(butansultam-1-yl)-benzimidazol-1-yl]-methyl]biphenyl-2-carbonsäurenitril und Natriumazid in Dimethylformamid.
Ausbeute: 30 % der Theorie,
Schmelzpunkt: 189-191°C

| $C_{29}H_{31}N_7O_2S$ (541,70) | | | | |
|---|---|---|---|---|
| Ber.: | C 64,30 | H 5,95 | N 18,10 | S 5,92 |
| Gef.: | 64,40 | 5,75 | 17,90 | 5,85 |

$R_f$-Wert: 0,37 (Kieselgel; Laufmittel: Methylenchlorid/ Ethanol = 9:1)

Beispiel 49

4'-[[2-n-Propyl-6-(butansultam-1-yl)-benzimidazol-1-yl]-methyl]-2-(1H-tetrazol-5-yl)-biphenyl

Hergestellt analog Beispiel 41 aus 4'-[[2-n-Propyl-6-(butansultam-1-yl)-benzimidazol-1-yl]-methyl]biphenyl-2-carbonsäurenitril und Natriumazid in Dimethylformamid.
Ausbeute: 37 % der Theorie,
Schmelzpunkt: 204-206°C

| $C_{28}H_{29}N_7O_2S$ (527,63) | | | | |
|---|---|---|---|---|
| Ber.: | C 63,73 | H 5,54 | N 18,58 | S 6,08 |
| Gef.: | 63,70 | 5,49 | 18,37 | 6,19 |

$R_f$-Wert: 0,36 (Kieselgel; Laufmittel: Methylenchlorid/ Ethanol = 9:1)
Massenspektrum: m/e = 527

Beispiel 50

4'-[[2-n-Butyl-6-(2-oxo-pyrrolidin-1-yl)-benzimidazol-1-yl]-methyl]-2-(1H-tetrazol-5-yl)-biphenyl

Hergestellt analog Beispiel 41 aus 4'-[[2-n-Butyl-6-(2-oxo-pyrrolidin-1-yl)-benzimidazol-1-yl]methyl]biphenyl-2-carbonsäurenitril und Natriumazid in Dimethylformamid.
Ausbeute: 15 % der Theorie,
Schmelzpunkt: 153-155°C

| $C_{29}H_{29}N_7O$ (491,60) | | | |
|---|---|---|---|
| Ber.: | C 70,85 | H 5,95 | N 19,95 |
| Gef.: | 70,79 | 6,17 | 19,71 |

$R_f$-Wert: 0,45 (Kieselgel; Laufmittel: Methylenchlorid/ Ethanol = 9:1)
Massenspektrum: $(M + H)^+ = 492$

Beispiel 51

4'-[[2-n-Butyl-6-(2-oxo-hexamethylenimino)-benzimidazol-1-yl]-methyl]-2-(1H-tetrazol-5-yl)-biphenyl

Hergestellt analog Beispiel 41 aus 4'-[[2-n-Butyl-6-(2-oxo-hexamethylenimino)-benzimidazol-1-yl]-methyl]biphenyl-2-carbonsäurenitril und Natriumazid in Dimethylformamid. Ausbeute: 34 % der Theorie,
Schmelzpunkt: amorph

| $C_{31}H_{33}N_7O$ (519,70) | | | |
|---|---|---|---|
| Ber.: | C 71,65 | H 6,40 | N 18,87 |
| Gef.: | 70,99 | 6,32 | 18,75 |

$R_f$-Wert: 0,15 (Kieselgel; Laufmittel: Methylenchlorid/ Ethanol = 9:1)

Beispiel 52

4'-[[2-n-Propyl-6-(2-oxo-piperidin-1-yl)-benzimidazol-1-yl]-methyl]-2-(1H-tetrazol-5-yl)-biphenyl

Hergestellt analog Beispiel 41 aus 4'-[[2-n-Propyl-6-(2-oxo-piperidin-1-yl)-benzimidazol-1-yl]-methyl]biphenyl-2-carbonsäurenitril und Natriumazid in Dimethylformamid.
Ausbeute: 14,5 % der Theorie,
Schmelzpunkt: ab 125°C sintern
$C_{29}H_{29}N_7O$ (491,60)
$R_f$-Wert: 0,25 (Kieselgel; Laufmittel: Methylenchlorid/ Ethanol = 9:1)
Massenspektrum: $(M + H)^+ = 492$

Beispiel 53

4'-[[2-n-Butyl-6-(3,3-dimethylglutarimido)-benzimidazol-1-yl]methyl]-2-(1H-tetrazol-5-yl)-biphenyl

a) 4'-[[2-n-Butyl-6-(3,3-dimethylglutarimido)-benzimidazol-1-yl]methyl]-2-(1-triphenylmethyl-tetrazol-5-yl)-biphenyl

Zu einer Lösung von 1,04 g (3,3 mMol) 2-n-Butyl-5-(3,3-dimethylglutarimido)-benzimidazol und 425 mg (3,8 mMol) Kalium-tert.butylat in 25 ml Dimethylsulfoxid werden 1,8 g (3,3 mMol) 4'-Brommethyl-2-(1-triphenylmethyl-tetrazol5-yl)-biphenyl gegeben. Das Gemisch wird 3 Stunden lang bei Raumtemperatur gerührt, dann in 150 ml Wasser eingerührt, dreimal mit je 30 ml Essigester extrahiert, die organischen Extrakte getrocknet und eingeengt. Der erhaltene Rückstand wird durch Säulenchromatographie (300 g Kieselgel; Laufmittel: Essigester/Petrolether (2:1)) gereinigt.
Ausbeute: 400 mg (15 % der Theorie),
$R_f$-Wert: 0,38 (Essigester/Petrolether = 6:1)

b) 4'-[[2-n-Butyl-6-(3,3-dimethylglutarimido)-benzimidazol-1-yl]methyl]-2-(1H-tetrazol-5-yl)-biphenyl

Eine Lösung von 400 mg (0,5 mMol) 4'-[[2-n-Butyl-6-(3,3-dimethylglutarimido)-benzimidazol-1-yl)methyl]-2-(1-triphenylmethyl-tetrazol-5-yl)-biphenyl in 10 ml Methanol wird mit 1,5 ml methanolischer Salzsäure versetzt und 2 Stunden lang bei Raumtemperatur gerührt, dann eingeengt, der Rückstand mit 15 ml Wasser versetzt und mit konzentriertem Ammoniak alkalisch gestellt, wobei das Produkt in Lösung geht. Durch Ansäurern mit Eisessig wird das Rohprodukt ausgefällt, das dann durch Säulenchromatographie gereinigt wird (150 g Kieselgel; Laufmittel: Methylenchlorid + 5 % Ethanol). Ausbeute: 150 mg (55 % der Theorie),
Schmelzpunkt: 184-186°C

| $C_{32}H_{33}N_7O_2$ (547,70) | | | |
|---|---|---|---|
| Ber.: | C 70,18 | H 6,07 | N 17,90 |
| Gef.: | 69,98 | 6,20 | 17,67 |

Beispiel 54

4'-[[2-n-Butyl-6-(N-methylaminocarbonyl-n-pentylamino)-benzimidazol-1-yl]methyl]-2-(1H-tetrazol-5-yl)-biphenyl

Hergestellt analog Beispiel 53 aus 4'-[[2-n-Butyl-6-(N-methylaminocarbonyl-n-pentylamino-benzimidazol-1-yl]methyl]-2-(1-triphenylmethyl-tetrazol-5-yl)-biphenyl und Salzsäure in Ethanol.
Ausbeute: 53,8 % der Theorie,
Schmelzpunkt: 124-126°C

| $C_{33}H_{38}N_8O$ (550,71) | | | |
|---|---|---|---|
| $R_f$-Wert: | 0,25 (Kieselgel; Laufmittel: Methylenchlorid/ Ethanol = 9:1) | | |
| Ber.: | C 69,79 | H 6,95 | N 20,35 |
| Gef.: | 69,78 | 7,05 | 20,31 |

Massenspektrum: $(M + H)^+ = 492$

Beispiel 55

4'-[[2-n-Butyl-5-(N-methylaminocarbonyl-n-pentylamino)-benzimidazol-1-yl]methyl]-2-(1H-tetrazol-5-yl)-biphenyl-dihydrat

Hergestellt analog Beispiel 53 aus 4'-[[2-n-Butyl-5-(N-methylaminocarbonyl-n-pentylamino)-benzimidazol-1-yl]methyl]-2-(1-triphenylmethyl-tetrazol-5-yl)-biphenyl und Salzsäure in Ethanol.
Ausbeute: 76,2 % der Theorie,
Schmelzpunkt: 201-203°C

| $C_{32}H_{38}N_8O$ x 2 $H_2O$ (586,74) | | | |
|---|---|---|---|
| Ber.: | C 65,50 | H 7,21 | N 19,09 |
| Gef.: | 65,43 | 7,07 | 19,12 |

Beispiel 56

4'-[[2-n-Butyl-6-(N-cyclohexylaminocarbonyl-n-pentylamino)-benzimidazol-1-yl]methyl]-2-(1H-tetrazol-5-yl)-biphenyl-hydrat

Hergestellt analog Beispiel 53 aus 4'-[[2-n-Butyl-6-(N-cyclohexylaminocarbonyl-n-pentylamino)-benzimidazol-1-yl]methyl]-2-(1-triphenylmethyl-tetrazol-5-yl)-biphenyl und Salzsäure in Ethanol.
Ausbeute: 95,2 % der Theorie,
Schmelzpunkt: 128-132°C

| $C_{37}H_{46}N_8O$ x $H_2O$ (636,84) | | | |
|---|---|---|---|
| Ber.: | C 69,78 | H 7,59 | N 17,59 |
| Gef.: | 69,61 | 7,71 | 17,41 |

$R_f$-Wert: 0,45 (Kieselgel; Laufmittel: Ethanol/Ammoniak = 80/40/2)

Beispiel 57

4'-[[2-n-Butyl-5-(N-cyclohexylaminocarbonyl-n-pentylamino)-benzimidazol-1-yl]methyl]-2-(1H-tetrazol-5-yl)-biphenyl-hydrat

Hergestellt analog Beispiel 53 aus 4'-[[2-n-Butyl-5-(N-cyclohexylaminocarbonyl-n-pentylamino)-benzimidazol-1-yl]methyl]-2-(1-triphenylmethyl-tetrazol-5-yl)-biphenyl und Salzsäure in Ethanol.
Ausbeute: 88,6 % der Theorie,
Schmelzpunkt: 117-120°C

| $C_{37}H_{46}N_8O$ x $H_2O$ (636,84) | | | |
|---|---|---|---|
| Ber.: | C 69,78 | H 7,59 | N 17,59 |
| Gef.: | 70,06 | 7,58 | 17,56 |

$R_f$-Wert: 0,45 (Kieselgel; Laufmittel: Ethanol/Ammoniak = 80/40/2)

Beispiel 58

4'-[[2-n-Butyl-6-(5-dimethylaminonapththalin-1-sulfonamino)-benzimidazol-1-yl]methyl]-2-(1H-tetrazol-5-yl)-biphenyl-hydrat

Hergestellt analog Beispiel 53 aus 4'-[[2-n-Butyl-6-(5-dimethylaminonapththalin-1-sulfonamino)-benzimidazol-1-yl]methyl]-2-(1-triphenylmethyl-tetrazol-5-yl)-biphenyl und Salzsäure in Ethanol.
Ausbeute: 44,7 % der Theorie,

| $C_{37}H_{36}N_8O_2S$ x $H_2O$ (674,81) | | | | |
|---|---|---|---|---|
| Ber.: | C 65,85 | H 5,67 | N 16,60 | S 4,75 |
| Gef.: | 65,80 | 5,46 | 16,42 | 4,90 |

Beispiel 59

4'-[[2-n-Butyl-6-(2-oxo-3,4-tetramethylen-pyrrolidin-1-yl)-benzimidazol-1-yl]methyl]biphenyl-2-carbonsäure

420 mg (0,81 mMol) 4'-[[2-n-Butyl-6-(2-oxo-1,2-dihydro-3,4-tetramethylen-pyrrol-1-yl)-benzimidazol-1-yl]methyl] biphenyl-2-carbonsäure werden in 60 ml Methanol und 60 ml Essigester gelöst und unter Zusatz von 200 mg Palladium auf Kohle (10%ig) bei 5 bar Wasserstoff-Druck und 40°C hydriert. Der Katalysator wird abgesaugt, das Lösungsmittel abgedampft und das Rohprodukt durch Säulenchromatographie (200 g Kieselgel; Laufmittel: Methylenchlorid + 3 % Ethanol) gereinigt. Ausbeute: 260 mg (62 % der Theorie),
Schmelzpunkt: amorph

| $C_{33}H_{35}N_3O_3$ (521,67) | | | |
|---|---|---|---|
| Ber.: | C 75,98 | H 6,76 | N 8,06 |
| Gef.: | 75,75 | 6,62 | 8,24 |

Beispiel 60

Gemisch aus 4'-[[2-n-Butyl-6-(5,5-pentamethylen-oxazolin-2-yl)-benzimidazol-1-yl)-methyl]-2-(1H-tetrazol-5-yl)-biphenyl und 4'-[[2-n-Butyl-5-(5,5-pentamethylen-oxazolin-2-yl)-benzimidazol-1-yl)-methyl]-2-(1H-tetrazol-5-yl)-biphenyl

Eine Lösung aus 930 mg (2 mMol) eines Isomerengemisches aus 4'-[[2-n-Butyl-6-carboxy-benzimidazol-1-yl)-methyl]-2-(1H-tetrazol-5-yl)-biphenyl und 4'-[[2-n-Butyl-5-carboxy-benzimidazol-1-yl)-methyl]-2-(1H-tetrazol-5-yl)-biphenyl und 356 mg (2,2 mMol) Carbonyldiimidazol in 30 ml Tetrahydrofuran wird 30 Minuten lang bei Raumtemperatur gerührt. Dann werden 332 mg (2 mMol) 1-(Aminomethyl)-cyclohexanol-dihydrochlorid hinzugefügt und weitere 15 Stunden bei Raumtemperatur gerührt. Danach wird das Gemisch eingedampft, langsam 2 ml Thionylchlorid zugetropft, eine Stunde gerührt, das Thionylchlorid abdestilliert und der Rückstand mit 5 ml Eiswasser versetzt. Das unlösliche Rohprodukt wird durch Säulenchromatographie (150 g Kieselgel; Laufmittel: Methylenchlorid + 5 % Ethanol) gereinigt. Man erhält so 25 mg (2 % der Theorie) eines Gemisches aus 4'-[[2-n-Butyl-6-(5,5-pentamethylen-oxazolin-2-yl)-benzimidazol-1-yl)-methyl]-2-(1H-tetrazol-5-yl)-biphenyl
und
4'-[[2-n-Butyl-5-(5,5-pentamethylen-oxazolin-2-yl)-benzimidazol-1-yl)-methyl]-2-(1H-tetrazol-5-yl)-biphenyl.
Schmelzpunkt: ab 215°C (Zers.)
$C_{33}H_{35}N_7O$ (545,67)
Massenspektrum: $(M + H)^+ = 546$

Beispiel 61

4'-[[2-n-Butyl-6-(N-methyl-phenylaminocarbonylamino)-benzimidazol-1-yl)-methyl]biphenyl-2-carbonsäure

Eine Lösung von 0,8 g (2,00 mMol) 4'-[[2-n-Butyl-6-aminobenzimidazol-1-yl)-methyl]biphenyl-2-carbonsäure und 0,9 g N-Methyl-isatosäureanhydrid in 3 ml Pyridin wird 48 Stunden unter Rückfluß gekocht, anschließend bis zur Trockne eingedampft, der Rückstand in ca. 5 ml Methylenchlorid augeschlämmt, abgesaugt, mit weiteren 5 ml Methylenchlorid gewaschen und getrocknet.
Ausbeute: 0,66 g (62 % der Theorie),
Schmelzpunkt: 274-276°C

| $C_{33}H_{32}N_4O_3$ (532,60) | | | |
|---|---|---|---|
| Ber.: | C 74,41 | H 6,06 | N 10,57 |
| Gef.: | 74,23 | 5,94 | 10,66 |

Beispiel 62

4'-[[2-n-Butyl-6-(2,3-dimethylmaleinsäureimino)-benzimidazol-1-yl]methyl]biphenyl-2-carbonsäure

52,5 mg (0,1 mMol) 4'-[[2-n-Butyl-6-(2,3-dimethylmaleinsäureamino]benzimidazol-1-yl)-methyl]biphenyl-2-carbonsäure werden in 2 ml Bis-(2-methoxy-ethyl)-ether eine Stunde zum Sieden erhitzt. Man destilliert vom Lösungsmittel ab, und verteilt den öligen Rückstand in Essigester/Wasser. Die organische Phase wird noch zweimal mit Wasser gewaschen, mit Magnesiumsulfat getrocknet und einrotiert. Der Rückstand wird in 1 ml Aceton verrieben, abgesaugt, mit Ether nachgewaschen und im Vakuum bei 75°C getrocknet.
Ausbeute: 29,0 mg (57,2 % der Theorie),
Schmelzpunkt: 289-291°C

| $C_{31}H_{29}N_3O_4$ (507,59) | | | |
|---|---|---|---|
| Ber.: | C 73,35 | H 5,76 | N 8,29 |
| Gef.: | 73,50 | 5,64 | 8,10 |

Beispiel 63

4'-[[2-n-Butyl-6-(3,4,5,6-tetrahydro-phthalimino)-benzimidazol-1-yl]methyl]biphenyl-2-carbonsäure-hydrat

0,275 g (0,5 mMol) 4'-[[2-n-Butyl-6-(2-carboxy-3,4,5,6-tetrahydrobenzamino)-benzimidazol-1-yl]methyl]biphenyl-2-carbonsäure werden in 5 ml Pyridin 4 Stunden am Rückfluß gekocht. Man rotiert im Vakuum zur Trockene ein und kristallisiert das Rohprodukt aus Aceton um. Es wird abgesaugt, mit Aceton gewaschen und im Vakuum bei 70°C getrocknet.
Ausbeute: 0,2 g (72,4 % der Theorie),
Schmelzpunkt: 226-228°C

| $C_{30}H_{31}N_3O_4$ x $H_2O$ (551,64) | | | |
|---|---|---|---|
| Ber.: | C 71,85 | H 6,03 | N 7,62 |
| Gef.: | 71,83 | 5,90 | 7,61 |

Beispiel 64

4'-[[2-n-Butyl-6-(pyrrolidinocarbonylamino)-benzimidazol-1-yl]methyl]biphenyl-2-carbonsäure-tert.butylester

2,0 g (15 mMol) Pyrrolidinocarbonylchlorid werden in 50 ml trockenem Chloroform vorgelegt und 2,3 g (6 mMol) 4'-[(6-Amino-2-n-butyl-benzimidazol-1-yl)-methyl]biphenyl-2-carbonsäure-tert.butylester, gelöst in 50 ml trockenem Pyridin, während einer Stunde zugetropft. Die Reaktionslösung wird 24 Stunden nachgerührt und anschließend einrotiert. Der ölige Rückstand wird in Essigester und 10%iger Natriumhydrogencarbonatlösung verteilt, die organische Phase abgetrennt und nach dem Trocknen mit Magnesiumsulfat einrotiert. Die Reinigung erfolgt über eine Kieselgel-Säule (Korngröße: 0,063 - 0,2 mm) wobei mit Petrolether/Essigester = 3:7 eluiert wird. Die entsprechenden Säulenfraktionen werden einrotiert und im Vakuum bei 50°C getrocknet.
Ausbeute: 1,7 g (61,8 % der Theorie),
Schmelzpunkt: 68-70°C (amorph)
$C_{34}H_{40}N_4O_3$ (552,72)
$R_f$-Wert: 0,35 (Kieselgel; Laufmittel: Essigester/Ethanol = 19:1)

Beispiel 65

4'-[[2-n-Butyl-6-(pyrrolidinocarbonylamino)-benzimidazol-1-yl]methyl]biphenyl-2-carbonsäure-trifluoracetat-monohydrat

Hergestellt analog Beispiel 1 aus 4'-[[2-n-Butyl-6-(pyrrolidinocarbonylamino)-benzimidazol-1-yl]methyl]biphenyl-2-carbonsäure-tert.butylester und Trifluoressigsäure/Methylenchlorid.
Ausbeute: 91,7 % der Theorie),
Schmelzpunkt: 233-234°C

| $C_{30}H_{32}N_4O_3$ x $CF_3COOH$ x $H_2O$ (628,25) | | | |
|---|---|---|---|
| Ber.: | C 61,14 | H 5,61 | N 8,91 |
| Gef.: | 61,25 | 5,62 | 9,09 |

$R_f$-Wert: 0,48 (Kieselgel; Laufmittel: Essigester/Ethanol/ Ammoniak = 50:45:5)

Beispiel 66

4'-[[2-n-Butyl-6-(2,3-dimethylmaleinsäureimino)-benzimidazol-1-yl]methyl]biphenyl-2-carbonsäure

314 mg (0,5 mMol) 4'-[(6-Amino-2-n-butyl-benzimidazol-1-yl)-methyl]biphenyl-2-carbonsäure-trifluoracetat werden zusammen mit 76 mg (0,6 mMol) 2,3-Dimethylmaleinsäureanhydrid in 10 ml Pyridin 18 Stunden am Rückfluß gekocht. Anschließend rotiert man vom Lösungsmittel ab und verteilt die ölige Substanz in Essigester und 10%iger Natriumhydrogencarbonatlösung. Die organische Phase wird abgetrennt, mit Magnesiumsulfat getrocknet und nach dem Abfiltrieren einrotiert. Durch Verreiben mit Aceton und Ether erhält man ein weißkristallines Produkt, welches man nach dem Absaugen im Vakuum bei 50°C trocknet.
Ausbeute: 165 mg (65,0 % der Theorie),
Schmelzpunkt: 288-290°C

| $C_{31}H_{29}N_3O_4$ (507,59) | | | |
|---|---|---|---|
| Ber.: | C 73,35 | H 5,76 | N 8,29 |
| Gef.: | 73,14 | 5,94 | 8,32 |

Beispiel 67

4'-[(2-n-Butyl-6-hexahydrohomophthalimino-benzimidazol-1-yl)-methyl]biphenyl-2-carbonsäure

Hergestellt analog Beispiel 66 aus 4'-[(6-Amino-2-n-butyl-benzimidazol-1-yl)-methyl]biphenyl-2-carbonsäure und Hexahydrohomophthalsäureanhydrid in Pyridin.
Ausbeute: 15,3 % der Theorie,
Schmelzpunkt: 183-185°C

| $C_{34}H_{35}N_3O_4$ (549,67) | | | |
|---|---|---|---|
| Ber.: | C 74,29 | H 6,49 | N 7,64 |
| Gef.: | 74,09 | 6,47 | 7,80 |

Beispiel 68

4'-[[2-n-Butyl-6-(benzofuran-2-carbonylamino)-benzimidazol-1-yl]methyl]biphenyl-2-carbonsäure

Hergestellt analog Beispiel 66 aus 4'-[(6-Amino-2-n-butylbenzimidazol-1-yl)-methyl]biphenyl-2-carbonsäure und Benzofuran-2-carbonsäureanhydrid in Pyridin.
Ausbeute: 80,7 % der Theorie,
Schmelzpunkt: 321-323°C

| $C_{34}H_{39}N_3O_4$ (543,62) | | | |
|---|---|---|---|
| Ber.: | C 75,12 | H 5,38 | N 7,73 |
| Gef.: | 74,92 | 5,45 | 7,87 |

Beispiel 69

4'-[[2-n-Butyl-6-(3-benzyl-3,4,5,6-tetrahydro-2(1H)-pyrimidinon-1-yl)-benzimidazol-1-yl]methyl]biphenyl-2-carbonsäure-hydrat

Hergestellt analog Beispiel 1 aus 4'-[[2-n-Butyl-6-(3-benzyl-3,4,5,6-tetrahydro-2(1H)-pyrimidinon-1-yl)-benzimidazol-1-yl]-methyl]biphenyl-2-carbonsäure-tert.butylester und Trifluoressigsäure in Methylenchlorid.
Ausbeute: 42,2 % der Theorie,
Schmelzpunkt: 119-122°C

| $C_{36}H_{36}N_4O_3$ x $H_2O$ (590,72) | | | |
|---|---|---|---|
| Ber.: | C 73,20 | H 6,48 | N 9,48 |
| Gef.: | 73,11 | 6,50 | 9,67 |

Beispiel 70

4'-[[2-n-Butyl-6-(2-carboxy-cyclohexylmethylcarbonylamino)-benzimidazol-1-yl]methyl]biphenyl-2-carbonsäure-hydrat

a) 4'-[[2-n-Butyl-6-(2-carboxy-cyclohexylmethylcarbonylamino)-benzimidazol-1-yl]methyl]biphenyl-2-carbonsäure-tert.butylester

1,3 g (2,86 mMol) 4'-[(6-Amino-2-n-butyl-benzimidazol-1-yl)-methyl]biphenyl-2-carbonsäure-tert.butylester, 0,6 g (5,35 mMol) Hexahydrohomophthalsäureanhydrid und 5 ml Pyridin werden unter Rühren 3 Stunden am Rückfluß gekocht. Danach wird das Pyridin im Vakuum abrotiert, der Rückstand aus Aceton kristallisiert, mit Aceton gewaschen und im Vakuum bei 70°C getrocknet.
Ausbeute: 0,67 g (37,6 % der Theorie),
Schmelzpunkt: 227-229°C

| $C_{38}H_{45}N_3O_5$ (623,79) | | | |
|---|---|---|---|
| Ber.: | C 73,17 | H 7,27 | N 6,74 |
| Gef.: | 72,93 | 7,15 | 6,94 |

b) 4'-[[2-n-Butyl-6-(2-carboxy-cyclohexylmethylcarbonylamino)-benzimidazol-1-yl]methyl]biphenyl-2-carbonäure

0,6 g (0,06 mMol) 4'-[[2-n-Butyl-6-(2-carboxy-cyclohexylmethylcarbonylamino)-benzimidazol-1-yl]methyl]biphenyl-2-carbonsäure-tert.butylester, 30 ml Methylenchlorid und 10 ml Trifluoressigsäure werden 3 Stunden bei Raumtemperatur gerührt. Es wird mit 20 ml Methylenchlorid verdünnt, mit Wasser ausgeschüttelt, die organische Phase über Natriumsulfat getrocknet und zur Trockene eingedampft. Der Rückstand wird in Ethanol gelöst und durch Zugabe von Ammoniak alkalisch gestellt. Das Lösungsmittel wird im Vakuum abdestilliert. Die verbliebene wässrige Lösung wird mit Essigsäure angesäuert, das auskristallisierte Produkt abgesaugt, mit Wasser gewaschen und im Vakuum bei 70°C getrocknet.
Ausbeute: 0,55 g (98,2 % der Theorie),
Schmelzpunkt: 160-162°C

| $C_{34}H_{37}N_3O_5$ x $H_2O$ (585,68) | | | |
|---|---|---|---|
| Ber.: | C 69,72 | H 6,71 | N 7,17 |
| Gef.: | 69,63 | 6,64 | 7,33 |

Beispiel 71

4'-[[2-n-Butyl-6-(2-carboxy-3,4,5,6-tetrahydrobenzamino)-benzimidazol-1-yl]methyl]biphenyl-2-carbonsäure

0,4 g (1 mMol) 4'-[(6-Amino-2-n-butyl-benzimidazol-1-yl)methyl]biphenyl-2-carbonsäure, gelöst in 7 ml Pyridin, werden bei Raumtemperatur mit 0,34 g (1,1 mMol) 1-Cyclohexen-1,2-dicarbonsäureanhydrid versetzt und 2 1/2 Stunden gerührt. Es wird mit Eis abgekühlt und das auskristallisierte Produkt abgesaugt, mit gekühltem Aceton gewaschen und im Vakuum bei 70°C getrocknet.

Ausbeute: 0,37 g (67,2 % der Theorie),
Schmelzpunkt: 250-252°C

| $C_{33}H_{33}N_3O_5$ (551,64) | | | |
|---|---|---|---|
| Ber.: | C 71,85 | H 6,03 | N 7,62 |
| Gef.: | 71,70 | 5,99 | 7,60 |

Beispiel 72

4'-[[2-n-Propyl-6-(1-methylbenzimidazol-2-yl)-benzimidazol-1-yl]methyl]biphenyl-2-carbonsäure

604 mg (1,0 mMol) 4'-[[2-Nitro-5-(1-methylbenzimidazol-2-yl)-N-n-butyryl-anilino]methyl]biphenyl-2-carbonsäure-tert.butyl-ester werden in 50 ml Methylenchlorid unter Zusatz von 10 ml Trifluoressigsäure drei Stunden lang bei Raumtemperatur gerührt. Das Lösungsmittel wird danach abdestilliert, der Rückstand in 25 ml Eisessig gelöst und unter Zusatz von 500 mg Palladium/Kohle (10 %) bei 80°C hydriert. Zur Aufarbeitung wird das Lösungsmittel im Vakuum abdestilliert, der Rückstand in 30 ml 2N Natronlauge gelöst und die Lösung mit 20 ml Diethylether gewaschen. Das Rohprodukt, das durch Ansäuern der wäßrigen Phase ausfällt, wird durch anschließende Säulenchromatographie (80 g Kieselgel, Laufmittel: Methylenchlorid/Methanol = 15:1) gereinigt.
Ausbeute: 90 mg (18 % der Theorie),
Schmelzpunkt: 214-216°C

| $C_{32}H_{28}N_4O_2$ (500,60) | | | |
|---|---|---|---|
| Ber.: | C 76,78 | H 5,64 | N 11,19 |
| Gef.: | 76,58 | 5,49 | 11,30 |

Beispiel 73

4'-[[2-n-Propyl-6-(1-methylbenzimidazol-2-yl)-benzimidazol-1-yl]methyl]biphenyl-2-carbonsäure

Eine Suspension von 940 mg (2,0 mMol) 4'-[(2-n-Propyl-6-carboxy-benzimidazol-1-yl)-methyl]biphenyl-2-carbonsäure-tert.butylester und 320 mg (2,0 mMol) Carbonyldiimidazol in einer Lösung von 1,0 ml Triethylamin in 30 ml Tetrahydrofuran wird 30 Minuten lang bei Raumtemperatur gerührt, anschließend 250 mg (2,0 mMol) 2-Methylamino-anilin hinzugefügt und weitere 16 Stunden gerührt. Danach wird zur Trockene eingedampft und der Rückstand in 20 ml Phosphoroxychlorid unter Rühren eine Stunde lang zum Rückfluß erhitzt. Der größte Teil des Phosphoroxychlorids wird danach abdestilliert, der dunkle, schmierige Rückstand mit 30 ml Wasser zersetzt, die so erhaltene stark saure Suspension ca. eine Stunde unter Rückfluß erhitzt, nach dem Abkühlen auf pH 6 eingestellt und eingedampft. Das erhaltene Rohprodukt wird durch Säulenchromatographie (120 g Kieselgel, Laufmittel: Methylenchlorid/Methanol = 15:1) gereinigt.
Ausbeute: 73 mg (7,3 % der Theorie),
Schmelzpunkt: 213-215°C

| $C_{32}H_{28}N_4O_2$ (500,60) | | | |
|---|---|---|---|
| Ber.: | C 76,78 | H 5,64 | N 11,19 |
| Gef.: | 76,61 | 5,64 | 10,94 |

Beispiel 74

4'-[[2-n-Propyl-6-(2-oxo-piperidin-1-yl)-benzimidazol-1-yl]methyl]-2-(1H-tetrazol-5-yl)-biphenyl

Zu einer Lösung von 650 mg (1,0 mMol) 4'-[(2-n-Propyl-6-amino-benzimidazol-1-yl)-methyl]-2-(1H-triphenylmethyl-tetrazol-5-yl)-biphenyl in 30 ml Tetrahydrofuran werden innerhalb 10 Minuten 155 mg (1,0 mMol) 5-Chlor-valeriansäurechlorid, gelöst in 5 ml Tetrahydrofuran, zugetropft und das Gemisch eine weitere Stunde lang bei Raumtemperatur gerührt, dann bis zur Trockne eingedampft. Der Rückstand wird in 20 ml Ethanol aufgerührt, dann eine Lösung von 2,0 mMol Natriumethylat in 20 ml Ethanol hinzugegeben und eine Stunde lang zum Rückfluß erhitzt. Nach dem Abkühlen werden 10 ml methanolische Salzsäure zugetropft, zwei weitere Stunden lang bei Raumtemperatur gerührt

und dann eingeengt. Der Rückstand wird mit 10 ml Wasser versetzt und mit konzentriertem Ammoniak alkalisch gestellt, wobei das Produkt in Lösung geht. Durch Ansäuern mit Eisessig wird das Rohprodukt ausgefällt, das dann durch Säulenchromatographie gereinigt wird (70 g Kieselgel, Laufmittel: Methylenchlorid + 5 % Ethanol).
Ausbeute: 54 mg (11 % der Theorie),
Schmelzpunkt: ab 117°C sintern

| $C_{29}H_{29}N_7O$ (491,60) | | | |
|---|---|---|---|
| Ber.: | C 70,85 | H 5,95 | N 19,95 |
| Gef.: | 70,69 | 5,94 | 19,99 |

Analog werden folgende Verbindungen erhalten:
4'-[[2-n-Butyl-6-(2-oxo-piperidin-1-yl)-benzimidazol-1-yl]methyl]-2-(1H-tetrazol-5-yl)-biphenyl
Ausbeute: 16 % der Theorie,
Schmelzpunkt: amorph

| $C_{30}H_{31}N_7O$ (505,63) | | | |
|---|---|---|---|
| Ber.: | C 67,94 | H 6,23 | N 17,33 |
| Gef.: | 67,81 | 6,29 | 17,18 |

4'-[[2-n-Butyl-6-(2-oxo-pyrrolidin-1-yl)-benzimidazol-1-yl]methyl]-2-(1H-tetrazol-5-yl)-biphenyl
Ausbeute: 9 % der Theorie,
Schmelzpunkt: 150-151°C

| $C_{29}H_{29}N_7O$ (491,60) | | | |
|---|---|---|---|
| Ber.: | C 70,85 | H 5,95 | N 19,95 |
| Gef.: | 70,61 | 6,08 | 19,80 |

Beispiel 75

4'-[[2-n-Butyl-6-(propansultam-1-yl)-benzimidazol-1-yl]methyl]-2-(1H-tetrazol-5-yl)-biphenyl

Zu einer Lösung von 665 mg (1,0 mMol) 4'-[(2-n-Butyl-6-aminobenzimidazol-1-yl)-methyl]-2-(1-triphenylmethyl-tetrazol-5-yl)-biphenyl und 1 ml Triethylamin in 30 ml Tetrahydrofuran wird innerhalb von 10 Minuten eine Lösung von 265 mg (1,5 mMol) 3-Chlor-propansulfonsäurechlorid in 5 ml Tetrahydrofuran zugetropft und 1,5 Stunden lang bei Raumtemperatur gerührt. Das Gemisch wird dann zur Trockne eingedampft, der Rückstand in 20 ml Ethanol aufgenommen, eine Lösung von 3,0 mMol Natriumethylat in 20 ml Ethanol hinzugegeben und zwei Stunden lang unter Rückfluß erhitzt. Nach dem Abkühlen werden 10 ml methanolischer Salzsäure zugetropft, zwei weitere Stunden bei Raumtemperatur gerührt und schließlich eingeengt. Der Rückstand wird mit 10 ml Wasser versetzt und mit konzentriertem Ammoniak in Lösung gebracht. Durch Ansäuern mit Eisessig wird das Rohprodukt ausgefällt und anschließend durch Säulenchromatographie gereinigt (70 g Kieselgel, Laufmittel: Methylenchlorid + 5 % Ethanol).
Ausbeute: 68,5 mg (13 % der Theorie),
Schmelzpunkt: 202-205°C

| $C_{28}H_{29}N_7O_2S$ (527,70) | | | |
|---|---|---|---|
| Ber.: | C 63,73 | H 5,54 | N 18,58 |
| Gef.: | 63,70 | 5,61 | 18,35 |

Analog werden folgende Verbindungen erhalten:
4'-[[2-n-Butyl-6-(butansultam-1-yl)-benzimidazol-1-yl]methyl]-2-(1H-tetrazol-5-yl)-biphenyl
Ausbeute: 10 % der Theorie,
Schmelzpunkt: 185-187°C

| $C_{29}H_{31}N_7O_2S$ (541,70) | | | |
|---|---|---|---|
| Ber.: | C 64,30 | H 5,95 | N 18,10 |
| Gef.: | 64,19 | 5,91 | 17,92 |

4'-[[2-n-Propyl-6-(butansultam-1-yl)-benzimidazol-1-yl]methyl]-2-(1H-tetrazol-5-yl)-biphenyl
Ausbeute: 17 % der Theorie,
Schmelzpunkt: 203-205°C

| $C_{28}H_{29}N_7O_2S$ (527,63) | | | |
|---|---|---|---|
| Ber.: | C 63,73 | H 5,54 | N 18,58 |
| Gef.: | 63,63 | 5,54 | 18,39 |

Beispiel 76

4'-[[2-n-Butyl-6-(1-benzyl-imidazolidin-2,4-dion-3-yl)-benzimidazol-1-yl]methyl]biphenyl-2-carbonsäure-trifluoracetat

Hergestellt analog Beispiel 1 aus 4'-[[2-n-Butyl-6-(1-benzylimidazolidin-2,4-dion-3-yl)-benzimidazol-1-yl]methyl]biphenyl-2-carbonsäure-tert.butylester und Trifluoressigsäure in Methylenchlorid.
Ausbeute: 58 % der Theorie,
Schmelzpunkt: amorph

| $C_{35}H_{32}N_4O_4$ x $CF_3COOH$ (686,71) | | | |
|---|---|---|---|
| Ber.: | C 64,72 | H 4,84 | N 8,16 |
| Gef.: | 64,48 | 4,68 | 8,09 |

Beispiel 77

4'-[[2-n-Propyl-6-(5,5-pentamethylen-imidazolidin-2,4-dion-3-yl)-benzimidazol-1-yl]methyl]biphenyl-2-carbonsäure

Hergestellt analog Beispiel 1 aus 4'-[[2-n-Propyl-6-(5,5-pen-tamethylen-imidazolidin-2,4-dion-3-yl)-benzimidazol-1-yl]methyl]biphenyl-2-carbonsäure-tert.butylester und Trifluoressigsäure in Methylenchlorid.
Ausbeute: 27 % der Theorie,
Schmelzpunkt: amorph

$C_{33}H_{34}N_4O_4$ (550,63)

| | | | |
|---|---|---|---|
| Ber.: | C 71,98 | H 6,22 | N 10,18 |
| Gef.: | 71,93 | 6,16 | 10,09 |

$R_f$-Wert: 0,60 (Kieselgel; Laufmittel: Methylenchlorid/Ethanol = 9:1)

Beispiel 78

4'-[[2-Ethyl-6-(2-oxo-piperidin-1-yl)-benzimidazol-1-yl]methyl]-2-(1H-tetrazol-5-yl)-biphenyl

Hergestellt analog Beispiel 41 aus 4'-[[2-Ethyl-6-(2-oxo-piperidin-1-yl)-benzimidazol-1-yl]methyl]biphenyl-2-carbonsäurenitril und Natriumazid in Dimethylformamid.
Ausbeute: 33 % der Theorie,
Schmelzpunkt: ab 150°C Sintern

| $C_{28}H_{27}N_7O$ (477,58) | | | |
|---|---|---|---|
| Ber.: | C 70,42 | H 5,70 | N 20,53 |
| Gef.: | 70,48 | 5,72 | 19,88 |

Beispiel 79

4'-[[2-Ethyl-6-(butansultam-1-yl)-benzimidazol-1-yl]methyl]-2-(1H-tetrazol-5-yl)-biphenyl

Hergestellt analog Beispiel 41 aus 4'-[[2-Ethyl-6-(butansultam-1-yl)-benzimidazol-1-yl]methyl]biphenyl-2-carbonsäurenitril und Natriumazid in Dimethylformamid.
Ausbeute: 36 % der Theorie,
Schmelzpunkt: ab 240°C Zersetzung

| $C_{27}H_{27}N_7O_2S$ (513,64) | | | |
|---|---|---|---|
| Ber.: | C 63,14 | H 5,30 | N 19,09 |
| Gef.: | 63,06 | 5,19 | 19,08 |

Beispiel 80

4'-[[2-n-Propyl-6-(3-n-hexyl-imidazo[4,5-b]pyridin-2-yl)-benzimidazol-1-yl]methyl]biphenyl-2-carbonsäure

Hergestellt analog Beispiel 1 aus 4'-[[2-n-Propyl-6-(3-n-hexyl-imidazo[4,5-b]pyridin-2-yl)-benzimidazol-1-yl]methyl]biphenyl-2-carbonsäure-tert.butylester und Trifluoressigsäure in Methylenchlorid.
Ausbeute: 57 % der Theorie,
Schmelzpunkt: amorph

| $C_{36}H_{37}N_5O_2$ (571,74) | | | |
|---|---|---|---|
| Ber.: | C 75,63 | H 6,52 | N 12,25 |
| Gef.: | 75,58 | 6,48 | 12,08 |

Beispiel 81

4'-[[2-n-Propyl-6-(3-methyl-imidazo[4,5-b]pyridin-2-yl)-benzimidazol-1-yl]methyl]biphenyl-2-carbonsäure

Hergestellt analog Beispiel 1 aus 4'-[[2-n-Propyl-6-(3-methyl-imidazo[4,5-b]pyridin-2-yl)-benzimidazol-1-yl]methyl] biphenyl-2-carbonsäure-tert.butylester und Trifluoressigsäure in Methylenchlorid.
Ausbeute: 40 % der Theorie,
Schmelzpunkt: 208-210°C

| $C_{31}H_{27}N_5O_2$ (501,60) | | | |
|---|---|---|---|
| Ber.: | C 74,23 | H 5,43 | N 13,96 |
| Gef.: | 74,19 | 5,32 | 13,94 |

Beispiel 82

4'-[[2-n-Propyl-6-(1-methyl-imidazolin-2-yl)-benzimidazol-1-yl]methyl]biphenyl-2-carbonsäure

Hergestellt analog Beispiel 1 aus 4'-[[2-n-Propyl-6-(1-methyl-imidazolin-2-yl)-benzimidazol-1-yl]methyl]biphenyl-2-carbonsäure-tert.butylester und Trifluoressigsäure in Methylenchlorid.
Ausbeute: 53 % der Theorie,
Schmelzpunkt: amorph

| $C_{28}H_{28}N_4O_2$ (452,57) | | | |
|---|---|---|---|
| Ber.: | C 74,31 | H 6,24 | N 12,38 |
| Gef.: | 74,31 | 6,11 | 12,27 |

Analog Beispiel 82 werden folgende Verbindungen erhalten:

4'-[[2-n-Butyl-6-(1-methyl-imidazolin-2-yl)-benzimidazol-1-yl]methyl]biphenyl-2-carbonsäure

4'-[[2-n-Propyl-6-(1-n-hexyl-imidazolin-2-yl)-benzimidazol-1-yl]methyl]biphenyl-2-carbonsäure

4'-[[2-n-Butyl-6-(1-n-butyl-imidazolin-2-yl)-benzimidazol-1-yl]methyl]biphenyl-2-carbonsäure

4'-[[2-n-Propyl-6-(1-cyclopropyl-imidazolin-2-yl)-benzimidazol-1-yl]methyl]biphenyl-2-carbonsäure

4'-[[2-n-Propyl-6-(1-cyclohexyl-imidazolin-2-yl)-benzimidazol-1-yl]methyl]biphenyl-2-carbonsäure

4'-[[2-n-Propyl-6-(l-methyl-imidazol-2-yl)-benzimidazol-1-yl]methyl]biphenyl-2-carbonsäure

4'-[[2-n-Butyl-6-(1-methyl-imidazol-2-yl)-benzimidazol-1-yl]methyl]biphenyl-2-carbonsäure

4'-[[2-n-Propyl-6-(1-n-hexyl-imidazol-2-yl)-benzimidazol-1-yl]methyl]biphenyl-2-carbonsäure

4'-[[2-n-Butyl-6-(1-n-butyl-imidazol-2-yl)-benzimidazol-1-yl]methyl]biphenyl-2-carbonsäure

4'-[[2-n-Propyl-6-(1-cyclopropyl-imidazol-2-yl)-benzimidazol-1-yl]methyl]biphenyl-2-carbonsäure

4'-[[2-n-Propyl-6-(1-cyclohexyl-imidazol-2-yl)-benzimidazol-1-yl]methyl]biphenyl-2-carbonsäure

Beispiel 83

4'-[[2-n-Propyl-6-(1,5-dimethyl-benzimidazol-2-yl)-benzimidazol-1-yl]methyl]biphenyl-2-carbonsäure

Hergestellt analog Beispiel 1 aus 4'-[[2-n-Propyl-6-(1,5-dimethyl-benzimidazol-2-yl)-benzimidazol-1-yl]methyl]biphenyl-2-carbonsäure-tert.butylester und Trifluoressigsäure in Methylenchlorid.
Ausbeute: 48 % der Theorie,
Schmelzpunkt: 256-258°C

| $C_{33}H_{30}N_4O_2$ (514,63) | | | |
|---|---|---|---|
| Ber.: | C 77,02 | H 5,88 | N 10,89 |
| Gef.: | 76,91 | 5,83 | 10,72 |

Beispiel 84

4'-[[2-n-Propyl-6-(1-methyl-5-trifluormethyl-benzimidazol-2-yl)-benzimidazol-1-yl]methyl]biphenyl-2-carbonsäure

Hergestellt analog Beispiel 1 aus 4'-[[2-n-Propyl-6-(1-methyl-5-trifluormethyl-benzimidazol-2-yl)-benzimidazol-1-yl]methyl]biphenyl-2-carbonsäure-tert.butylester und Trifluoressigsäure in Methylenchlorid.
Ausbeute: 56 % der Theorie,
Schmelzpunkt: 183-186°C

| $C_{33}H_{27}F_3N_4O_2$ (568,61) | | | |
|---|---|---|---|
| Ber.: | C 69,71 | H 4,79 | N 9,85 |
| Gef.: | 69,58 | 4,72 | 9,80 |

Beispiel 85

4'-[[2-n-Propyl-6-(5-methyl-imidazolidin-2,4-dion-3-yl)-benzimidazol-1-yl]methyl]biphenyl-2-carbonsäure

Hergestellt analog Beispiel 1 aus 4'-[[2-n-Propyl-6-(5-methyl-imidazolidin-2,4-dion-3-yl)-benzimidazol-1-yl]methyl] biphenyl-2-carbonsäure-tert.butylester und Trifluoressigsäure in Methylenchlorid.
Ausbeute: 29 % der Theorie,
Schmelzpunkt: amorph

| $C_{28}H_{26}N_4O_4$ (482,55) | | | |
|---|---|---|---|
| Ber.: | C 69,69 | H 5,43 | N 11,61 |
| Gef.: | 69,67 | 5,40 | 11,55 |

Beispiel 86

4'-[(2-n-Propyl-6-(1-methyl-imidazolidin-2,4-dion-3-yl)-benzimidazol-1-yl)-methyl]biphenyl-2-carbonsäure

Hergestellt analog Beispiel 1 aus 4'-[(2-n-Propyl-6-(1-methyl-imidazolidin-2,4-dion-3-yl)-benzimidazol-1-yl)-methyl]biphenyl-2-carbonsäure-tert.butylester und Trifluoressigsäure in Methylenchlorid.
Ausbeute: 32 % der Theorie,
Schmelzpunkt: amorph

| $C_{28}H_{26}N_4O_4$ (482,55) | | | |
|---|---|---|---|
| Ber.: | C 69,69 | H 5,43 | N 11,61 |
| Gef.: | 69,61 | 5,38 | 11,49 |

Beispiel 87

4'-[(2-n-Propyl-6-(1-butyl-benzimidazol-2-yl)-benzimidazol-1-yl)-methyl]biphenyl-2-carbonsäure

Hergestellt analog Beispiel 1 aus 4'-[(2-n-Propyl-6-(1-butylbenzimidazol-2-yl)-benzimidazol-1-yl)-methyl]biphenyl-2-carbonsäure-tert.butylester und Trifluoressigsäure in Methylenchlorid.
Ausbeute: 59 % der Theorie,
Schmelzpunkt: ab 149°C sintern

| $C_{35}H_{34}N_4O_2$ (542,69) | | | |
|---|---|---|---|
| Ber.: | C 77,46 | H 6,32 | N 10,32 |
| Gef.: | 77,37 | 6,31 | 10,35 |

Beispiel 88

4'-[(2-n-Butyl-6-(1H-benzimidazol-2-yl)-benzimidazol-1-yl)methyl]biphenyl-2-carbonsäure

Hergestellt analog Beispiel 1 aus 4'-[(2-n-Butyl-6-(1H-benzimidazol-2-yl)-benzimidazol-1-yl)-methyl]biphenyl-2-carbonsäure-tert.butylester und Trifluoressigsäure in Methylenchlorid.
Ausbeute: 62 % der Theorie,
Schmelzpunkt: 200-202°C

| $C_{32}H_{28}N_4O_2$ (500,61) | | | |
|---|---|---|---|
| Ber.: | C 76,78 | H 5,64 | N 11,19 |
| Gef.: | 76,54 | 5,60 | 11,16 |

Beispiel 89

4'-[(2-n-Butyl-6-hexahydrohomophthalimino-benzimidazol-1-yl)methyl]biphenyl-2-carbonsäure

0,4 g (0,64 mMol) 4'-[(2-n-Butyl-6-(2-carboxy-cyclohexylmethylcarbonylamino)-benzimidazol-1-yl)-methyl]biphenyl-2-carbonsäure-tert.butylester werden unter Rühren in 5 ml Phosphoroxychlorid 1 1/2 Stunden unter Rückfluß erhitzt. Nach dem Abkühlen gießt man auf Eiswasser und saugt das ausgefallene Rohprodukt ab. Dieses wird in Ethanol/Wasser gelöst, mit Ammoniak alkalisch gestellt und bis zum Auskristallisieren im Vakuum eingeengt. Anschließend saugt man ab, wäscht mit Wasser und trocknet im Vakuum bei 120°C.
Ausbeute: 0,15 g (42,8 % der Theorie),
Schmelzpunkt: 241-243°C

| $C_{34}H_{35}N_3O_4$ (549,66) | | | |
|---|---|---|---|
| Ber.: | C 74,29 | H 6,49 | N 7,64 |
| Gef.: | 74,14 | 6,64 | 7,81 |

Beispiel 90

4'-[(2-n-Butyl-6-(7-nitro-benzofurazan-4-yl-amino)-benzimidazol-1-yl)-methyl]biphenyl-2-carbonsäure

Hergestellt aus 4'-[(6-Amino-2n-butyl-benzimidazol-1-yl)-methyl]biphenyl-2-carbonsäure und 4-Chlor-7-nitro-benzofurazan in Pyridin bei Raumtemperatur.
Ausbeute: 13,1 % der Theorie,
$R_f$-Wert: 0,75 (Kieselgel; Methylenchlorid/Ethanol = 9:1)

| $C_{31}H_{26}N_6O_5$ (562,58) | | | |
|---|---|---|---|
| Ber.: | C 66,18 | H 4,66 | N 14,93 |
| Gef.: | 66,35 | 4,76 | 15,13 |

Beispiel 91

4'-[[2-Ethyl-6-(pyrrolidinocarbonylamino)-benzimidazol-1-yl]methyl]biphenyl-2-carbonsäure-trifluoracetat-semihydrat

Hergestellt analog Beispiel 1 aus 4'-[[2-Ethyl-6-(pyrrolidinocarbonylamino)-benzimidazol-1-yl]-methyl]biphenyl-2-carbonsäure-tert.butylester und Trifluoressigsäure in Methylenchlorid.
Ausbeute: 80,9 % der Theorie,
Schmelzpunkt: 178-179°C

| $C_{28}H_{28}N_4O_3$ x $CF_3COOH$ x 0,5 $H_2O$ (591,59) | | | |
|---|---|---|---|
| Ber.: | C 60,90 | H 5,11 | N 9,47 |
| Gef.: | 61,10 | 5,22 | 9,26 |

$R_f$-Wert: 0,48 (Kieselgel; Essigester/Ethanol/Ammoniak = 50:45:5)

Beispiel 92

4'-[[2-Methyl-6-(pyrrolidinocarbonylamino)-benzimidazol-1-yl]methyl]biphenyl-2-carbonsäure-trifluoracetat

Hergestellt analog Beispiel 1 aus 4'-[[2-Methyl-6-(pyrrolidinocarbonylamino)-benzimidazol-1-yl]-methyl]biphenyl-2-carbonsäure-tert.butylester und Trifluoressigsäure in Methylenchlorid.
Ausbeute: 82,1 % der Theorie,
Schmelzpunkt: 181-182°C

| $C_{27}H_{26}N_4O_3$ x $CF_3COOH$ (568,55) | | | |
|---|---|---|---|
| Ber.: | C 61,26 | H 4,79 | N 9,85 |
| Gef.: | 60,99 | 5,09 | 9,89 |

$R_f$-Wert: 0,38 (Kieselgel; Essigester/Ethanol/Ammoniak = 50:45:5)

Beispiel 93

4'-[[2-n-Propyl-6-(pyrrolidinocarbonylamino)-benzimidazol-1-yl]methyl]biphenyl-2-carbonsäure-trifluoracetat

Hergestellt analog Beispiel 1 aus 4'-[[2-n-Propyl-6-(pyrrolidinocarbonylamino)-benzimidazol-1-yl]methyl]biphenyl-2-carbonsäure-tert.butylester und Trifluoressigsäure in Methylenchlorid.
Ausbeute: 79,7 % der Theorie,
Schmelzpunkt: 207-208°C

| $C_{29}H_{30}N_4O_3$ x $CF_3COOH$ (596,61) | | | |
|---|---|---|---|
| Ber.: | C 62,41 | H 5,24 | N 9,39 |
| Gef.: | 62,38 | 5,36 | 9,42 |

$R_f$-Wert: 0,55 (Kieselgel; Essigester/Ethanol/Ammoniak = 50:45:5)

Beispiel 94

4'-[[2-Methylmercapto-6-(pyrrolidinocarbonylamino)-benzimidazol-1-yl]methyl]biphenyl-2-carbonsäure-trifluoracetat

Hergestellt analog Beispiel 1 aus 4'-[[2-Methylmercapto-6-(pyrrolidinocarbonylamino)-benzimidazol-1-yl]methyl]biphenyl-2-carbonsäure-tert.butylester und Trifluoressigsäure in Methylenchlorid.
Ausbeute: 96,1 % der Theorie,
Schmelzpunkt: 177-178°C

| $C_{27}H_{26}N_4O_3S$ x $CF_3COOH$ (600,61) | | | |
|---|---|---|---|
| Ber.: | C 57,99 | H 4,53 | N 9,33 |
| Gef.: | 57,68 | 4,75 | 9,30 |

$R_f$-Wert: 0,52 (Kieselgel; Essigester/Ethanol/Ammoniak = 50:45:5)

Beispiel 95

4'-[[6-(2,3-Dimethylmaleinsäureimido)-2-methylmercapto-benzimidazol-1-yl]methyl]biphenyl-2-carbonsäure

Hergestellt analog Beispiel 1 aus 4'-[[6-(2,3-Dimethylmaleinsäureimido)-2-methylmercapto-benzimidazol-1-yl]methyl]biphenyl-2-carbonsäure-tert.butylester und Trifluoressigsäure in Methylenchlorid.
Ausbeute: 91,7 % der Theorie,
Schmelzpunkt: 276-277°C

| $C_{28}H_{23}N_3O_4S$ (497,57) | | | | |
|---|---|---|---|---|
| Ber.: | C 67,59 | H 4,66 | N 8,45 | S 6,44 |
| Gef.: | 67,57 | 4,94 | 8,40 | 6,37 |

$R_f$-Wert: 0,47 (Kieselgel; Essigester/Ethanol/Ammoniak = 50:45:5)

Beispiel 96

4'-[[2-n-Butyl-6-[3-(7-nitrobenzofurazan-4-yl-amino)-propionylamino]benzimidazol-1-yl]methyl]-2-(1H-tetrazol-5-yl)-biphenyl-hydrat

Hergestellt analog Beispiel 53 aus 4'-[[2-n-Butyl-6-[3-(7-nitrobenzofurazan-4-yl-amino)-propionylamino]benzimidazol-1-yl]methyl]-2-(1-triphenylmethyl-tetrazol-5-yl)-biphenyl und 2 N Salzsäure in Ethanol.
Ausbeute: 33,3 % der Theorie,
Schmelzpunkt: 179-181°C

| $C_{34}H_{31}N_{11}O_4$ x $H_2O$ (675,70) | | | |
|---|---|---|---|
| Ber.: | C 60,43 | H 4,92 | N 22,80 |
| Gef.: | 60,24 | 5,09 | 22,69 |

Beispiel 97

4'-[[2-n-Butyl-6-[3-(7-nitrobenzofurazan-4-yl-amino)-propionylamino]benzimidazol-1-yl]methyl]biphenyl-2-carbonsäure-trifluoracetat-hydrat

Hergestellt analog Beispiel 1 aus 4'-[[2-n-Butyl-6-[3-(7-nitrobenzofurazan-4-yl-amino)-propionylamino]benzimidazol-1-yl]methyl]biphenyl-2-carbonsäure-tert.butylester und Trifluoressigsäure in Methylenchlorid.
Ausbeute: 87,5 % der Theorie,
Schmelzpunkt: 127°C (Zers.)

| $C_{34}H_{31}N_7O_6$ x $CF_3COOH$ x $H_2O$ (765,69) | | | |
|---|---|---|---|
| Ber.: | C 56,47 | H 4,47 | N 12,80 |
| Gef.: | 56,68 | 4,27 | 12,67 |

Beispiel 98

4'-[[6-(2,3-Dimethylmaleinsäureimido)-2-methyl-benzimidazol-1-yl]methyl]biphenyl-2-carbonsäure

Hergestellt analog Beispiel 1 aus 4'-[[6-(2,3-Dimethylmaleinsäureimido)-2-methyl-benzimidazol-1-yl]methyl]biphenyl-2-carbonsäure-tert.butylester und Trifluoressigsäure in Methylenchlorid.
Ausbeute: 94,4 % der Theorie,
Schmelzpunkt: 327-328°C

| $C_{28}H_{23}N_3O_4$ (465,51) | | | |
|---|---|---|---|
| Ber.: | C 72,25 | H 4,98 | N 9,03 |
| Gef.: | 72,00 | 5,08 | 9,06 |

$R_f$-Wert: 0,33 (Kieselgel; Essigester/Ethanol/Ammoniak = 50:45:5)

Beispiel 99

4'-[[6-(2,3-Dimethylmaleinsäureimido)-benzimidazol-1-yl]methyl]biphenyl-2-carbonsäure-semihydrat

Hergestellt analog Beispiel 1 aus 4'-[[6-(2,3-Dimethylmaleinsäureimido)-benzimidazol-1-yl]methyl]biphenyl-2-carbonsäure-tert.butylester und Trifluoressigsäure in Methylenchlorid.
Ausbeute: 95,5 % der Theorie,
Schmelzpunkt: 223-224°C

| $C_{27}H_{21}N_3O_4$ x 0,5 $H_2O$ (460,49) | | | |
|---|---|---|---|
| Ber.: | C 70,42 | H 4,82 | N 9,13 |

(fortgesetzt)

| $C_{27}H_{21}N_3O_4$ x 0,5 $H_2O$ (460,49) | | | |
|---|---|---|---|
| Gef.: | 70,30 | 4,88 | 8,81 |

$R_f$-Wert: 0,34 (Kieselgel; Essigester/Ethanol/Ammoniak = 50:45:5)

Beispiel 100

4'-[[6-(2,3-Dimethylmaleinsäureimido)-2-n-propyl-benzimidazol-1-yl]methyl]biphenyl-2-carbonsäure-monohydrat

Hergestellt analog Beispiel 1 aus 4'-[[6-(2,3-Dimethylmaleinsäureimido)-2-n-propyl-benzimidazol-1-yl]methyl]biphenyl-2-carbonsäure-tert.butylester und Trifluoressigsäure in Methylenchlorid.
Ausbeute: 92,5 % der Theorie,
Schmelzpunkt: 309-310°C

| $C_{30}H_{27}N_3O_4$ x $H_2O$ (511,58) | | | |
|---|---|---|---|
| Ber.: | C 70,44 | H 5,71 | N 8,21 |
| Gef.: | 70,44 | 5,64 | 8,19 |

$R_f$-Wert: 0,47 (Kieselgel; Essigester/Ethanol/Ammoniak = 50:45:5)

Beispiel 101

4'-[[6-(2,3-Dimethylmaleinsäureimido)-2-ethyl-benzimidazol-1-yl]methyl]biphenyl-2-carbonsäure

Hergestellt analog Beispiel 1 aus 4'-[[6-(2,3-Dimethylmaleinsäureimido)-2-ethyl-benzimidazol-1-yl]methyl]biphenyl-2-carbonsäure-tert.butylester und Trifluoressigsäure in Methylenchlorid.
Ausbeute: 87,5 % der Theorie,
Schmelzpunkt: 307-308°C

| $C_{29}H_{25}N_3O_4$ (479,53) | | | |
|---|---|---|---|
| Ber.: | C 72,64 | H 5,25 | N 8,76 |
| Gef.: | 72,41 | 5,37 | 8,94 |

$R_f$-Wert: 0,40 (Kieselgel; Essigester/Ethanol/Ammoniak = 50:45:5)

Beispiel 102

4'-[[2-Ethyl-6-(1-methylbenzimidazol-2-yl)-benzimidazol-1-yl]methyl]biphenyl-2-carbonsäure

Hergestellt analog Beispiel 1 aus 4'-[[2-Ethyl-6-(1-methylbenzimidazol-2-yl)-benzimidazol-1-yl]methyl]biphenyl-2-carbonsäure-tert.butylester und Trifluoressigsäure in Methylenchlorid.
Ausbeute: 31 % der Theorie,
Schmelzpunkt: 183-185°C

| $C_{31}H_{26}N_4O_2$ (486,60) | | | |
|---|---|---|---|
| Ber.: | C 76,52 | H 5,39 | N 11,52 |
| Gef.: | 76,73 | 5,49 | 11,70 |

Beispiel 103

4'-[[2-Methyl-6-(butansultam-1-yl)-benzimidazol-1-yl]methyl]-2-(1H-tetrazol-5-yl)-biphenyl

Hergestellt analog Beispiel 41 aus 4'-[[2-Methyl-6-(butansultam-1-yl)-benzimidazol-1-yl]methyl]-2-cyano-biphenyl

und Natriumazid in Dimethylformamid.
Ausbeute: 27 % der Theorie,
Schmelzpunkt: 173-175°C

| $C_{26}H_{25}N_7O_2S$ (499,60) | | | | |
|---|---|---|---|---|
| Ber.: | C 62,51 | H 5,04 | N 19,63 | S 6,42 |
| Gef.: | 62,39 | 5,05 | 19,44 | 6,33 |

Massenspektrum: m/e = 499

Beispiel 104

4'-[[2-Methyl-6-(1-methylbenzimidazol-2-yl)-benzimidazol-1-yl]methyl]-2-(1H-tetrazol-5-yl)-biphenyl

Hergestellt analog Beispiel 41 aus 4'-[[2-Methyl-6-(1-methylbenzimidazol-2-yl)-benzimidazol-1-yl]methyl]-2-cyano-biphenyl und Natriumazid in Dimethylformamid.
Ausbeute: 26,5 % der Theorie,
Schmelzpunkt: 214-217°C

| $C_{30}H_{24}N_8$ (496,80) | | | |
|---|---|---|---|
| Ber.: | C 72,56 | H 4,87 | N 22,56 |
| Gef.: | 72,32 | 5,01 | 22,23 |

Beispiel 105

4'-[[6-(butansultam-1-yl)-benzimidazol-1-yl]methyl]-2-(1H-tetrazol-5-yl)-biphenyl

Hergestellt analog Beispiel 41 aus 4'-[[6-(butansultam-1-yl)benzimidazol-1-yl]methyl]-2-cyano-biphenyl und Natriumazid in Dimethylformamid.
Ausbeute: 60,0 % der Theorie,
Schmelzpunkt: 246-249°C

| $C_{25}H_{23}N_7O_2S$ (485,60) | | | |
|---|---|---|---|
| Ber.: | C 61,84 | H 4,77 | N 20,19 |
| Gef.: | 61,75 | 4,92 | 20,28 |

Beispiel 106

4'-[[2-Ethyl-6-(1-methylbenzimidazol-2-yl)-benzimidazol-1-yl]methyl]-2-(1H-tetrazol-5-yl)-biphenyl

Hergestellt analog Beispiel 41 aus 4'-[[2-Ethyl-6-(1-methylbenzimidazol-2-yl)-benzimidazol-1-yl]methyl]-2-cyano-biphenyl und Natriumazid in Dimethylformamid.
Ausbeute: 41,0 % der Theorie,
Schmelzpunkt: ab 178°C (sintern)

| $C_{31}H_{26}N_8$ (510,60) | | | |
|---|---|---|---|
| Ber.: | C 72,92 | H 5,13 | N 21,95 |
| Gef.: | 72,94 | 5,25 | 21,71 |

Massenspektrum: m/e = 510

Beispiel 107

4'-[[2-Ethyl-6-(N-benzolsulfonyl-methylamino)-benzimidazol-1-yl]methyl]-2-(1H-tetrazol-5-yl)-biphenyl

Hergestellt analog Beispiel 41 aus 4'-[[2-Ethyl-6-(N-benzolsulfonyl-methylamino)-benzimidazol-1-yl]methyl]-2-cyano-biphenyl und Natriumazid in Dimethylformamid.
Ausbeute: 66,0 % der Theorie,
Schmelzpunkt: 226-228°C

| $C_{30}H_{27}N_7O_2S$ (549,70) | | | | |
|---|---|---|---|---|
| Ber.: | C 65,55 | H 4,95 | N 17,84 | S 5,83 |
| Gef.: | 65,38 | 4,95 | 17,59 | 5,79 |

Beispiel 108

4'-[[2-n-Propyl-6-(N-benzolsulfonyl-methylamino)-benzimidazol-1-yl]methyl]-2-(1H-tetrazol-5-yl)-biphenyl

Hergestellt analog Beispiel 41 aus 4'-[[2-n-Propyl-6-(N-benzolsulfonyl-methylamino)-benzimidazol-1-yl]methyl]-2-cyano-biphenyl und Natriumazid in Dimethylformamid.
Ausbeute: 83,4 % der Theorie,
Schmelzpunkt: 177-179°C

| $C_{31}H_{29}N_7O_2S$ (563,70) | | | | |
|---|---|---|---|---|
| Ber.: | C 66,05 | H 5,18 | N 17,40 | S 5,69 |
| Gef.: | 65,89 | 5,14 | 17,21 | 5,73 |

Beispiel 109

4'-[[2-n-Butyl-6-(3-benzyl-3,4,5,6-tetrahydro-2(1H)-pyrimidinon-1-yl)-benzimidazol-1-yl]methyl]-2-(1H)-tetrazol-5-yl) biphenyl

Hergestellt analog Beispiel 53 aus 4'-[[2-n-Butyl-6-(3-benzyl-3,4,5,6-tetrahydro-2(1H)-pyrimidinon-1-yl)-benzimidazol-1-yl]methyl]-2-(1-triphenylmethyl-tetrazol-5-yl)-biphenyl und Methanol in methanolischer Salzsäure.
Ausbeute: 28,0 % der Theorie,
Schmelzpunkt: ab 125°C (Zers.)

| $C_{36}H_{36}N_8O$ (596,80) | | | |
|---|---|---|---|
| Ber.: | C 72,46 | H 6,08 | N 18,78 |
| Gef.: | 72,26 | 5,94 | 18,85 |

Beispiel 110

4'-[[2-n-Butyl-5-(3-benzyl-3,4,5,6-tetrahydro-2(1H)-pyrimidinon-1-yl)-benzimidazol-1-yl)methyl]-2-(1H)-tetrazol-5-yl) biphenyl

Hergestellt analog Beispiel 53 aus 4'-[[2-n-Butyl-5-(3-benzyl-3,4,5,6-tetrahydro-2(1H)-pyrimidinon-1-yl)-benzimidazol-1-yl]methyl]-2-(1-triphenylmethyl-tetrazol-5-yl)-biphenyl und Methanol in methanolischer Salzsäure.
Ausbeute: 31,0 % der Theorie,
Schmelzpunkt: ab 125°C (Zers.)

| $C_{36}H_{36}N_8O$ (596,80) | | | |
|---|---|---|---|
| Ber.: | C 72,46 | H 6,08 | N 18,78 |
| Gef.: | 72,27 | 6,03 | 18,61 |

Beispiel 111

4'-[[2-n-Propyl-6-(3-benzyl-3,4,5,6-tetrahydro-2(1H)-pyrimidinon-1-yl)-benzimidazol-1-yl]methyl]-2-(1H)-tetrazol-5-yl) biphenyl

Hergestellt analog Beispiel 53 aus 4'-[[2-n-Propyl-6-(3-benzyl-3,4,5,6-tetrahydro-2(1H)-pyrimidinon-1-yl)-benzimidazol-1-yl]methyl]-2-(1-triphenylmethyl-tetrazol-5-yl)-biphenyl und Methanol in methanolischer Salzsäure.
Ausbeute: 35,0 % der Theorie,
Schmelzpunkt: ab 132°C (Zers.)

| $C_{35}H_{34}N_8O$ (582,71) | | | |
|---|---|---|---|
| Ber.: | C 72,14 | H 5,88 | N 19,23 |
| Gef.: | 71,98 | 6,02 | 19,11 |

Beispiel 112

4'-[[2-Ethyl-6-(3-benzyl-3,4,5,6-tetrahydro-2(1H)-pyrimidinon-1-yl)-benzimidazol-1-yl]methyl]-2-(1H)-tetrazol-5-yl)biphenyl

Hergestellt analog Beispiel 53 aus 4'-[[2-Ethyl-6-(3-benzyl-3,4,5,6-tetrahydro-2(1H)-pyrimidinon-1-yl)-benzimidazol-1-yl]methyl]-2-(1-triphenylmethyl-tetrazol-5-yl)-biphenyl und Methanol in methanolischer Salzsäure.
Ausbeute: 22,0 % der Theorie,
Schmelzpunkt: ab 106°C (Zers.)

| $C_{34}H_{32}N_8O$ (568,68) | | | |
|---|---|---|---|
| Ber.: | C 71,81 | H 5,67 | N 19,70 |
| Gef.: | 71,73 | 5,54 | 19,92 |

Beispiel 113

4'-[[2-n-Butyl-6-(4,5-dihydro-2H-pyridazin-3-on-6-yl)-benzimidazol-1-yl]methyl]biphenyl-2-carbonsäure

Hergestellt aus 4'-[[2-n-Butyl-6-(4,5-dihydro-2H-pyridazin-3-on-6-yl)-benzimidazol-1-yl]methyl]biphenyl-2-carbonsauremethylester und Natronlauge in Ethanol.
Ausbeute: 80,0 % der Theorie,
Schmelzpunkt: 276-283°C

| $C_{29}H_{28}N_4O_3$ (480,60) | | | |
|---|---|---|---|
| Ber.: | C 72,48 | H 5,87 | N 11,66 |
| Gef.: | 72,20 | 6,13 | 11,53 |

Massenspektrum: m/e = 480

Analog Beispiel 113 werden folgende Verbindungen erhalten:

4'-[[2-Ethyl-6-(2H-pyridazin-3-on-6-yl)-benzimidazol-1-yl]methyl]biphenyl-2-carbonsäure

4'-[[2-n-Propyl-6-(2H-pyridazin-3-on-6-yl)-benzimidazol-1-yl]methyl]biphenyl-2-carbonsäure

4'-[[2-n-Butyl-6-(2H-pyridazin-3-on-6-yl)-benzimidazol-1-yl]methyl]biphenyl-2-carbonsäure

4'-[[2-n-Propyl-6-(2-methyl-4,5-dihydro-pyridazin-3-on-6-yl)-benzimidazol-1-yl]methyl]biphenyl-2-carbonsäure

4'-[[2-n-Propyl-6-(2-benzyl-4,5-dihydro-pyridazin-3-on-6-yl)-benzimidazol-1-yl]methyl]biphenyl-2-carbonsäure

Beispiel 114

4'-[[2-n-Propyl-6-(4,5-dihydro-2H-pyridazin-3-on-6-yl)-benzimidazol-1-yl]methyl]biphenyl-2-carbonsäure

Hergestellt analog Beispiel 113 aus 4'-[[2-n-Propyl-6-(4,5-dihydro-2H-pyridazin-3-on-6-yl)-benzimidazol-1-yl]methyl]biphenyl-2-carbonsäure-methylester und Natronlauge in Ethanol. Ausbeute: 66,0 % der Theorie,
Schmelzpunkt: 236-241°C

| $C_{28}H_{26}N_4O_3$ (466,54) | | | |
|---|---|---|---|
| Ber.: | C 72,09 | H 5,62 | N 12,01 |
| Gef.: | 71,88 | 5,61 | 11,95 |

Beispiel 115

4'-[[2-Ethyl-6-(4,5-dihydro-2H-pyridazin-3-on-6-yl)-benzimidazol-1-yl]methyl]biphenyl-2-carbonsäure

Hergestellt analog Beispiel 113 aus 4'-[[2-Ethyl-6-(4,5-dihydro-2H-pyridazin-3-on-6-yl)-benzimidazol-1-yl]methyl] biphenyl-2-carbonsäure-methylester und Natronlauge in Ethanol.
Ausbeute: 71,0 % der Theorie,
Schmelzpunkt: 255-257°C

| $C_{27}H_{24}N_4O_3$ (452,51) | | | |
|---|---|---|---|
| Ber.: | C 71,67 | H 5,35 | N 12,38 |
| Gef.: | 71,41 | 5,51 | 12,12 |

Beispiel 116

4'-[[2-n-Butyl-6-(3-cyclohexyl-propylamino)-benzimidazol-1-yl]methyl]biphenyl-2-carbonsäure

Hergestellt analog Beispiel 1 aus 4'-[[2-n-Butyl-6-(3-cyclohexyl-propylamino)-benzimidazol-1-yl]methyl]biphenyl-2-carbonsäure-tert.butylester und Trifluoressigsäure in Methylenchlorid.
Ausbeute: 85,7 % der Theorie,
Schmelzpunkt: 152-153°C

| $C_{34}H_{11}N_3O_2$ x 0,75 $CF_3COOH$ (609,24) | | | |
|---|---|---|---|
| Ber.: | C 69,99 | H 6,91 | N 6,90 |
| Gef.: | 70,02 | 6,93 | 6,84 |

$R_f$-Wert: 0,24 (Kieselgel; Essigester/Ethanol/Ammoniak = 80:40:2)

Bei den nachfolgenden pharmazeutischen Anwendungsbeispielen kann als Wirksubstanz jede geeignete Verbindung der Formel I eingesetzt werden, insbesondere die Verbindungen A bis L des pharmakologischen Versuchsberichtes:

Beispiel I

| Ampullen, enthaltend 50 mg Wirkstoff pro 5 ml | |
|---|---|
| Wirkstoff | 50 mg |
| $KH_2PO_4$ | 2 mg |
| $Na_2HPO_4$ x $2H_2O$ | 50 mg |
| NaCl | 12 mg |
| Wasser für Injektionszwecke ad | 5 ml |

Herstellung:

In einem Teil des Wassers werden die Puffersubstanzen und das Isotonans gelöst. Der Wirkstoff wird zugegeben und nach vollständiger Lösung mit Wasser auf das Nennvolumen aufgefüllt.

Beispiel II

| Ampullen, enthaltend 100 mg Wirkstoff pro 5 ml | |
|---|---|
| Wirkstoff | 100 mg |
| Methylglucamin | 35 mg |
| Glykofurol | 1000 mg |
| Polyethylenglykol-Polypropylenglykol-Blockpolymer | 250 mg |
| Wasser für Injektionszwecke ad | 5 ml |

Herstellung:

In einem Teil des Wassers wird Methylglucamin gelöst und der Wirkstoff unter Rühren und Erwärmen in Lösung gebracht. Nach Zugabe der Lösungsmittel wird mit Wasser auf das Nennvolumen aufgefüllt.

Beispiel III

| Tabletten, enthaltend 50 mg Wirkstoff | |
|---|---|
| Wirkstoff | 50,0 mg |
| Calciumphosphat | 70,0 mg |
| Milchzucker | 40,0 mg |
| Maisstärke | 35,0 mg |
| Polyvinylpyrrolidon | 3,5 mg |
| Magnesiumstearat | 1,5 mg |
| | 200,0 mg |

Herstellung:

Der Wirkstoff, $CaHPO_4$, Milchzucker und Maisstärke werden mit einer wässrigen PVP-Lösung gleichmäßig befeuchtet. Die Masse wird durch ein 2-mm-Sieb gegeben, im Umlufttrockenschrank bei 50°C getrocknet und erneut gesiebt.

Nach Zumischen des Schmiermittels wird das Granulat auf einer Tablettiermaschine verpresst.

Beispiel IV

| Dragees, enthaltend 50 mg Wirkstoff | |
|---|---|
| Wirkstoff | 50,0 mg |

| | |
|---|---|
| Lysin | 25,0 mg |
| Milchzucker | 60,0 mg |
| Maisstärke | 34,0 mg |
| Gelatine | 10,0 mg |
| Magnesiumstearat | 1,0 mg |
| | 180,0 mg |

Herstellung:

Der Wirkstoff wird mit den Hilfsstoffen gemischt und mit einer wässrigen Gelatine-Lösung befeuchtet. Nach Siebung und Trocknung wird das Granulat mit Magnesiumstearat vermischt und zu Kernen verpresst.

Die so hergestellten Kerne werden nach bekannten Verfahren mit einer Hülle überzogen. Der Dragiersuspension oder -lösung kann Farbstoff zugegeben werden.

Beispiel V

| Dragees, enthaltend 100 mg Wirkstoff | |
|---|---|
| Wirkstoff | 100,0 mg |
| Lysin | 50,0 mg |
| Milchzucker | 86,0 mg |
| Maisstärke | 50,0 mg |
| Polyvinylpyrrolidon | 2,8 mg |
| Mikrokristalline Cellulose | 60,0 mg |
| Magnesiumstearat | 1,2 mg |
| | 350,0 mg |

Herstellung:

Der Wirkstoff wird mit den Hilfsstoffen gemischt und mit einer wässrigen PVP-Lösung befeuchtet. Die feuchte Masse wird durch ein 1,5-mm-Sieb gegeben und bei 45°C getrocknet.

Nach dem Trocknen wird erneut gesiebt und das Magnesiumstearat zugemischt. Diese Mischung wird zu Kernen verpreßt.

Die so hergestellten Kerne werden nach bekannten Verfahren mit einer Hülle überzogen. Der Dragiersuspension oder -lösung können Farbstoffe zugegeben werden.

Beispiel VI

| Kapseln, enthaltend 250 mg Wirkstoff | |
|---|---|
| Wirkstoff | 250,0 mg |
| Maisstärke | 68,5 mg |
| Magnesiumstearat | 1,5 mg |
| | 320,0 mg |

Herstellung:

Wirkstoff und Maisstärke werden gemischt und mit Wasser befeuchtet. Die feuchte Masse wird gesiebt und getrocknet. Das trockene Granulat wird gesiebt und mit Magnesiumstearat gemischt. Die Endmischung wird in Hartgelatinekapseln Größe 1 abgefüllt.

Beispiel VII

Orale Suspension, enthaltend 50 mg Wirkstoff pro 5 ml

| | |
|---|---|
| Wirkstoff | 50,0 mg |
| Hydroxyethylcellulose | 50,0 mg |
| Sorbinsäure | 5,0 mg |
| Sorbit 70%ig | 600,0 mg |
| Glycerin | 200,0 mg |
| Aroma | 15,0 mg |
| Wasser ad | 5,0 ml |

Herstellung:

Destilliertes Wasser wird auf 70°C erhitzt. Hierin wird unter Rühren Hydroxyethylcellulose gelöst. Durch Zugabe von Sorbitlösung und Glycerin wird auf Raumtemperatur abgekühlt. Bei Raumtemperatur werden Sorbinsäure, Aroma und Wirkstoff zugegeben. Zur Entlüftung der Suspension wird unter Rühren evakuiert. Eine Dosis = 50 mg ist enthalten in 5,0 ml.

Beispiel VIII

| Suppositorien, enthaltend 100 mg Wirkstoff | |
|---|---|
| Wirkstoff | 100,0 mg |
| Adeps solidus | 1600,0 mg |
| | 1700,0 mg |

Herstellung:

Das Hartfett wird geschmolzen. Bei 40°C wird die gemahlene Wirksubstanz in der Schmelze homogen dispergiert. Es wird auf 38°C abgekühlt und in schwach vorgekühlte Suppositorienformen ausgegossen.

**Patentansprüche**

**1.** Benzimidazole der allgemeinen Formel

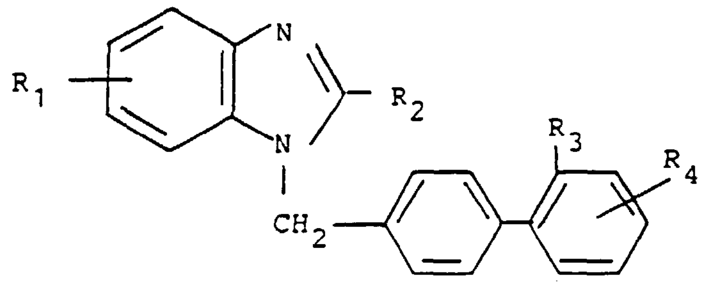

, (I)

in der

$R_1$ eine Tetrahydrobenzimidazolyl- oder Imidazopyridinylgruppe,

eine gegebenenfalls im Phenylkern durch ein Fluor-, Chlor- oder Bromatom, durch eine Alkylgruppe mit 1 bis 3 Kohlenstoffatomen, durch eine Alkoxygruppe mit 1 bis 3 Kohlenstoffatomen oder durch eine Trifluormethylgruppe substituierte Benzimidazolyl- oder Benzoxazolylgruppe, wobei die NH-Gruppe der vorstehend erwähnten Imidazolringe zusätzlich durch eine Alkylgruppe mit 1 bis 6 Kohlenstoffatomen oder durch eine Cycloalkylgruppe mit 3 bis 7 Kohlenstoffatomen substituiert sein kann,

eine durch eine Bicyclohexylcarbonyl- oder Biphenylcarbonylgruppe substituierte Aminogruppe, die am N-Atom jeweils zusätzlich durch eine Alkylgruppe mit 1 bis 3 Kohlenstoffatomen substituiert sein können,

eine durch eine Bicyclohexyl- oder Biphenylgruppe substituierte Aminocarbonylaminogruppe, die zusätzlich durch eine oder zwei Alkylgruppen mit jeweils 1 bis 3 Kohlenstoffatomen am N-Atom substituiert sein kann,

mit Ausnahme der 2-Oxo-3,4-tetramethylen-pyrrolidin-1-yl-gruppe eine gegebenenfalls durch eine oder zwei Alkylgruppen mit 1 bis 3 Kohlenstoffatomen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt ist,

eine gegebenenfalls durch eine Alkyl- oder Phenylalkylgruppe mit jeweils 1 bis 3 Kohlenstoffatomen im Alkylteil substituierte 3,4,5,6-Tetrahydro-2(1H)-pyrimidinongruppe,

eine gegebenenfalls durch eine Alkylgruppe mit 1 bis 3 Kohlenstoffatomen oder durch eine Phenylgruppe mono- oder disubstituierte Maleinsäureamido- oder Maleinsäureimidogruppe, wobei die Substituenten gleich oder verschieden sein können,

eine gegebenenfalls durch eine Alkylgruppe mit 1 bis 6 Kohlenstoffatomen oder durch eine Cycloalkylgruppe mit 3 bis 7 Kohlenstoffatomen substituierte Imidazolin- oder Imidazolgruppe,

eine gegebenenfalls durch eine Alkylgruppe mit 1 bis 3 Kohlenstoffatomen, durch eine Phenylalkylgruppe mit 1 bis 3 Kohlenstoffatomen im Alkylteil, durch eine Tetramethylen-, Pentamethylen- oder Hexamethylengruppe substituierte Imidazolidindiongruppe,

eine durch eine Alkylsulfonylgruppe mit 4 bis 6 Kohlenstoffatomen oder durch eine Phenylalkylsulfonylgruppe substituierte Alkylamino- oder Phenylalkylaminogruppe, in denen der Alkylteil jeweils 1 bis 3 Kohlenstoffatome enthalten kann,

eine durch eine Naphthalinsulfonylgruppe substituierte Amino- oder Alkylaminogruppe, welche im Naphthalinring durch eine Dialkylaminogruppe, durch eine oder zwei Alkoxygruppen substituiert sein können, wobei der Alkylteil jeweils 1 bis 3 Kohlenstoffatome enthalten kann,

eine Pyridazin-3-on- oder Dihydro-pyridazin-3-on-gruppe, die in 2-Stellung durch eine gegebenenfalls durch eine Phenylgruppe substituierte Alkylgruppe mit 1 bis 3 Kohlenstoffatomen und zusätzlich im Kohlenstoffgerüst durch 1 oder 2 Alkylgruppen mit jeweils 1 bis 3 Kohlenstoffatomen substituiert sein kann,

eine durch 2 Alkylgruppen mit jeweils 1 bis 3 Kohlenstoffatomen substituierte Pyrrolidino-, Piperidino- oder Hexamethyleniminogruppe,

eine 7-Nitro-benzofurazan-4-yl-aminoalkanoylaminogruppe, in der der Alkanoylteil 2 oder 3 Kohlenstoffatome enthalten kann,

eine Heptamethylenimino-, 1H,3H-Chinazolin-2,4-dion-3-yl-, Pentamethylen-oxazolin-2-yl-, Benzofurancarbonylamino- oder 7-Nitro-benzofurazan-4-yl-amino-gruppe

oder, wenn $R_3$ eine Carboxygruppe und $R_2$ eine n-Butylgruppe darstellen, $R_1$ in 6-Stellung auch eine durch eine Phenylsulfonyl-, Cyclohexylmethylaminocarbonyl-, 2-Carboxycyclohexylmethylcarbonyl-, 2-tert.Butoxycarbonyl-cyclohexylmethylcarbonyl-, 2-Carboxy-3,4,5,6-tetrahydrobenzoyl-, N-Methylphenylaminocarbonyl- oder 3-Cyclohexylpropylgruppe substituierte Aminogruppe, eine durch eine Propylsulfonyl-, Phenylsulfonyl-, Methylphenylsulfonyl- oder Chlorphenylsulfonylgruppe substituierte Methylaminogruppe, eine durch eine Phenylsulfonyl- oder Methoxyphenylsulfonylgruppe substituierte n-Pentylaminogruppe, eine durch eine Methylphenylsulfonyl- oder Methoxyphenylsulfonylgruppe substituierte n-Propylaminogruppe, eine durch eine Benzoyl- oder Chlorphenylsulfonylgruppe substituierte Isopropylaminogruppe, eine N-Acetylcyclohexylmethylamino-, 3,4,5,6-Tetrahydrophthalimido-, Hexahydrohomophthalimido-, N-Methansulfonyl-2-phenylethylamino-, N-Chlorphenylsulfonyl-benzylamino-, Piperidino-, 4-Methyl-piperidino- oder Hexamethyleniminogruppe

oder, wenn $R_3$ eine Carboxygruppe und $R_2$ eine n-Butylgruppe darstellen, $R_1$ in 5- oder 6-Stellung auch eine 2-Oxo-1,2-dihydro-3,4-tetramethylen-pyrrolidin-1-yl-gruppe

oder, wenn $R_3$ eine Carboxygruppe und $R_2$ eine Methyl-, Ethyl-, n-Propyl-, n-Butyl- oder Methylmercaptogruppe darstellen, $R_1$ in 6-Stellung auch eine Pyrrolidinocarbonylaminogruppe

oder, wenn $R_3$ eine Tetrazolylgruppe und $R_2$ eine n-Butylgruppe darstellen, $R_1$ in 5- oder 6-Stellung auch eine durch eine Methylaminocarbonyl- oder Cyclohexylaminocarbonylgruppe substituierte n-Pentylaminogruppe oder in 6-Stellung eine 3,3-Dimethyl-glutarsäureimido- oder 4,4-Tetramethylen-glutarsäureimidogruppe,

oder, wenn $R_3$ eine Tetrazolylgruppe und $R_2$ eine Ethyl- oder n-Propylgruppe darstellen, $R_1$ in 6-Stellung auch eine N-Benzolsulfonyl-methylaminogruppe

oder, wenn $R_3$ eine tert.Butoxycarbonylgruppe und $R_2$ eine N-Butylgruppe darstellen, $R_1$ in 6-Stellung auch eine 2-Carboxycyclohexylmethylcarbonylamino- oder Pyrrolidinocarbonylaminogruppe,

$R_2$ ein Wasserstoffatom oder eine geradkettige oder verzweigte Alkylgruppe mit 1 bis 5 Kohlenstoffatomen, in welcher eine Methylengruppe durch ein Schwefelatom ersetzt sein kann,

$R_3$ eine Carboxy-, Cyano-, 1H-Tetrazolyl- oder 1-Triphenylmethyl-tetrazolylgruppe oder eine Alkoxycarbonylgruppe mit insgesamt 2 bis 5 Kohlenstoffatomen und

$R_4$ ein Wasserstoff-, Fluor-, Chlor- oder Bromatom bedeuten,

deren 1-, 3-Isomerengemische und deren Salze mit anorganischen oder organischen Säuren oder Basen

sowie die Verbindungen

4'[[2-n-Butyl-6-(piperidin-1-yl)-benzimidazol-1-yl]methyl]biphenyl-2-carbonsäure und

4'[[2-n-Butyl-6-(4-methylpiperidin-1-yl)-benzimidazol-1-yl]methyl]biphenyl-2-carbonsäure.

**2.** Benzimidazole der allgemeinen Formel I gemäß Anspruch 1, in der

$R_1$ eine Tetrahydrobenzimidazolyl- oder Imidazopyridinylgruppe,

eine gegebenenfalls im Phenylkern durch ein Fluor-, Chlor- oder Bromatom, durch eine Methyl-, Methoxy- oder Trifluormethylgruppe substituierte Benzimidazolylgruppe, wobei die NH-Gruppe der vorstehend erwähnten Imidazolringe zusätzlich durch eine Alkylgruppe mit 1 bis 6 Kohlenstoffatomen oder durch eine Cycloalkylgruppe mit 3 bis 6 Kohlenstoffatomen substituiert sein kann,

eine gegebenenfalls durch eine Methylgruppe substituierte Benzoxazol-2-yl-gruppe,

eine durch eine Bicyclohexylcarbonyl-, Biphenylcarbonyl- oder Benzofuryl-2-carbonylgruppe substituierte Aminogruppe,

eine in 3-Stellung durch eine Bicyclohexyl- oder Biphenylgruppe substituierte Aminocarbonylaminogruppe,

eine gegebenenfalls durch eine oder zwei Methylgruppen oder durch eine Tetramethylen- oder Pentamethylengruppe substituierte 5-, 6- oder 7-gliedrige Alkylenimino- oder Alkenyleniminogruppe, in welcher eine Methylengruppe durch eine Carbonyl- oder Sulfonylgruppe ersetzt ist,

eine gegebenenfalls durch eine Methyl- oder Benzylgruppe substituierte 3,4,5,6-Tetrahydro-2(1H)-pyrimidinongruppe,

eine gegebenenfalls durch eine Methylgruppe oder durch eine Phenylgruppe mono- oder disubstituierte Maleinsäureamido- oder Maleinsäureimidogruppe, wobei die Substituenten gleich oder verschieden sein können,

eine in 1-Stellung durch eine Alkylgruppe mit 1 bis 6 Kohlenstoffatomen oder durch eine Cycloalkylgruppe mit 3 bis 7 Kohlenstoffatomen substituierte Imidazolin-2-yl- oder Imidazol-2-yl-gruppe,

eine gegebenenfalls durch eine Methyl-, Benzyl-, Tetramethylen- oder Pentamethylengruppe substituierte Imidazolidindiongruppe,

eine durch eine Butansulfonylgruppe oder durch eine Phenylmethansulfonylgruppe substituierte Methylamino- oder Benzylaminogruppe,

eine durch eine Naphthalinsulfonylgruppe substituierte Amino- oder Methylaminogruppe, welche im Naphthalinring durch eine Dimethylaminogruppe oder durch 2 Methoxygruppen substituiert sein können,

eine gegebenenfalls durch eine Methyl- oder Benzylgruppe substituierte Pyridazin-3-on- oder Dihydro-pyridazin-3-on-gruppe,

eine durch zwei Methylgruppen substituierte Pyrrolidino-, Piperidino- oder Hexamethyleniminogruppe,

eine Heptamethylenimino-, 1H,3H-Chinazolin-2,4-dion-3-yl-, 4,5-Pentamethylen-oxazolin-2-yl-, 7-Nitro-benzofurazan-4-yl-amino- oder 7-Nitro-benzofurazan-4-yl-aminopropionylaminogruppe

oder, wenn $R_3$ eine Carboxygruppe und $R_2$ eine n-Butylgruppe darstellen, $R_1$ in 6-Stellung auch eine durch eine Phenylsulfonyl-, Cyclohexylmethylaminocarbonyl-, 2-Carboxycyclohexylmethylcarbonyl-, 2-tert.Butoxycarbonyl-cyclohexylmethylcarbonyl-, 2-Carboxy-3,4,5,6-tetrahydrobenzoyl-, N-Methyl-phenylaminocarbonyl- oder 3-Cyclohexylpropylgruppe substituierte Aminogruppe, eine durch eine Propylsulfonyl-, Phenylsulfonyl-, 4-Methylphenylsulfonyl- oder 4-Chlorphenylsulfonylgruppe substituierte Methylaminogruppe, eine durch eine Phenylsulfonyl- oder 4-Methoxyphenylsulfonylgruppe substituierte n-Pentylaminogruppe, eine durch eine 4-Methylphenylsulfonyl- oder 4-Methoxyphenylsulfonylgruppe substituierte n-Propylaminogruppe, eine durch eine Benzoyl- oder 4-Chlorphenylsulfonylgruppe substituierte Isopropylaminogruppe, eine N-Acetyl-cyclohexylmethylamino-, 3,4,5,6-Tetrahydrophthalimido-, Hexahydrohomophthalimido-, N-Methansulfonyl-2-phenylethylamino-, N-(4-Chlorphenylsulfonyl)-benzylamino-, Piperidino-, 4-Methyl-piperidino- oder Hexamethyleniminogruppe

oder, wenn $R_3$ eine Carboxygruppe und $R_2$ eine n-Butylgruppe darstellen, $R_1$ in 5- oder 6-Stellung auch eine 2-Oxo-1,2-dihydro-3,4-tetramethylen-pyrrolidin-1-yl-gruppe

oder, wenn $R_3$ eine Carboxygruppe und $R_2$ eine Methyl-, Ethyl-, n-Propyl-, n-Butyl- oder Methylmercaptogruppe darstellen, $R_1$ in 6-Stellung auch eine Pyrrolidinocarbonylaminogruppe

oder, wenn $R_3$ eine Tetrazolylgruppe und $R_2$ eine n-Butylgruppe darstellen, $R_1$ in 5- oder 6-Stellung auch eine durch eine Methylaminocarbonyl- oder Cyclohexylaminocarbonylgruppe substituierte n-Pentylaminogruppe oder in 6-Stellung eine 3,3-Dimethyl-glutarsäureimido- oder 4,4-Tetramethylen-glutarsäureimidogruppe,

oder, wenn $R_3$ eine Tetrazolylgruppe und $R_2$ eine Ethyl- oder n-Propylgruppe darstellen, $R_1$ in 6-Stellung auch eine N-Benzolsulfonyl-methylaminogruppe

oder, wenn $R_3$ eine tert.Butoxycarbonylgruppe und $R_2$ eine N-Butylgruppe darstellen, $R_1$ in 6-Stellung auch eine 2-Carboxy-cyclohexylmethylcarbonylamino- oder Pyrrolidinocarbonylaminogruppe,

$R_2$ ein Wasserstoffatom oder eine geradkettige oder verzweigte Alkylgruppe mit 1 bis 4 Kohlenstoffatomen, in welcher eine Methylengruppe durch ein Schwefelatom ersetzt sein kann,

$R_3$ eine Carboxy-, Cyano-, 1H-Tetrazolyl- oder 1-Triphenylmethyl-tetrazolylgruppe oder eine Alkoxycarbonylgruppe mit insgesamt 2 bis 5 Kohlenstoffatomen und

$R_4$ ein Wasserstoff-, Fluor-, Chlor- oder Bromatom bedeuten,

deren 1-, 3-Isomerengemische sowie deren Salze mit anorganischen oder organischen Säuren oder Basen.

**3.** Benzimidazole der allgemeinen Formel I gemäß Anspruch 1, in der

$R_1$ in 6-Stellung eine 1-Methylbenzimidazol-2-yl-, 3,4,5,6-Tetrahydro-phthalimino-, 2,3-Diphenyl-maleinsäureimido-, 2,3-Dimethyl-maleinsäureimido-, N-Phenylmethansulfonyl-methylamino-, 2-Oxo-pyrrolidin-1-yl-, 2-Oxo-piperidin-1-yl-, 2-Oxo-hexamethylenimino-, 2-Oxo-3,4-tetramethylen-pyrrolidin-2-yl-, 3,3-Dimethylglutarimido-, N-Methylaminocarbonyl-n-pentylamino-, Propansultam-1-yl- oder Butansultam-1-yl-gruppe,

$R_2$ die Methyl-, Ethyl-, n-Propyl- oder n-Butylgruppe,

$R_3$ eine Carboxy- oder 1H-Tetrazolylgruppe und

$R_4$ ein Wasserstoffatom bedeuten,

und deren Salze mit organischen oder anorganischen Säuren oder Basen.

**4.** Benzimidazole der allgemeinen Formel I gemäß Anspruch 1:

4'-[[2-n-Propyl-6-(1-methylbenzimidazol-2-yl)-benzimidazol-1-yl]methyl]biphenyl-2-carbonsäure,

4'-[[2-n-Butyl-6-(3,4,5,6-tetrahydro-phthalimino)-benzimidazol-1-yl]methyl]biphenyl-2-carbonsäure,

4'-[[2-n-Butyl-6-(2,3-diphenyl-maleinsäureimido)-benzimidazol-1-yl]methyl]biphenyl-2-carbonsäure,

4'-[[2-n-Butyl-6-(2,3-dimethyl-maleinsäureimido)-benzimidazol-1-yl]methyl]biphenyl-2-carbonsäure,

4'-[[2-n-Butyl-6-(N-phenylmethansulfonyl-methylamino)-benzimidazol-1-yl]methyl]biphenyl-2-carbonsäure,

4'-[[2-n-Butyl-6-(2-oxo-piperidin-1-yl)-benzimidazol-1-yl]methyl]-2-(1H-tetrazol-5-yl)-biphenyl,

4'-[[2-n-Butyl-6-(2-oxo-pyrrolidin-1-yl)-benzimidazol-1-yl]methyl]-2-(1H-tetrazol-5-yl)-biphenyl,

4'-[[2-n-Butyl-6-(2-oxo-hexamethylenimino)-benzimidazol-1-yl]methyl]-2-(1H-tetrazol-5-yl)-biphenyl,

4'-[[2-n-Butyl-6-(3,3-dimethylglutarimido)-benzimidazol-1-yl]methyl]-2-(1H-tetrazol-5-yl)-biphenyl,

4'-[[2-n-Butyl-6-(N-methylaminocarbonyl-n-pentylamino)-benzimidazol-1-yl]methyl]-2-(1H-tetrazol-5-yl)-biphenyl,

4'-[[2-n-Butyl-6-(cyclohexylaminocarbonyl-n-pentylamino)-benzimidazol-1-yl]methyl]-2-(1H-tetrazol-5-yl)-biphenyl,

4'-[[2-n-Butyl-6-(2-oxo-3,4-tetramethylen-pyrrolidin-1-yl)benzimidazol-1-yl]methyl]biphenyl-2-carbonsäure,

4'-[[2-n-Butyl-6-(propansultam-1-yl)-benzimidazol-1-yl]methyl]-2-(1H-tetrazol-5-yl)-biphenyl,

4'-[[2-n-Propyl-6-(butansultam-1-yl)-benzimidazol-1-yl]methyl]-2-(1H-tetrazol-5-yl)-biphenyl,

4'-[[2-n-Butyl-6-(butansultam-1-yl)-benzimidazol-1-yl]methyl]-2-(1H-tetrazol-5-yl)-biphenyl,

4'-[[2-n-Propyl-6-(1-methyl-benzimidazol-2-yl)-benzimidazol-1-yl]methyl]-2-(1H-tetrazol-5-yl)-biphenyl und

4'-[[2-n-Propyl-6-(2-oxo-piperidin-1-yl)-benzimidazol-1-yl]methyl]-2-(1H-tetrazol-5-yl)-biphenyl

und deren Salze mit anorganischen oder organischen Säuren oder Basen.

**5.** Physiologisch verträgliche Salze der Verbindungen nach mindestens einem der Ansprüche 1 bis 4 mit anorganischen oder organischen Säuren oder Basen.

**6.** Arzneimittel, enthaltend eine Verbindung der allgemeinen Formel I, in der $R_1$, $R_2$ und $R_4$ sowie $R_3$ mit Ausnahme der tert.Butoxycarbonyl-, Cyano- und 1-Triphenylmethyl-tetrazolylgruppe wie mindestens in einem der Ansprüche 1 bis 4 definiert sind oder ein physiologisch verträgliches Salz hiervon gemäß Anspruch 5 und gegebenenfalls eine weitere Wirksubstanz neben gegebenenfalls einem oder mehreren inerten Trägerstoffen und/oder Verdünnungsmitteln.

**7.** Verwendung einer Verbindung der allgemeinen Formel I, in der $R_1$, $R_2$ und $R_4$ sowie $R_3$ mit Ausnahme der tert. Butoxycarbonyl-, Cyano- und l-Triphenylmethyl-tertrazolylgruppe wie mindestens in einem der Ansprüche 1 bis 4 definiert sind oder ein physiologisch verträgliches Salz hiervon gemäß Anspruch 5 zur Herstellung eines Arzneimittels mit Angiotensin-antagonistischer Wirkung.

**8.** Verfahren zur Herstellung eines Arzneimittels gemäß Anspruch 6, dadurch gekennzeichnet, daß auf nichtchemischem Wege eine Verbindung der allgemeinen Formel I, in der $R_1$, $R_2$ und $R_4$ sowie $R_3$ mit Ausnahme der tert. Butoxycarbonyl-, Cyano- und l-Triphenylmethyl-tertrazolylgruppe wie mindestens in einem der Ansprüche 1 bis 4 definiert sind oder ein physiologisch verträgliches Salz hiervon in einen oder mehrere inerte Trägerstoffe und/oder Verdünnungsmittel eingearbeitet wird.

**9.** Verfahren zur Herstellung der Benzimidazole gemäß den Ansprüchen 1 bis 5, dadurch gekennzeichnet, daß

a) eine Verbindung der allgemeinen Formel

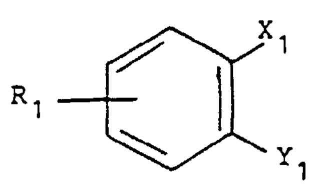

,(II)

in der

$R_1$ wie in den Ansprüchen 1 bis 4 definiert ist,
einer der Reste $X_1$ oder $Y_1$ eine Gruppe der allgemeinen Formel

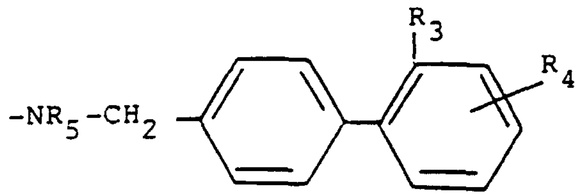

und der andere der Reste $X_1$ oder $Y_1$ eine Gruppe der allgemeinen Formel

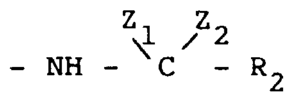

darstellen, wobei
$R_2$ bis $R_4$ wie in den Ansprüchen 1 bis 4 definiert sind,
$R_5$ ein Wasserstoffatom oder eine $R_2CO$-Gruppe, wobei $R_2$ wie vorstehend erwähnt definiert ist,
$Z_1$ und $Z_2$, die gleich oder verschieden sein können, gegebenenfalls substituierte Aminogruppen oder gegebenenfalls durch niedere Alkylgruppen substituierte Hydroxy- oder Mercaptogruppen oder
$Z_1$ und $Z_2$, zusammen ein Sauerstoff- oder Schwefelatom, eine gegebenenfalls durch eine Alkylgruppe mit 1 bis 3 Kohlenstoffatomen substituierte Iminogruppe, eine Alkylendioxy- oder Alkylendithiogruppe mit jeweils 2 oder 3 Kohlenstoffatomen bedeuten, wobei jedoch einer der Reste $X_1$ oder $Y_1$ eine Gruppe der allgemeinen Formel

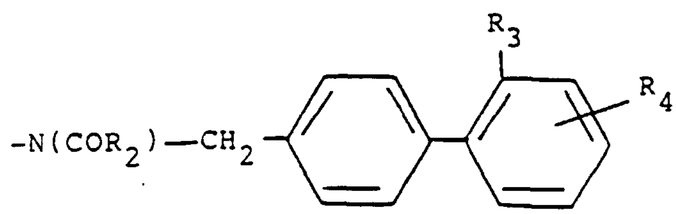

oder

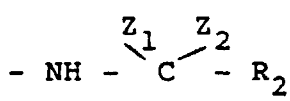

darstellen muß, cyclisiert und anschließend ein so gegebenenfalls erhaltenes N-Oxid reduziert wird oder

b) ein Benzimidazol der allgemeinen Formel

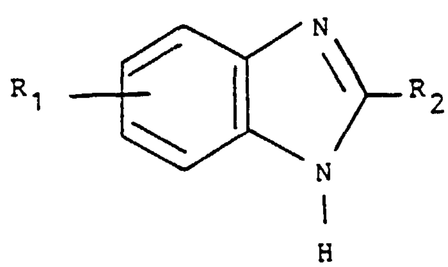

,(III)

in der

$R_1$ und $R_2$ wie in den Ansprüchen 1 bis 4 definiert sind, mit einer Biphenylverbindung der allgemeinen Formel

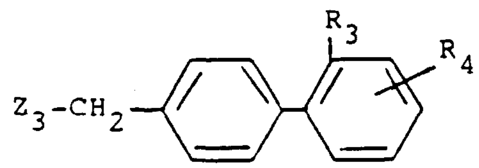

, (IV)

in der

$R_3$ und $R_4$ wie in den Ansprüchen 1 bis 4 definiert sind und

$Z_3$ eine nukleophile Austrittsgruppe wie ein Halogenatom oder eine substituierte Sulfonyloxygruppe darstellt, umgesetzt wird oder

c) zur Herstellung einer Verbindung der allgemeinen Formel I, in der $R_3$ eine Carboxygruppe darstellt, eine Verbindung der allgemeinen Formel

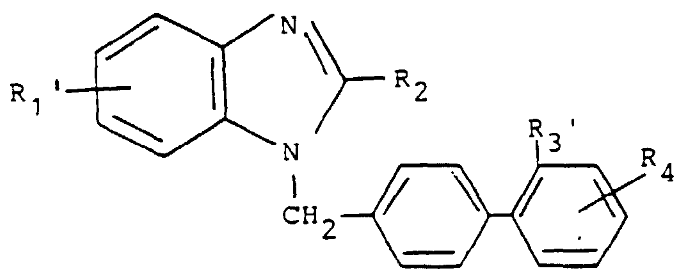

, (V)

in der

$R_2$ und $R_4$ wie in den Ansprüchen 1 bis 4 definiert sind,

$R_1'$ die für $R_1$ in den Ansprüchen 1 bis 4 erwähnten Bedeutungen besitzt und eine 3-Alkoxycarbonylpropionyl- oder 3-Alkoxycarbonyl-2-methyl-propionylgruppe, in der der Alkoxyteil jeweils 1 bis 3 Kohlenstoffatome enthalten kann, und

$R_3'$ eine mittels Hydrolyse, Thermolyse oder Hydrogenolyse in eine Carboxygruppe überführbare Gruppe darstellt, in eine entsprechende Carboxyverbindung übergeführt wird oder

d) zur Herstellung einer Verbindung der allgemeinen Formel I, in der $R_3$ eine 1H-Tetrazolylgruppe darstellt, von einer Verbindung der allgemeinen Formel

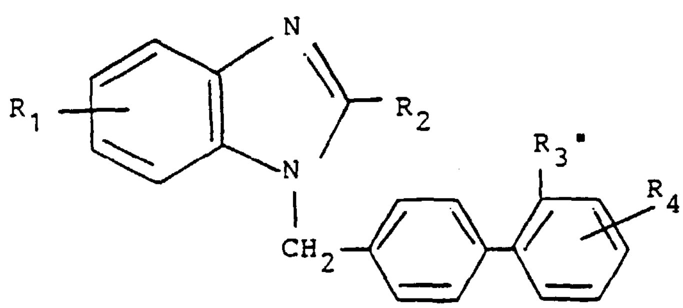

, (VI)

in der

$R_1$, $R_2$ und $R_4$ wie in den Ansprüchen 1 bis 4 definiert sind und

$R_3''$ eine in 1- oder 3-Stellung durch einen Schutzrest geschützte 1H-Tetrazolylgruppe darstellt, ein Schutzrest abgespalten wird oder

e) zur Herstellung einer Verbindung der allgemeinen Formel I, in der $R_3$ eine 1H-Tetrazolylgruppe darstellt, eine Verbindung der allgemeinen Formel

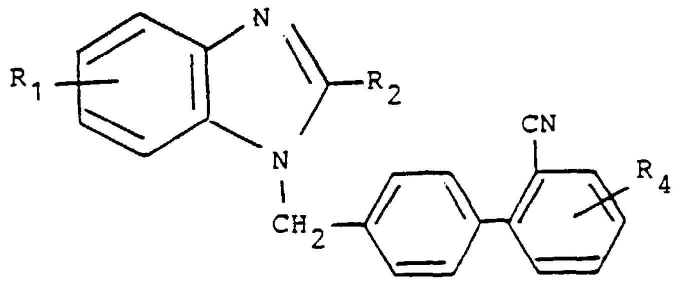

,(VII)

in der
$R_1$, $R_2$ und $R_4$ wie in den Ansprüchen 1 bis 4 definiert sind, mit Stickstoffwasserstoffsäure oder mit deren Salzen umgesetzt wird oder

f) zur Herstellung von Verbindungen der allgemeinen Formel I, in der $R_1$ eine Pentamethylen-oxazolin-2-yl-gruppe darstellt, eine Verbindung der allgemeinen Formel

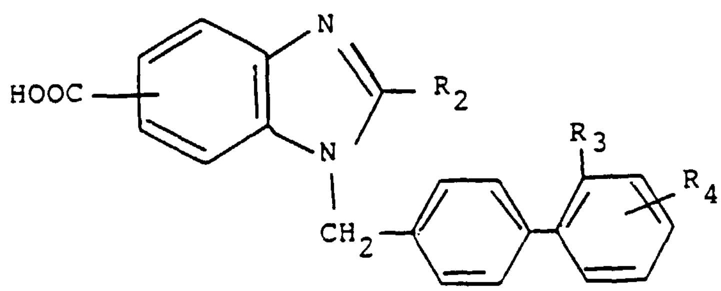

,(VIII)

in der

$R_2$ bis $R_4$ wie in den Ansprüchen 1 bis 4 definiert sind,
mit 1-Aminomethyl-cyclohexanol in Gegenwart eines die Säure aktivierenden Mittels umgesetzt wird oder

g) zur Herstellung einer Verbindung der allgemeinen Formel I, in der $R_1$ eine 2-Oxo-3,4-tetramethylen-pyrrolidin-1-yl-gruppe darstellt, eine Verbindung der allgemeinen Formel

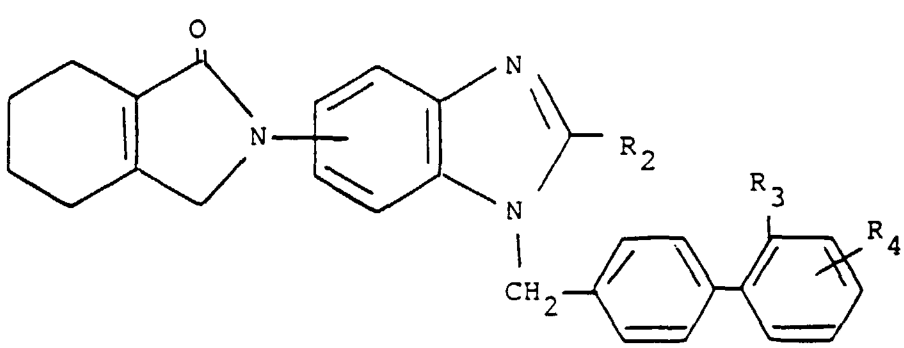

,(IX)

in der
$R_2$, $R_3$ und $R_4$ wie in den Ansprüchen 1 bis 4 definiert sind, hydriert wird oder

h) zur Herstellung von Verbindungen der allgemeinen Formel I, in der $R_1$ eine durch eine Bicyclohexylcarbonyl- oder Biphenylcarbonylgruppe substituierte Aminogruppe, die am N-Atom jeweils zusätzlich durch eine Alkylgruppe mit 1 bis 3 Kohlenstoffatomen substituiert sein können, eine durch eine Bicyclohexyl- oder Biphenylgruppe substituierte Aminocarbonylaminogruppe, die zusätzlich durch eine oder zwei Alkylgruppen mit jeweils 1 bis 3 Kohlenstoffatomen am N-Atom substituiert sein kann, eine gegebenenfalls durch eine Alkylgruppe mit 1 bis 3 Kohlenstoffatomen oder durch eine Phenylgruppe mono- oder disubstituierte Maleinsäureamido- oder Maleinsäureimidogruppe, wobei die Substituenten gleich oder verschieden sein können, eine durch eine Alkylsulfonylgruppe mit 4 bis 6 Kohlenstoffatomen oder durch eine Phenylalkylsulfonylgruppe substituierte Alkylamino- oder Phenylalkylaminogruppe, in denen der Alkylteil jeweils 1 bis 3 Kohlenstoffatome enthalten kann, eine durch eine Naphthalinsulfonylgruppe substituierte Amino- oder Alkylaminogruppe, welche im Naphthalinring durch eine Dialkylaminogruppe, durch eine oder zwei Alkoxygruppen substituiert sein können, wobei der Alkylteil jeweils 1 bis 3 Kohlenstoffatome enthalten kann, eine 7-Nitro-benzofurazan-4-yl-aminoalkanoylaminogruppe, in der der Alkanoylteil 2 oder 3 Kohlenstoffatome enthalten kann, eine Benzofurancarbonylamino- oder 7-Nitro-benzofurazan-4-yl-amino-gruppe

oder auch, wenn $R_3$ eine Carboxygruppe und $R_2$ eine n-Butylgruppe darstellen, $R_1$ in 6-Stellung eine durch eine Phenylsulfonyl-, Cyclohexylmethylaminocarbonyl-, 2-Carboxycyclohexylmethylcarbonyl-, 2-tert.Butoxycarbonyl-cyclohexylmethylcarbonyl-, 2-Carboxy-3,4,5,6-tetrahydrobenzoyl-, N-N-Methyl-phenylaminocarbonyl- oder 3-Cyclohexylpropylgruppe substituierte Aminogruppe, eine durch eine Propylsulfonyl-, Phenylsulfonyl-, Methylphenylsulfonyl- oder Chlorphenylsulfonylgruppe substituierte Methylaminogruppe, eine durch eine Phenylsulfonyl- oder Methoxyphenylsulfonylgruppe substituierte n-Pentylaminogruppe, eine durch eine Methylphenylsulfonyl- oder Methoxyphenylsulfonylgruppe substituierte n-Propylaminogruppe, eine durch eine Benzoyl- oder Chlorphenylsulfonylgruppe substituierte Isopropylaminogruppe, eine N-Acetyl-cyclohexylmethylamino-, 3,4,5,6-Tetrahydrophthalimido-, Hexahydrohomophthalimido-, N-Methansulfonyl-2-phenylethylamino- oder N-Chlorphenylsulfonyl-benzylamino-gruppe

oder, wenn $R_3$ eine Carboxygruppe und $R_2$ eine n-Butylgruppe darstellen, $R_1$ in 5- oder 6-Stellung eine 2-Oxo-1,2-dihydro-3,4-tetramethylen-pyrrolidin-1-yl-gruppe

oder, wenn $R_3$ eine Carboxygruppe und $R_2$ eine Methyl-, Ethyl-, n-Propyl-, n-Butyl- oder Methylmercaptogruppe darstellen, $R_1$ in 6-Stellung eine Pyrrolidinocarbonylaminogruppe

oder auch, wenn $R_3$ eine Tetrazolylgruppe und $R_2$ eine n-Butylgruppe darstellen, $R_1$ in 5- oder 6-Stellung eine durch eine Methylaminocarbonyl- oder Cyclohexylaminocarbonylgruppe substituierte n-Pentylaminogruppe oder in 6-Stellung eine 3,3-Dimethyl-glutarsäureimido- oder 4,4-Tetramethylen-glutarsäureimidogruppe,

oder auch, wenn $R_3$ eine Tetrazolylgruppe und $R_2$ eine Ethyl- oder n-Propylgruppe darstellen, $R_1$ in 6-Stellung eine N-Benzolsulfonyl-methylaminogruppe

oder auch, wenn $R_3$ eine tert.Butoxycarbonylgruppe und $R_2$ eine N-Butylgruppe darstellen, $R_1$ in 6-Stellung eine 2-Carboxy-cyclohexylmethylcarbonylamino- oder Pyrrolidinocarbonylaminogruppe bedeuten, eine Verbindung der allgemeinen Formel

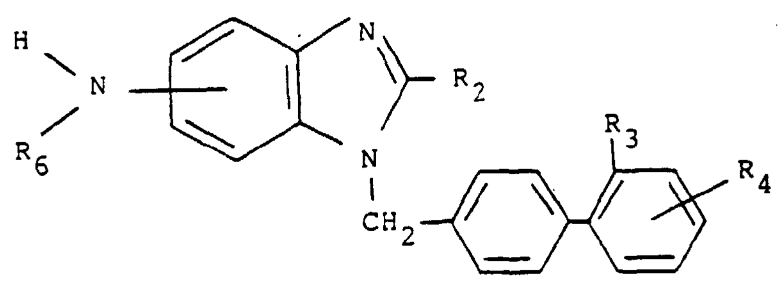

, (X)

in der

$R_2$, $R_3$ und $R_4$ wie in den Ansprüchen 1 bis 4 definiert sind und

$R_6$ ein Wasserstoffatom, eine n-Pentyl-, Cyclohexylmethyl-, Alkyl- oder Phenylalklygruppe mit jeweils 1 bis 3 Kohlenstoffatomen im Alkylteil bedeutet, mit einer Verbindung der allgemeinen Formel

$$Z_4 - W - R_7, \qquad (XI)$$

in der
$Z_4$ eine nukleophile Austrittsgruppe,
W eine -CO- oder -$SO_2$-Gruppe und
$R_7$ eine 2-Hydroxycarbonyl-ethenylgruppe, in der der Ethenylteil durch eine Alkylgruppe mit 1 bis 3 Kohlenstoffatomen oder durch eine Phenylgruppe mono- oder disubstituiert ist und die Substituenten gleich oder verschieden sein können, eine Alkylgruppe mit 3 bis 6 Kohlenstoffatomen, eine Phenylalkylgruppe mit 1 bis 3 Kohlenstoffatomen im Alkylteil, eine gegebenenfalls durch eine Dialkylaminogruppe, durch eine oder zwei Alkoxygruppen substituierte Naphthalingruppe, in denen der Alkylteil jeweils 1 bis 3 Kohlenstoffatome enthalten kann, eine Methyl-, Phenyl-, Methylphenyl-, Methoxyphenyl-, Chlorphenyl-, Biphenyl-, Bicyclohexyl-, 2-Carboxy-cyclohexylmethyl-, 2-Carboxy-3,4,5,6-tetrahydrophenyl-, 3-Carboxyl,l-dimethyl-propyl-, 3-Carboxy-2,2-tetramethylenpropyl-, 7-Nitro-benzofurazan-4-yl-aminomethyl- oder 7-Nitro-benzofurazan-4-yl-aminoethylgruppe,

oder auch, wenn Weine -CO-Gruppe darstellt, eine $R_8NR_9$-Gruppe, in der

$R_8$ ein Wasserstoffatom oder eine Alkylgruppe mit 1 bis 3 Kohlenstoffatomen,

$R_9$ eine Methyl-, Cyclohexyl-, Cyclohexylmethyl-, Phenyl-, Biphenyl- oder Bicyclohexylgruppe oder

$R_8$ und $R_9$ zusammen mit dem dazwischenliegenden Stickstoffatom eine Pyrrolidinogruppe

oder auch $Z_4$ zusammen mit $R_9$ eine weitere Kohlenstoff-Stickstoff-Bindung darstellen,

oder auch $R_7$ zusammen mit W eine 7-Nitro-benzofurazan-4-yl-aminogruppe bedeuten, umgesetzt wird oder
i) zur Herstellung von Verbindungen der allgemeinen Formel I, in der $R_1$ eine Tetrahydrobenzimidazolyl- oder Imidazopyridinylgruppe oder eine gegebenenfalls im Phenylkern durch ein Fluor-, Chlor- oder Bromatom, durch eine Alkylgruppe mit 1 bis 3 Kohlenstoffatomen, durch eine Alkoxygruppe mit 1 bis 3 Kohlenstoffatomen oder durch eine Trifluormethylgruppe substituierte Benzimidazolylgruppe, wobei die NH-Gruppe der vorstehend erwähnten Imidazolringe zusätzlich durch eine Alkylgruppe mit 1 bis 6 Kohlenstoffatomen oder durch eine Cycloalkylgruppe mit 3 bis 7 Kohlenstoffatomen substituiert sein kann, eine Hydroxycycloalkylaminocarbonylgruppe mit 5 bis 7 Kohlenstoffatomen im Cycloalkylteil, die am N-Atom zusätzlich durch eine Alkylgruppe mit 1 bis 3 Kohlenstoffatomen substituiert sein kann, oder eine geradkettige oder verzweigte Hydroxyalkylaminocarbonylgruppe mit 4 bis 6 Kohlenstoffatomen im Alkylteil bedeutet, eine Verbindung der allgemeinen Formel

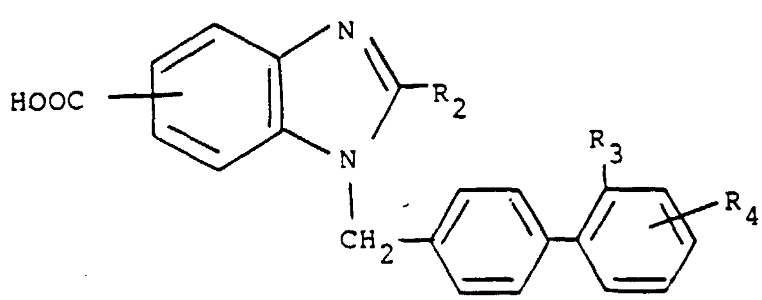

, (XII)

in der

$R_2$ bis $R_4$ wie in den Ansprüchen 1 bis 4 definiert sind, oder deren reaktionsfähige Derivate mit einem Amin der allgemeinen Formel

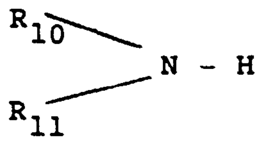

,(XIII)

in der

$R_{10}$ ein Wasserstoffatom, eine Cycloalkylgruppe oder eine Alkylgruppe mit 1 bis 6 Kohlenstoffatomen und

$R_{11}$ eine Hydroxyalkylgruppe mit 4 bis 6 Kohlenstoffatomen, eine Hydroxycycloalkylgruppe mit 5 bis 7 Kohlenstoffatomen oder eine 2-Aminophenylgruppe, die im Phenylkern durch ein Fluor-, Chlor- oder Bromatom, durch eine Alkylgruppe mit 1 bis 3 Kohlenstoffatomen, durch eine Alkoxygruppe mit 1 bis 3 Kohlenstoffatomen oder durch eine Trifluormethylgruppe substituiert sein kann, eine 2-Aminocyclohexyl- oder 2-Aminopyridylgruppe bedeuten, unter gegebenenfalls gleichzeitiger Decarboxylierung umgesetzt wird oder k) zur Herstellung von Verbindungen der allgemeinen Formel I, in der $R_1$ eine Dihydro-pyridazin-3-on- oder Pyridazin-3-on-gruppe darstellt, die in 2-Stellung durch eine gegebenenfalls Phenylgruppe substituierte Alkylgruppe mit 1 bis 3 Kohlenstoffatomen oder im Kohlenstoffgerüst durch eine oder zwei Alkylgruppen mit jeweils 1 bis 3 Kohlenstoffatomen substituiert sein kann, eine Carbonsäure der allgemeinen Formel

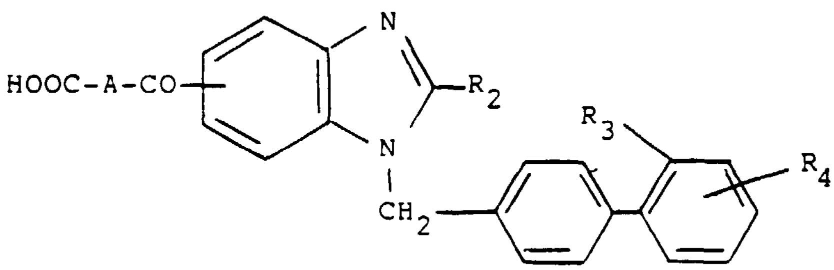

,(XIV)

in der

$R_1$ bis $R_4$ wie in den Ansprüchen 1 bis 4 definiert sind und

A eine gegebenenfalls durch eine oder zwei Alkylgruppen mit jeweils 1 bis 3 Kohlenstoffatomen substituierte Ethylen- oder Ethenylengruppe darstellt, oder deren reaktionsfähige Säurederivate mit einem Hydrazin der allgemeinen Formel

$$H_2N - NHR_{12}, \qquad (XV)$$

in der

$R_{12}$ ein Wasserstoffatom oder eine gegebenenfalls durch eine Phenylgruppe substituierte Alkylgruppe mit 1 bis 3 Kohlenstoffatomen darstellt, umgesetzt wird und

erforderlichenfalls ein während der Umsetzungen a) bis k) zum Schutze von reaktiven Gruppen verwendeter Schutzrest abgespalten wird und/oder

gewünschtenfalls anschließend ein so erhaltenes 1-, 3-Isomerengemisch einer Verbindung der allgemeinen Formel I mittels Isomerentrennung in ihr 1- und 3-Isomer aufgetrennt wird oder eine so erhaltene Verbindung der allgemeinen Formel I in ihr Salz, insbesondere für die pharmazeutische Anwendung in ihr physiologisch verträgliches Salz mit einer anorganischen oder organischen Säure oder Base, übergeführt wird.

## Claims

**1.** Benzimidazoles of general formula

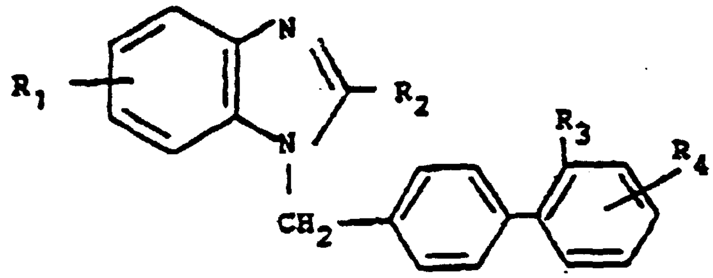

(I)

wherein

$R_1$ represents a tetrahydrobenzimidazolyl or imidazopyridinyl group,

a benzimidazolyl or benzoxazolyl group optionally substituted in the phenyl nucleus by a fluorine, chlorine or bromine atom, by a $C_{1-3}$-alkyl group, by a $C_{1-3}$-alkoxy or by a trifluoromethyl group, whilst the NH group of the above-mentioned imidazole rings may additionally be substituted by a $C_{1-6}$-alkyl group or by a $C_{3-7}$-cycloalkyl group,

an amino group substituted by a bicyclohexylcarbonyl or biphenylcarbonyl group which may additionally be substituted at the N-atom by a $C_{1-3}$-alkyl group,

an aminocarbonylamino group substituted by a bicyclohexyl or biphenyl group and optionally also substituted by one or two $C_{1-3}$alkyl groups at the N-atom,

with the exception of the 2-oxo-3,4-tetramethylenepyrrolidin-1-yl group a 5-, 6- or 7-membered alkyleneimino or alkenyleneimino group optionally substituted by one or two $C_{1-3}$-alkyl groups or by a tetramethylene or pentamethylene group, in which a methylene group is replaced by a carbonyl or sulphonyl group,

a 3,4,5,6-tetrahydro-2(1H)-pyrimidinone group optionally substituted by an alkyl or phenylalkyl group each having 1 to 3 carbon atoms in the alkyl moiety,

a maleic acid amido or maleic acid imido group optionally mono- or disubstituted by a $C_{1-3}$-alkyl group or by a phenyl group, in which the substituents may be identical or different,

an imidazoline or imidazole group optionally substituted by a $C_{1-6}$-alkyl group or by a $C_{3-7}$-cycloalkyl group,

an imidazolidinedione group optionally substituted by a $C_{1-3}$-alkyl group, by a phenylalkyl group having 1 to 3 carbon atoms in the alkyl moiety, by a tetramethylene, pentamethylene or hexamethylene group,

an alkylamino or phenylalkylamino group substituted by a $C_{4-6}$-alkylsulphonyl group or by a phenylalkyl sulphonyl group, wherein the alkyl moiety in each case may contain from 1 to 3 carbon atoms,

an amino or alkylamino group substituted by a naphthalenesulphonyl group, which may be substituted in the naphthalene ring by a dialkylamino group or by one or two alkoxy groups, in which the alkyl moiety in each case may contain from 1 to 3 carbon atoms,

a pyridazin-3-one or dihydro-pyridazin-3-one group which may be substituted in the 2-position by an optionally phenyl-substituted $C_{1-3}$alkyl group and in the carbon structure by 1 or 2 alkyl groups having 1 to 3 carbon atoms,

a pyrrolidino, piperidino or hexamethyleneimino group substituted by two $C_{1-3}$-alkyl groups,

a 7-nitrobenzofurazan-4-yl-aminoalkanoylamino group in which the alkanoyl moiety may contain 2 or 3 carbon atoms,

a heptamethyleneimino, 1H,3H-quinazolin-2,4-dion-3-yl, pentamethylene-oxazolin-2-yl, benzofuran-carbonylamino or 7-nitro-benzofurazan-4-yl-amino group

or, if $R_3$ represents a carboxy group and $R_2$ represents an n-butyl group, $R_1$ in the 6-position may represent an amino group substituted by a phenylsulphonyl, cyclohexylmethylaminocarbonyl, 2-carboxycyclohexylmethylcarbonyl, 2-tert.butoxycarbonyl-cyclohexylmethylcarbonyl, 2-carboxy-3,4,5,6-tetrahydrobenzoyl, N-methyl-phenylaminocarbonyl or 3-cyclohexylpropyl group, a methylamino group substituted by a propylsulphonyl, phenylsulphonyl, methylphenylsulphonyl or chlorophenylsulphonyl group, an n-pentylamino group substituted by a phenylsulphonyl or methoxyphenylsulphonyl group, an n-propylamino group substituted by a methylphenylsulphonyl or methoxyphenylsulphonyl group, an isopropylamino group substituted by a benzoyl or chlorophenylsulphonyl group, an N-acetyl-cyclohexylmethylamino, 3,4,5,6-tetrahydrophthalimido, hexahydrohomophthalimido, N-methanesulphonyl-2-phenylethylamino, N-chlorophenylsulphonyl-benzylamino, piperidino, 4-methyl-piperidino or hexamethyleneimino group

or, if $R_3$ represents a carboxy group and $R_2$ represents an n-butyl group, $R_1$ in the 5- or 6-position represents a 2-oxo-1,2-dihydro-3,4-tetramethylene-pyrrolidin-1-yl group

or, if $R_3$ represents a carboxy group and $R_2$ represents a methyl, ethyl, n-propyl, n-butyl or methylmercapto group, $R_1$ in the 6-position represents a pyrrolidinocarbonylamino group

or, if $R_3$ represents a tetrazolyl group and $R_2$ represents an n-butyl group, $R_1$ in the 5- or 6-position may represent an n-pentylamino group substituted by a methyl-aminocarbonyl or cyclohexylaminocarbonyl group or in the 6-position may represent a 3,3-dimethyl-glutaric acid imido or 4,4-tetramethylene-glutaric acid imido group,

or, if $R_3$ represents a tetrazolyl group and $R_2$ represents an ethyl or n-propyl group, $R_1$ in the 6-position may represent an N-benzenesulphonyl-methylamino group

or, if $R_3$ represents a tert.butoxycarbonyl group and $R_2$ represents an n-butyl group, $R_1$ in the 6-position may represent a 2-carboxycyclohexylmethylcarbonylamino or pyrrolidinocarbonylamino group,

$R_2$ represents a hydrogen atom or a straight-chained or branched $C_{1-5}$-alkyl group in which a methylene group may be replaced by a sulphur atom,

$R_3$ represents a carboxy, cyano, 1H-tetrazolyl or 1-triphenylmethyl-tetrazolyl group or an alkoxycarbonyl group with a total of 2 to 5 carbon atoms and

$R_4$ represents a hydrogen, fluorine, chlorine or bromine atom,

the 1-, 3-isomer mixtures thereof and the addition salts thereof with organic or inorganic acids or bases and the compounds

4'[[2-n-butyl-6-(piperidin-1-yl)-benzimidazol-1-yl]methyl]biphenyl-2-carboxylic acid and

4'[[2-n-butyl-6-(4-methylpiperidin-1-yl)-benzimidazol-1-yl]-methyl]biphenyl-2-carboxylic acid.

**2.** Benzimidazoles of general formula I according to claim 1, wherein

$R_1$ represents a tetrahydrobenzimidazolyl or imidazopyridinyl group,

a benzimidazolyl group optionally substituted in the phenyl nucleus by a fluorine, chlorine or bromine atom or by a methyl, methoxy or trifluoromethyl group, whilst the NH-group of the above-mentioned imidazole rings may additionally be substituted by a $C_{1-6}$-alkyl group or by a $C_{3-6}$-cycloalkyl group,

a benzoxazol-2-yl group optionally substituted by a methyl group,

an amino group substituted by a bicyclohexylcarbonyl, biphenylcarbonyl or benzofuryl-2-carbonyl group,

an aminocarbonylamino group substituted in the 3-position by a bicyclohexyl or biphenyl group,

a 5-, 6- or 7-membered alkyleneimino or alkenyleneimino group optionally substituted by one or two methyl groups or by a tetramethylene or pentamethylene group, wherein a methylene group is replaced by a carbonyl or sulphonyl group,

a 3,4,5,6-tetrahydro-2(1H)-pyrimidinone group optionally substituted by a methyl or benzyl group,

a maleic acid amido or maleic acid imido group optionally mono- or disubstituted by a methyl group or by a phenyl group, wherein the substituents may be identical or different,

an imidazolin-2-yl or imidazol-2-yl group substituted in the 1-position by a $C_{1-6}$-alkyl group or by a $C_{3-7}$-cycloalkyl group,

an imidazolidinedione group optionally substituted by a methyl, benzyl, tetramethylene or pentamethylene group,

a methylamino or benzylamino group substituted by a butanesulphonyl group or by a phenylmethanesulphonyl group,

an amino or methylamino group substituted by a naphthalenesulphonyl group, which may be substituted in the naphthalene ring by a dimethylamino group or by 2 methoxy groups,

a pyridazin-3-one or dihydro-pyridazin-3-one group optionally substituted by a methyl or benzyl group,

a pyrrolidino, piperidino or hexamethyleneimino group substituted by two methyl groups,

a heptamethyleneimino, 1H,3H-quinazolin-2,4-dion-3-yl, 4,5-pentamethylene-oxazolin-2-yl, 7-nitro-benzofurazan-4-yl-amino or 7-nitro-benzofurazan-4-yl-aminopropionylamino group

or, if $R_3$ represents a carboxy group and $R_2$ represents an n-butyl group, $R_1$ in the 6-position may represent an amino group substituted by a phenylsulphonyl, cyclohexylmethylaminocarbonyl, 2-carboxycyclohexylmethylcarbonyl, 2-tert.-butoxycarbonyl-cyclohexylmethylcarbonyl, 2-carboxy-3,4,5,6-tetrahydrobenzoyl, N-methylphenylaminocarbonyl or 3-cyclohexylpropyl group, a methylamino group substituted by a propylsulphonyl, phenylsulphonyl, 4-methylphenylsulphonyl or 4-chlorophenylsulphonyl group, an n-pentylamino group substituted by a phenylsulphonyl or 4-methoxyphenylsulphonyl group, an n-propylamino group substituted by a 4-methylphenylsulphonyl or 4-methoxyphenylsulphonyl group, an isopropylamino group substituted by a benzoyl or 4-chlorophenylsulphonyl group, an N-acetylcyclohexylmethylamino, 3,4,5,6-tetrahydrophthalimido, hexahydrohomophthalimido, N-methanesulphonyl-2-phenylethylamino, N -(4-chlorophenylsulphonyl)benzylamino, piperidino, 4-methyl-piperidino or hexamethyleneimino group

or, if $R_3$ represents a carboxy group and $R_2$ represents an n-butyl group, $R_1$ in the 5- or 6-position represents a 2-oxo-1,2-dihydro-3,4-tetramethylene-pyrrolidin-1-yl group

or, if $R_3$ represents a carboxy group and $R_2$ represents a methyl, ethyl, n-propyl, n-butyl or methylmercapto group, $R_1$ in the 6-position represents a pyrrolidinocarbonylamino group

or, if $R_3$ represents a tetrazolyl group and $R_2$ represents an n-butyl group, $R_1$ in the 5- or 6-position may represent an n-pentylamino group substituted by a methylaminocarbonyl or cyclohexylaminocarbonyl group or, in the 6-position, a 3,3-dimethyl-glutaric acid imido or 4,4-tetramethylene-glutaric acid imido group,

or, if $R_3$ represents a tetrazolyl group and $R_2$ represents an ethyl or n-propyl group, $R_1$ in the 6-position may represent an N-benzenesulphonyl-methylamino group

or, if $R_3$ represents a tert.butoxycarbonyl group and $R_2$ represents an n-butyl group, $R_1$ in the 6-position may represent a 2-carboxy-cyclohexylmethylcarbonylamino or pyrrolidinocarbonylamino group,

$R_2$ represents a hydrogen atom or a straight-chained or branched $C_{1-4}$-alkyl group in which a methylene group may be replaced by a sulphur atom,

$R_3$ represents a carboxy, cyano, 1H-tetrazolyl or 1-triphenylmethyl-tetrazolyl group or an alkoxycarbonyl group with a total of 2 to 5 carbon atoms and

$R_4$ represents a hydrogen, fluorine, chlorine or bromine atom,

the 1-, 3-isomer mixtures thereof and the addition salts thereof with organic or inorganic acids or bases.

**3.** Benzimidazoles of general formula I according to claim 1, wherein

$R_1$ in the 6-position represents a 1-methylbenzimidazol-2-yl, 3,4,5,6-tetrahydro-phthalimino, 2,3-diphenyl-maleic acid imido, 2,3-dimethyl-maleic acid imido, N-phenylmethanesulphonyl-methylamino, 2-oxo-pyrrolidin-1-yl, 2-oxo-piperidin-1-yl, 2-oxo-hexamethyleneimino, 2-oxo-3,4-tetramethylene-pyrrolidin-2-yl, 3,3-dimethylglutarimido, N-methylaminocarbonyl-n-pentylamino, propanesultam-1-yl or butanesultam-1-yl group,

$R_2$ represents a methyl, ethyl, n-propyl or n-butyl group,

$R_3$ represents a carboxy or 1H-tetrazolyl group and

$R_4$ represents a hydrogen atom,

and the addition salts thereof with organic or inorganic acids or bases.

**4.** Benzimidazoles of general formula I according to claim 1:

4'-[[2-n-propyl-6-(1-methylbenzimidazol-2-yl)benzimidazol-1-yl]methyl]biphenyl-2-carboxylic acid,

4'-[[2-n-butyl-6-(3,4,5,6-tetrahydro-phthalimino)benzimidazol-1-yl]methyl]biphenyl-2-carboxylic acid,

4'-[[2-n-butyl-6-(2,3-diphenyl-maleic acid imido)benzimidazol-1-yl]methyl]biphenyl-2-carboxylic acid,

4'-[[2-n-butyl-6-(2,3-dimethyl-maleic acid imido)benzimidazol-1-yl]methyl]biphenyl-2-carboxylic acid,

4'-[[2-n-butyl-6-(N-phenylmethanesulphonyl-methylamino)benzimidazol-1-yl]methyl]biphenyl-2-carboxylic acid,

4'-[[2-n-butyl-6-(2-oxo-piperidin-1-yl)-benzimidazol-1-yl]methyl]-2-(1H-tetrazol-5-yl)-biphenyl,

4'-[[2-n-butyl-6-(2-oxo-pyrrolidin-1-yl)-benzimidazol-1-yl]methyl]-2-(1H-tetrazol-5-yl)-biphenyl,

4'-[[2-n-butyl-(2-oxo-hexamethyleneimino)-benzimidazol-1-yl]methyl]-2-(1H-tetrazol-5-yl)-biphenyl,

4-[[2-n-butyl-6-(3,3-dimethylglutarimido)-benzimidazol-1-yl]methyl]-2-(1H-tetrazol-5-yl)-biphenyl,

4-[[2-n-butyl-(N-methylaminocarbonyl-n-pentylamino)-benzimidazol-1-yl]methyl]-2-(1H-tetrazol-5-yl)-biphenyl,

4'-[[2-n-butyl-6-(cyclohexylaminocarbonyl-n-pentylamino)-benzimidazol-1-yl]methyl]-2-(1H-tetrazol-5-yl)-biphenyl,

4'-[[2-n-butyl-6-(2-oxo-3,4-tetramethylene-pyrrolidin-1-yl)-benzimidazol-1-yl]methyl]biphenyl-2-carboxylic acid,

4-[[2-n-butyl-6-(propanesultam-1-yl)-benzimidazol-1-yl]methyl]-2-(1H-tetrazol-5-yl)-biphenyl,

4'-[[2-n-propyl-6-(butanesultam-1-yl)-benzimidazol-1-yl]methyl]-2-(1H-tetrazol-5-yl)-biphenyl,

4'-[[2-n-butyl-6-(butanesultam-1-yl)-benzimidazol-1-yl]methyl]-2-(1H-tetrazol-5-yl)-biphenyl,

4'-[[2-n-propyl-6-(1-methyl-benzimidazol-2-yl)-benzimidazol-1-yl]methyl]-2-(1H-tetrazol-5-yl)-biphenyl and

4'-[[2-n-propyl-6-(2-oxo-piperidin-1-yl)-benzimidazol-1-yl]methyl]-2-(1H-tetrazol-5-yl)-biphenyl

and the addition salts thereof with organic or inorganic acids or bases.

**5.** Physiologically acceptable addition salts of the compounds according to at least one of claims 1 to 4 with organic or inorganic acids or bases.

**6.** Pharmaceutical compositions containing a compound of general formula I wherein $R_1$, $R_2$ and $R_4$ and $R_3$, with the exception of the tert.butoxycarbonyl, cyano and 1-triphenylmethyl-tetrazolyl group, are defined as in at least one of claims 1 to 4, or a physiologically acceptable addition salt thereof according to claim 5, and optionally another active substance, optionally together with one or more inert carriers and/or diluents.

**7.** Use of a compound of general formula I, wherein $R_1$, $R_2$ and $R_4$ and $R_3$, with the exception of the tert.butoxycarbonyl, cyano and 1-triphenylmethyl-tetrazolyl group, are defined as in at least one of claims 1 to 4 or a physiologically acceptable addition salt thereof according to claim 5, for preparing a pharmaceutical composition having an angiotensin-antagonistic activity.

**8.** Process for preparing a pharmaceutical composition according to claim 6, characterised in that a compound of general formula I wherein $R_1$, $R_2$ and $R_4$, and $R_3$ with the exception of the tert.butoxycarbonyl, cyano and 1-triphenylmethyl-tetrazolyl group are defined as in at least one of claims 1 to 4 or a physiologically acceptable salt thereof is incorporated in one or more inert carriers and/or diluents by a non-chemical method.

**9.** Process for preparing the benzimidazoles according to claims 1 to 5, characterised in that

a) a compound of general formula

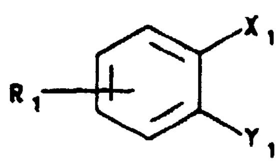

(II)

wherein
$R_1$ is defined as in claims 1 to 4,

one of the groups $X_1$ or $Y_1$ represents a group of general formula

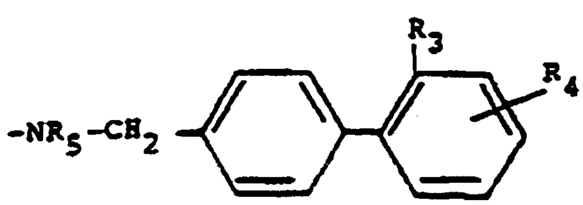

and the other group $X_1$ or $Y_1$ represents a group of the general formula

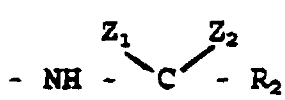

wherein
$R_2$ to $R_4$ are defined as in claims 1 to 4,
$R_5$ represents a hydrogen atom or an $R_2CO$ group, wherein
$R_2$ is defined as hereinbefore,
$Z_1$ and $Z_2$, which may be identical or different, represent optionally substituted amino groups or hydroxy or mercapto groups optionally substituted by lower alkyl groups or
$Z_1$ and $Z_2$ together represent an oxygen or sulphur atom, an imino group optionally substituted by a $C_{1-3}$-alkyl group, or a $C_{2-3}$-alkylenedioxy or $C_{2-3}$alkylenedithio group, but one of the groups $X_1$ or $Y_1$ must represent a group of general formula

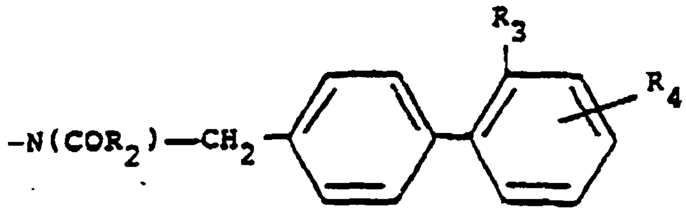

or

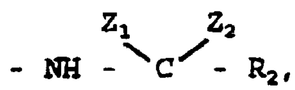

is cyclised and subsequently an N-oxide optionally obtained in this way is reduced or

b) a benzimidazole of general formula

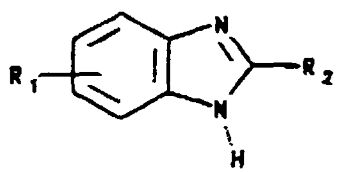

(III)

wherein

$R_1$ and $R_2$ are defined as in claims 1 to 4, is reacted with a biphenyl compound of general formula

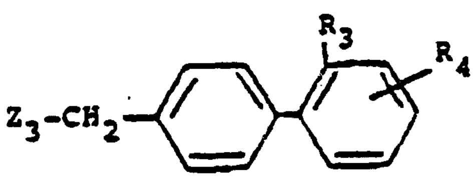

, (IV)

wherein
$R_3$ and $R_4$ are defined as in claims 1 to 4 and
$Z_3$ represents a nucleophilic leaving group such as a halogen atom or a substituted sulphonyloxy group, or

c) in order to prepare a compound of general formula I wherein $R_3$ represents a carboxy group,
a compound of general formula

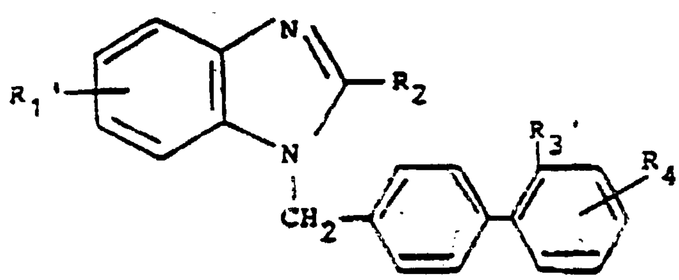

, (V)

wherein

$R_2$ and $R_4$ are defined as in claims 1 to 4,
$R_1$' has the meanings given for $R_1$ in claims 1 to 4 and may represent a 3-alkoxycarbonylpropionyl or 3-alkoxycarbonyl-2-methylpropionyl group in which the alkoxy part may contain from 1 to 3 carbon atoms, and
$R_3$' represents a group which may be converted into a carboxy group by hydrolysis, thermolysis or hydrogenolysis, is converted into a corresponding carboxy compound, or

d) in order to prepare a compound of general formula I wherein $R_3$ represents a 1H-tetrazolyl group, a protecting group is cleaved from a compound of general formula

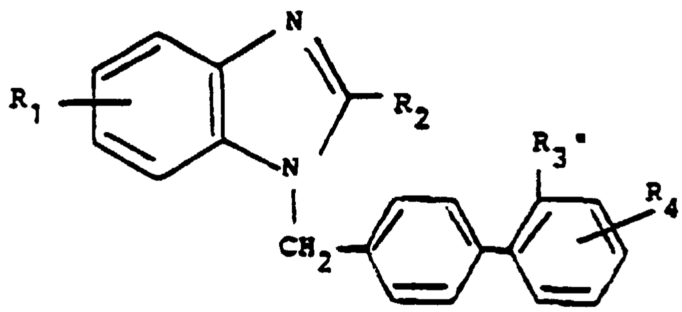

, (VI)

wherein

$R_1$, $R_2$ and $R_4$ are defined as in claims 1 to 4 and
$R_3$" represents a 1H-tetrazolyl group protected in the 1-or 3-position by a protecting group, or

e) in order to prepare a compound of general formula I wherein $R_3$ represents a 1H-tetrazolyl group, a compound of general formula

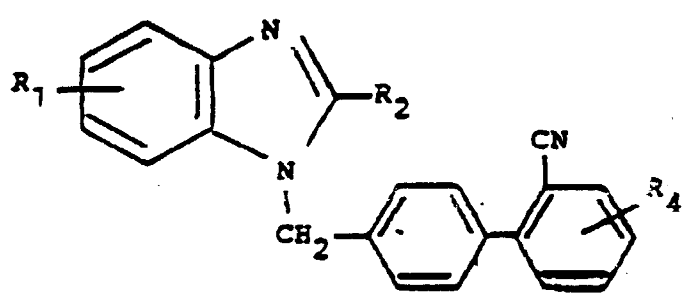

, (VII)

wherein
$R_1$, $R_2$ and $R_4$ are defined as in claims 1 to 4, is reacted with hydrazoic acid or the salts thereof, or
f) in order to prepare compounds of general formula I wherein $R_1$ represents a pentamethylene-oxazolin-2-yl

group, a compound of general formula

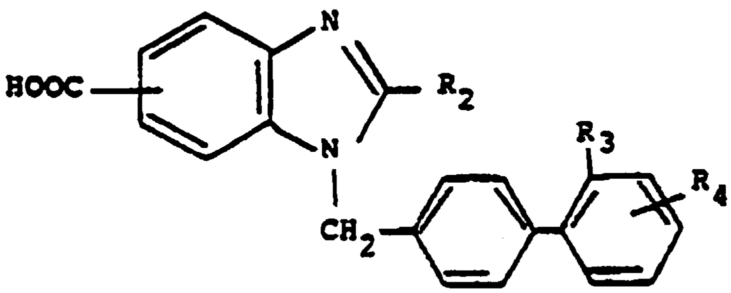

, (VIII)

wherein
$R_2$ to $R_4$ are defined as in claims 1 to 4, is reacted with 1-aminomethyl-cyclohexanol in the presence of an acid-activating agent, or
g) in order to prepare a compound of general formula I wherein $R_1$ represents a 2-oxo-3,4-tetramethylenepyrrolidin-1-yl group, a compound of general formula

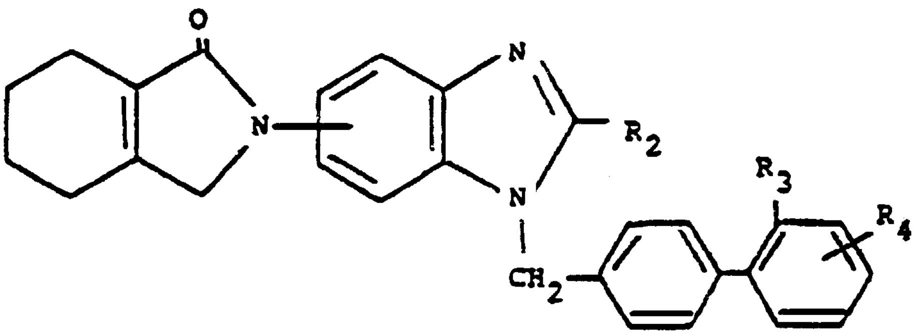

, (IX)

wherein
$R_2$, $R_3$ and $R_4$ are defined as in claims 1 to 4 is hydrogenated, or
h) in order to prepare compounds of general formula I wherein $R_1$ represents an amino group substituted by a bicyclohexylcarbonyl or biphenylcarbonyl group, which may additionally be substituted at the N-atom by a $C_{1-3}$-alkyl group, an aminocarbonylamino group substituted by a bicyclohexyl or biphenyl group and optionally additionally substituted by one or two $C_{1-3}$-alkyl groups at the N-atom, a maleic acid amido or maleic acid imido group optionally mono- or disubstituted by a $C_{1-3}$-alkyl group or by a phenyl group, in which the substituents may be identical or different, an alkylamino or phenylalkylamino group substituted by a $C_{4-6}$-alkylsulphonyl group or by a phenylalkylsulphonyl group, wherein the alkyl moiety may contain 1 to 3 carbon atoms, an amino or alkylamino group substituted by a naphthalenesulphonyl group and optionally substituted in the naphthalene ring by a dialkylamino group or by one or two alkoxy groups, in which the alkyl moiety may contain 1 to 3 carbon atoms, a 7-nitro-benzofurazan-4-yl-aminoalkanoylamino group wherein the alkanoyl moiety may contain 2 or 3 carbon atoms, a benzofurancarbonylamino or 7-nitro-benzofurazan-4-yl-amino group

or, if $R_3$ represents a carboxy group and $R_2$ represents an n-butyl group, $R_1$ in the 6-position may represent an amino group substituted by a phenylsulphonyl, cyclohexylmethylaminocarbonyl, 2-carboxycyclohexylmethylcarbonyl, 2-tert.-butoxycarbonylcyclohexylmethylcarbonyl, 2-carboxy-3,4,5,6-tetrahydrobenzoyl, N-methyl-phenylaminocarbonyl or 3-cyclohexylpropyl group, a methylamino group substituted by a propylsulphonyl, phenylsulphonyl, methylphenylsulphonyl or chlorophenylsulphonyl group, an n-pentylamino group substituted by a phenylsulphonyl or methoxyphenylsulphonyl group, an n-propylamino group substituted by a methylphenylsulphonyl or methoxyphenylsulphonyl group, an isopropylamino group substituted by a benzoyl or chlorophenylsulphonyl group, an N-acetylcyclohexylmethylamino, 3,4,5,6-tetrahydrophthalimido, hexahydrohomophthalimido, N-methanesulphonyl-2-phenylethylamino or N-chlorophenylsulphonyl-benzylamino group

or, if $R_3$ represents a carboxy group and $R_2$ represents an n-butyl group, $R_1$ in the 5- or 6-position represents a 2-oxo-1,2-dihydro-3,4-tetramethylene-pyrrolidin-1-yl group or, if $R_3$ represents a carboxy group and $R_2$ represents a methyl, ethyl, n-propyl, n-butyl or methylmercapto group, $R_1$ in the 6-position represents a pyrrolidinocarbonylamino group

or, if $R_3$ represents a tetrazolyl group and $R_2$ represents an n-butyl group, $R_1$ in the 5- or 6-position may represent an n-pentylamino group substituted by a methylamino-carbonyl or cyclohexylaminocarbonyl group or in the 6-position it may represent a 3,3-dimethylglutaric acid imido or 4,4-tetramethylene-glutaric acid imido group,

or, if $R_3$ represents a tetrazolyl group and $R_2$ represents an ethyl or n-propyl group, $R_1$ in the 6-position may represent an N-benzenesulphonyl-methylamino group

or, if $R_3$ represents a tert.-butoxycarbonyl group and $R_2$ represents an n-butyl group, $R_1$ in the 6-position may represent a 2-carboxy-cyclohexylmethylcarbonylamino or pyrrolidinocarbonylamino group,

a compound of general formula

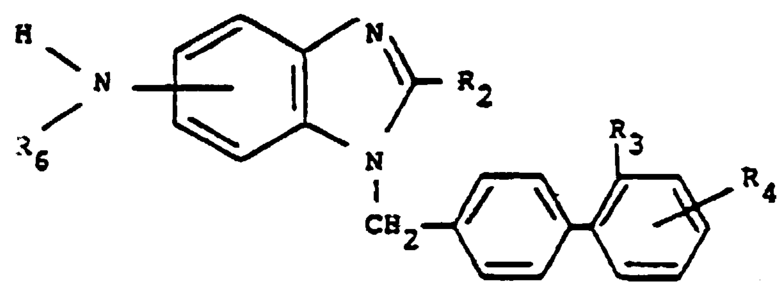

, (X)

wherein

$R_2$, $R_3$ and $R_4$ are defined as in claims 1 to 4 and
$R_6$ represents a hydrogen atom, an n-pentyl, cyclohexylmethyl, alkyl or phenylalkyl group each having 1 to 3 carbon atoms in the alkyl moiety,
is reacted with a compound of general formula

$$Z_4 - W - R_7 \quad (XI)$$

wherein
$Z_4$ represents a nucleophilic leaving group,
W represents a -CO- or -$SO_2$- group and
$R_7$ represents a 2-hydroxycarbonyl-ethenyl group wherein the ethenyl moiety is mono- or disubstituted by a $C_{1-3}$-alkyl group or by a phenyl group and the substituents may be identical or different, a $C_{3-6}$-alkyl group, a phenylalkyl group having 1 to 3 carbon atoms in the alkyl moiety, a naphthalene group optionally substituted by a dialkylamino group or by one or two alkoxy groups, wherein the alkyl moiety may contain from 1 to 3 carbon atoms, a methyl, phenyl, methylphenyl, methoxyphenyl, chlorophenyl, biphenyl, bicyclohexyl, 2-carboxycyclohexylmethyl, 2-carboxy-3,4,5,6-tetrahydrophenyl, 3-carboxy-1,1-dimethyl-propyl, 3-carboxy-2,2-tetramethylene-propyl, 7-nitro-benzofurazan-4-yl-aminomethyl or 7-nitro-benzofurazan-4-yl-aminoethyl group,

or, if W represents a -CO- group, an $R_8NR_9$ group wherein $R_8$ represents a hydrogen atom or a $C_{1-3}$-alkyl group,

$R_9$ represents a methyl, cyclohexyl, cyclohexylmethyl, phenyl, biphenyl or bicyclohexyl group or

$R_8$ and $R_9$ together with the nitrogen atom between them represent a pyrrolidino group

or $Z_4$ together with $R_9$ represents another carbon-nitrogen bond,

or $R_7$ together with W represents a 7-nitro-benzofurazan-4-yl-amino group, or

i) in order to prepare compounds of general formula I wherein $R_1$ represents a tetrahydrobenzimidazolyl or imidazopyridinyl group or a benzimidazolyl group optionally substituted in the phenyl nucleus by a fluorine, chlorine or bromine atom, by a $C_{1-3}$-alkyl group, by a $C_{1-3}$-alkoxy group or by a trifluoromethyl group, whilst the NH-group of the above-mentioned imidazole rings may additionally be substituted by a $C_{1-6}$-alkyl group or by a $C_{3-7}$-cycloalkyl group, a hydroxycycloalkylaminocarbonyl group having 5 to 7 carbon atoms in the cycloalkyl moiety, which may additionally be substituted at the N-atom by a $C_{1-3}$-alkyl group, or a straight-chained or branched hydroxyalkylaminocarbonyl group having 4 to 6 carbon atoms in the alkyl moiety, a compound of general formula

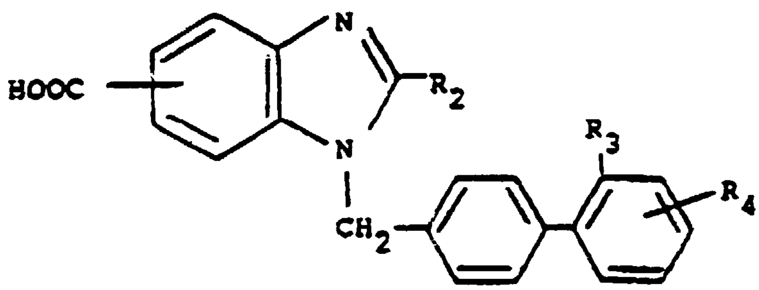

(XII)

wherein

$R_2$ to $R_4$ are defined as in claims 1 to 4, or a reactive derivative thereof, is reacted with an amine of general formula

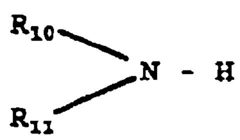

(XIII)

wherein
$R_{10}$ represents a hydrogen atom, a cycloalkyl group or a $C_{1-6}$-alkyl group and
$R_{11}$ represents a $C_{4-6}$-hydroxyalkyl group, a $C_{5-7}$-hydroxycycloalkyl group or a 2-aminophenyl group which may be substituted in the phenyl nucleus by a fluorine, chlorine or bromine atom, by a $C_{1-3}$-alkyl group, by a $C_{1-3}$-alkoxy group or by a trifluoromethyl group, a 2-aminocyclohexyl or 2-aminopyridyl group, optionally with simultaneous decarboxylation, or

k) in order to prepare compounds of general formula I wherein $R_1$ represents a dihydro-pyridazin-3-one or pyridazin-3-one group which may be substituted in the 2-position by an optionally phenyl-substituted $C_{1-3}$-alkyl group or in the carbon skeleton by one or two $C_{1-3}$-alkyl groups, a carboxylic acid of general formula

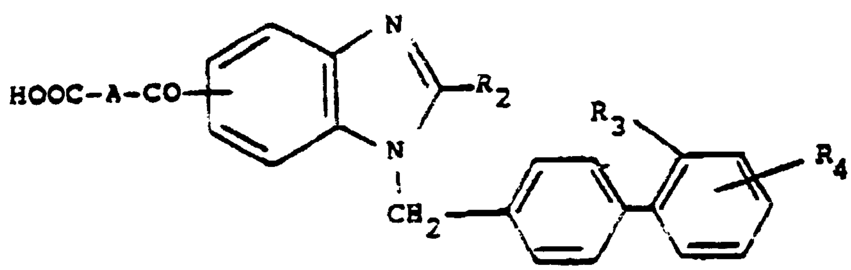

(XIV)

wherein

$R_1$ to $R_4$ are defined as in claims 1 to 4 and
A represents an ethylene or ethenylene group optionally substituted by one or two $C_{1-3}$-alkyl groups, or a reactive acid derivative thereof, is reacted with a hydrazine of general formula

$$H_2N - NHR_{12} \qquad (XV)$$

wherein
$R_{12}$ represents a hydrogen atom or an optionally phenyl-substituted $C_{1-3}$-alkyl group, and

if necessary a protecting group used during reactions a) to k) in order to protect any reactive groups is cleaved and/or

subsequently, if desired, a 1-, 3-isomer mixture of a compound of general formula I thus obtained is resolved by isomer separation into the 1- and 3-isomers thereof or

a compound of general formula I thus obtained is converted into an addition salt thereof, more particularly, for pharmaceutical use, into a physiologically acceptable salt thereof with an organic or inorganic acid or base.

## Revendications

1. Benzimidazoles de formule générale

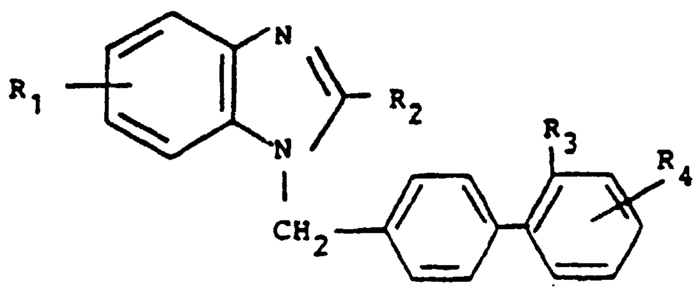

(I)

dans laquelle

$R_1$ représente un groupe tétrahydrobenzimidazolyle ou imidazopyridinyle,
un groupe benzimidazolyle ou benzoxazolyle éventuellement substitué dans le noyau phényle par un atome de fluor, de chlore ou de brome, par un groupe alkyle de 1 à 3 atomes de carbone, par un groupe alcoxy de 1 à 3 atomes de carbone ou par un groupe trifluorométhyle, le groupe NH des cycles imidazole cités précédemment pouvant être substitué en outre par un groupe alkyle de 1 à 6 atomes de carbone ou par un groupe cycloalkyle de 3 à 7 atomes de carbone,
un groupe amino substitué par un groupe bicyclohexylcarbonyle ou biphénylcarbonyle, qui peuvent être substitués en outre chacun sur l'atome N par un groupe alkyle de 1 à 3 atomes de carbone,
un groupe aminocarbonylamino substitué par un groupe bicyclohexyle ou biphényle, qui peut être substitué en outre sur l'atome N par un ou deux groupes alkyle de 1 à 3 atomes de carbone chacun,
à l'exception du groupe 2-oxo-3,4-tétraméthylènepyrrolidin-1-yle un groupe alkylène-imino ou alcénylène-imino à 5, 6 ou 7 chaînons éventuellement substitué par un ou deux groupes alkyle de 1 à 3 atomes de carbone ou par un groupe tétraméthylène ou pentaméthylène, dans lequel un groupe méthylène est remplacé par un groupe carbonyle ou sulfonyle,
un groupe 3,4,5,6-tétrahydro-2(1H)-pyrimidinone éventuellement substitué dans la partie alkyle par un groupe alkyle ou phénylalkyle de 1 à 3 atomes de carbone dans chaque cas,

un groupe maléamido ou maléimido éventuellement mono- ou disubstitué par un groupe alkyle de 1 à 3 atomes de carbone ou par un groupe phényle, les substituants pouvant être identiques ou différents,
un groupe imidazoline ou imidazole éventuellement substitué par un groupe alkyle de 1 à 6 atomes de carbone ou par un groupe cycloalkyle de 3 à 7 atomes de carbone,
un groupe imidazolidinedione éventuellement substitué par un groupe alkyle de 1 à 3 atomes de carbone, par un groupe phénylalkyle de 1 à 3 atomes de carbone dans la partie alkyle, par un groupe tétraméthylène, pentaméthylène ou hexaméthylène,
un groupe alkylamino ou phénylalkylamino substitué par un groupe alkylsulfonyle de 4 à 6 atomes de carbone ou par un groupe phénylalkylsulfonyle, dans lesquels la partie alkyle peut contenir dans chaque cas 1 à 3 atomes de carbone,
un groupe amino ou alkylamino substitué par un groupe naphtalènesulfonyle, qui peuvent être substitués dans le cycle naphtalène par un groupe dialkylamino, par un ou deux groupes alcoxy, la partie alkyle pouvant contenir dans chaque cas 1 à 3 atomes de carbone,
un groupe pyridazin-3-one ou dihydro-pyridazin-3-one, qui peut être substitué en position 2 par un groupe alkyle de 1 à 3 atomes de carbone éventuellement substitué par un groupe phényle et en outre dans le squelette carboné par 1 ou 2 groupes alkyle de 1 à 3 atomes de carbone chacun,
un groupe pyrrolidino, pipéridino ou hexaméthylène-imino substitué par 2 groupes alkyle de 1 à 3 atomes de carbone chacun,
un groupe 7-nitro-benzofurazan-4-yl-aminoalcanoylamino dans lequel la partie alcanoyle peut contenir 2 ou 3 atomes de carbone,
un groupe heptaméthylène-imino-, 1H,3H-quinazolin-2,4-dion-3-yle, pentaméthylèneoxazolin-2-yle, benzofuranecarbonylamino ou 7-nitro-benzofurazan-4-yl-amino ou, lorsque $R_3$ représente un groupe carboxyle et $R_2$ représente un groupe n-butyle, $R_1$ en position 6 représente aussi un groupe amino substitué par un groupe phénylsulfonyle, cyclohexylméthylaminocarbonyle, 2-carboxycyclohexylméthylcarbonyle, 2-tert-butoxycarbonyl-cyclohexylméthylcarbonyle,
2-carboxy-3,4,5,6-tétrahydrobenzoyle, N-méthylphénylaminocarbonyle ou 3-cyclohexylpropyle, un groupe méthylamino substitué par un groupe propylsulfonyle, phénylsulfonyle, méthylphénylsulfonyle ou chlorophénylsulfonyle, un groupe n-pentylamino substitué par un groupe phénylsulfonyle ou méthoxyphénylsulfonyle, un groupe n-propylamino substitué par un groupe méthylphénylsulfonyle ou méthoxyphénylsulfonyle, un groupe isopropylamino substitué par un groupe benzoyle ou chlorophénylsulfonyle, un groupe N-acétylcyclohexylméthylamino, 3,4,5,6-tétrahydrophtalimido, hexahydrohomophtalimido, N-méthanesulfonyl-2-phényléthylamino, N-chlorophénylsulfonyl-benzylamino, pipéridino, 4-méthyl-pipéridino- ou hexaméthylène-imino
ou, lorsque $R_3$ représente un groupe carboxyle et $R_2$ représente un groupe n-butyle, $R_1$ en position 5 ou 6 représente aussi un groupe 2-oxo-1,2-dihydro-3,4-tétraméthylène-pyrrolidin-1-yle
ou, lorsque $R_3$ représente un groupe carboxyle et $R_2$ représente un groupe méthyle, éthyle, n-propyle, n-butyle ou méthylmercapto, $R_1$ en position 6 représente aussi un groupe pyrrolidinocarbonylamino
ou, lorsque $R_3$ représente un groupe tétrazolyle et $R_2$ représente un groupe n-butyle, $R_1$ en position 5 ou 6 représente aussi un groupe n-pentylamino substitué par un groupe méthylaminocarbonyle ou cyclohexylaminocarbonyle ou en position 6 un groupe 3,3-diméthyl-glutarimido ou 4,4-tétraméthylène-glutarimido,
ou, lorsque $R_3$ représente un groupe tétrazolyle et $R_2$ représente un groupe éthyle ou n-propyle, $R_1$ en position 6 représente aussi un groupe N-benzènesulfonyl-méthylamino ou, lorsque $R_3$ représente un groupe tert-butoxycarbonyle et $R_2$ représente un groupe N-butyle, $R_1$ en position 6 représente aussi un groupe 2-carboxycyclohexylméthylcarbonylamino ou pyrrolidinocarbonylamino,
$R_2$ représente un atome d'hydrogène ou un groupe alkyle linéaire ou ramifié de 1 à 5 atomes de carbone, dans lequel un groupe méthylène peut être remplacé par un atome de soufre,
$R_3$ représente un groupe carboxyle, cyano, 1H-tétrazolyle ou 1-triphénméthyl-tétrazolyle ou un groupe alcoxycarbonyle de 2 à 5 atomes de carbone au total et
$R_4$ représente un atome d'hydrogène, de fluor, de chlore ou de brome,
leurs mélanges d'isomères 1, 3 et leurs sels avec des acides ou des bases inorganiques ou organiques ainsi que les composés acide 4'-[[2-n-butyl-6-(pipéridin-1-yl)-benzimidazol-1-yl]méthyl]biphényle-2-carboxylique et acide 4'-[[2-n-butyl-6-(4-méthylpipéridin-1-yl)-benzimidazol-1-yl]méthyl]biphényle-2-carboxylique.

**2.** Benzimidazoles de formule générale I selon la revendication 1, dans laquelle

$R_1$ représente un groupe tétrahydrobenzimidazolyle ou imidazopyridinyle,
un groupe benzimidazolyle éventuellement substitué dans le noyau phényle par un atome de fluor, de chlore ou de brome, par un groupe méthyle, méthoxy ou trifluorométhyle, le groupe NH des cycles imidazole cités précédemment pouvant être substitué en outre par un groupe alkyle de 1 à 6 atomes de carbone ou par un

groupe cycloalkyle de 3 à 6 atomes de carbone,
un groupe benzoxazol-2-yle éventuellement substitué par un groupe méthyle,
un groupe amino substitué par un groupe bicyclohexylcarbonyle, biphénylcarbonyle ou benzofuryl-2-carbonyle,
un groupe aminocarbonylamino substitué en position 3 par un groupe bicyclohexyle ou biphényle,
un groupe alkylène-imino ou alcénylène-imino à 5, 6 ou 7 chaînons éventuellement substitué par un ou deux groupes méthyle ou par un groupe tétraméthylène ou pentaméthylène, dans lequel un groupe méthylène est remplacé par un groupe carbonyle ou sulfonyle,
un groupe 3,4,5,6-tétrahydro-2(1H)-pyrimidinone éventuellement substitué par un groupe méthyle ou benzyle,
un groupe maléamido ou maléimido éventuellement mono- ou disubstitué par un groupe méthyle ou par un groupe phényle, les substituants pouvant être identiques ou différents,
un groupe imidazolin-2-yle ou imidazol-2-yle substitué en position 1 par un groupe alkyle de 1 à 6 atomes de carbone ou par un groupe cycloalkyle de 3 à 7 atomes de carbone,
un groupe imidazolidinedione éventuellement substitué par un groupe méthyle, benzyle, tétraméthylène ou pentaméthylène,
un groupe méthylamino ou benzylamino substitué par un groupe butanesulfonyle ou par un groupe phénylméthanesulfonyle,
un groupe amino ou méthylamino substitué par un groupe naphtalènesulfonyle, qui peuvent être substitués dans le cycle naphtalène par un groupe diméthylamino ou par 2 groupes méthoxy,
un groupe pyridazin-3-one ou dihydro-pyridazin-3-one éventuellement substitué par un groupe méthyle ou benzyle, un groupe pyrrolidino, pipéridino ou hexaméthylène-imino substitué par deux groupes méthyle,
un groupe heptaméthylène-imino, 1H,3H-quinazoline-2,4-dion-3-yle, 4,5-pentaméthylène-oxazolin-2-yle, 7-nitro-benzofurazan-4-yl-amino ou 7-nitro-benzofurazan-4-yl-aminopropionylamino
ou, lorsque $R_3$ représente un groupe carboxyie et $R_2$ représente un groupe n-butyle, $R_1$ en position 6 représente aussi un groupe amino substitué par un groupe phénylsulfonyle, cyclohexylméthylaminocarbonyle, 2-carboxycyclohexylméthylcarbonyle, 2-tert-butoxycarbonyl-cyclohexylméthylcarbonyle, 2-carboxy-3,4,5,6-tétrahydrobenzoyle,
N-méthylphénylaminocarbonyle ou 3-cyclohexylpropyle, un groupe méthylamino substitué par un groupe propylsulfonyle, phénylsulfonyle, 4-méthylphénylsulfonyle ou 4-chlorophénylsulfonyle, un groupe n-pentylamino substitué par un groupe phénylsulfonyle ou 4-méthoxyphénylsulfcnyle, un groupe n-propylamino substitué par un groupe 4-méthylphénylsulfonyle ou 4-méthoxyphénylsulfonyle, un groupe isopropylamino substitué par un groupe benzoyle ou 4-chlorophénylsulfonyle, un groupe N-acétylcyclohexylméthylamino, 3,4,5,6-tétrahydrophtalimido, hexahydrohomophtalimido, N-méthanesulfonyl-2-phényléthylamino, N-(4-chlorophénylsulfonyl)-benzylamino, pipéridino, 4-méthyl-pipéridino- ou hexaméthylène-imino
ou, lorsque $R_3$ représente un groupe carboxyle et $R_2$ représente un groupe n-butyle, $R_1$ en position 5 ou 6 représente aussi un groupe 2-oxo-1,2-dihydro-3,4-tétraméthylène-pyrrolidin-1-yle
ou, lorsque $R_3$ représente un groupe carboxyle et $R_2$ représente un groupe méthyle, éthyle, n-propyle, n-butyle ou méthylmercapto, $R_1$ en position 6 représente aussi un groupe pyrrolidinocarbonylamino
ou, lorsque $R_3$ représente un groupe tétrazolyle et $R_2$ représente un groupe n-butyle, $R_1$ en position 5 ou 6 représente aussi un groupe n-pentylamino substitué par un groupe méthylaminocarbonyle ou cyclohexylaminocarbonyle ou en position 6 un groupe 3,3-diméthyl-glutarimido ou 4,4-tétraméthylène-glutarimido,
ou, lorsque $R_3$ représente un groupe tétrazolyle et $R_2$ représente un groupe éthyle ou n-propyle, $R_1$ en position 6 représente aussi un groupe N-benzènesulfonyl-méthylamino ou, lorsque $R_3$ représente un groupe tert-butoxycarbonyle et
$R_2$ représente un groupe N-butyle, $R_1$ en position 6 représente aussi un groupe 2-carboxycyclohexylméthylcarbonylamino ou pyrrolidinocarbonylamino,
$R_2$ représente un atome d'hydrogène ou un groupe alkyle linéaire ou ramifié de 1 à 4 atomes de carbone, dans lequel un groupe méthylène peut être remplacé par un atome de soufre,
$R_3$ représente un groupe carboxyle, cyano, 1H-tétrazolyle ou 1-triphénylméthyl-tétrazolyle ou un groupe alcoxycarbonyle de 2 à 5 atomes de carbone au total et
$R_4$ représente un atome d'hydrogène, de fluor, de chlore ou de brome,
leurs mélanges d'isomères 1, 3 et leurs sels avec des acides ou des bases inorganiques ou organiques.

**3.** Benzimidazoles de formule générale I selon la revendication 1, dans laquelle

$R_1$ en position 6 représente un groupe 1-méthylbenzimidazol-2-yle, 3,4,5,6-tétrahydro-phtalimido, 2,3-diphénylmaléimido, 2,3-diméthyl-maléimido, N-phénylméthanesulfonylméthylamino, 2-oxo-pyrrolidin-1-yle, 2-oxo-pipéridin-1-yle, 2-oxo-hexaméthylène-imino, 2-oxo-3,4-tétraméthylènepyrrolidin-2-yle, 3,3-diméthylglutarimido, N-méthylaminocarbonyl-n-pentylamino, propanesultam-1-yle ou butanesultam-1-yle,
$R_2$ représente le groupe méthyle, éthyle, n-propyle ou n-butyle,
$R_3$ représente un groupe carboxyle ou 1H-tétrazolyle et
$R_4$ représente un atome d'hydrogène,
et leurs sels avec des acides ou des bases organiques ou inorganiques.

**4.** Benzimidazoles de formule générale I selon la revendication 1 :

acide
4'-[[2-n-propyl-6-(1-méthylbenzimidazol-2-yl)-benzimidazol-1-yl]méthyl]biphényle-2-carboxylique,
acide
4'-[[2-n-butyl-6-13,4,5,6-tétrahydro-phtalimino)-benzimidazol-1-yl]méthyl]biphényle-2-carboxylique,
acide
4'-[[2-n-butyl-6-(2,3-diphényl-maléimido)-benzimidazol-1-yl]méthyl]biphényle-2-carboxylique,
acide

4'-[[2-n-butyl-6-(2,3-diméthyl-maléimido)-benzimidazol-1-yl]méthyl]biphényle-2-carboxylique,
4'-[[2-n-butyl-6-(N-phénylméthanesulfonyl-méthylamino)-benzimidazol-1-yl]méthyl]biphényle-2-carboxylique,
4'-[[2-n-butyl-6-(2-oxo-pipéridin-1-yl)-benzimidazol-1-yl]méthyl]-2-(1H-tétrazol-5-yl)-biphényle,
4'-[[2-n-butyl-6-(2-oxo-pyrrolidin-1-yl)-benzimidazol-1-yl] méthyl]-2-(1H-tétrazol-5-yl)-biphényle,
4'-[[2-n-butyl-6-(2-oxo-hexaméthylène-imino)-benzimidazol-1-yl]méthyl]-2-(1H-tétrazol-5-yl)-biphényle,
4'-[[2-n-butyl-6-(3,3-diméthylg1utarimido)-benzimidazol-1-yl]-méthyl]-2-(1H-tétrazol-5-yl)-biphényle,
4'-[[2-n-butyl-6-(N-méthylaminocarbonyl-n-pentylamino)-benzimidazol-1-yl]méthyl]-2-(1H-tétrazol-5-yl)-biphényle,
4'-[[2-n-butyl-6-(cyclohexylaminocarbonyl-n-pentylamino)-benzimidazol-1-yl]méthyl]-2-(1H-tétrazol-5-yl)-biphényle,

acide

4'-[[2-n-butyl-6-(2-oxo-3,4-tétraméthylène-pyrrolidin-1-yl)benzimidazol-1-yl]méthyl]biphényle-2-carboxylique,
4'-[[2-n-butyl-6-(propanesultam-1-yl)-benzimidazol-1-yl]méthyl]-2-(1H-tétrazol-5-yl)-biphényle,
4'-[[2-n-propyl-6-(butanesultam-1-yl)-benzimidazol-1-yl]méthyl]-2-(1H-tétrazol-5-yl)-biphényle,
4'-[[2-n-butyl-6-(butanesultam-1-yl)-benzimidazol-1-yl]méthyl]-2-(1H-tétrazol-5-yl)-biphényle,
4'-[[2-n-propyl-6-(1-méthyl-benzimidazol-2-yl)-benzimidazol-1-yl]méthyl]-2-(1H-tétrazol-5-yl)-biphényle et
4'-[[2-n-propyl-6-(2-oxo-pipéridin-1-yl)-benzimidazol-1-yl]méthyl]-2-(1H-tétrazol-5-yl)-biphényle

et leurs sels avec des acides ou des bases inorganiques ou organiques.

**5.** Sels physiologiquement acceptables des composés selon au moins l'une des revendications 1 à 4 avec des acides ou des bases inorganiques ou organiques.

**6.** Médicament contenant un composé de formule générale I dans laquelle $R_1$, $R_2$ et $R_4$ ainsi que $R_3$ sont tels que définis dans au moins l'une des revendications 1 à 4 à l'exception des groupes tert-butoxycarbonyle, cyano et 1-triphénylméthyl-tétrazolyle, ou un sel physiologiquement acceptable de celui-ci selon la revendication 5 et éventuellement une autre substance active outre éventuellement un ou plusieurs supports et/ou diluants inertes.

**7.** Utilisation d'un composé de formule générale I dans laquelle $R_1$, $R_2$ et $R_4$ ainsi que $R_3$ sont tels que définis dans au moins l'une des revendications 1 à 4 à l'exception des groupe tert-butoxycarbonyle, cyano et 1-triphénylméthyl-tétrazolyle, ou d'un sel physiologiquement acceptable de celui-ci selon la revendication 5 pour la préparation d'un médicament à action antagoniste de l'angiotensine.

**8.** Procédé de préparation d'un médicament selon la revendication 6, caractérisé en ce que, par voie non chimique, un composé de formule générale I dans laquelle $R_1$, $R_2$ et $R_4$ ainsi que $R_3$ sont tels que définis dans au moins l'une des revendications 1 à 4 à l'exception du groupe tert-butoxycarbonyle, cyano et 1-triphénylméthyltétrazolyle, ou un sel physiologiquement acceptable de celui-ci est incorporé dans un ou plusieurs supports et/ou diluants

inertes.

**9.** Procédé de préparation des benzimidazoles selon les revendications 1 à 5, caractérisé en ce que

a) un composé de formule générale

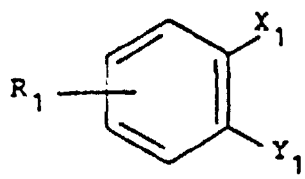

(II)

dans laquelle

$R_1$ est défini comme dans les revendications 1 à 4,
l'un des restes $X_1$ et $Y_1$ représente un groupe de formule générale

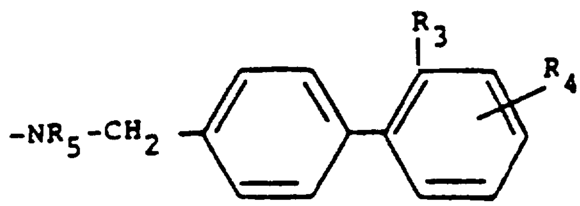

et l'autre des restes $X_1$ et $Y_1$ représente un groupe de formule générale

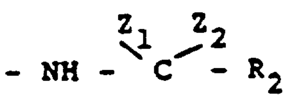

$R_2$ à $R_4$ étant définis comme dans les revendications 1 à 4,
$R_5$ représente un atome d'hydrogène ou un groupe $R_2CO$, $R_2$ étant défini comme indiqué ci-dessus,
$Z_1$ et $Z_2$, qui peuvent être identiques ou différents, représentent des groupes amino éventuellement substitués ou des groupes hydroxyle ou mercapto éventuellement substitués par des groupes alkyle inférieurs ou
$Z_1$ et $Z_2$, avec un atome d'oxygène ou de soufre, représentent un groupe imino éventuellement substitué par un groupe alkyle de 1 à 3 atomes de carbone, un groupe alkylènedioxy ou alkylènedithio de 2 ou 3 atomes de carbone dans chaque cas, l'un des restes $X_1$ et $Y_1$ devant toutefois représenter un groupe de formule générale

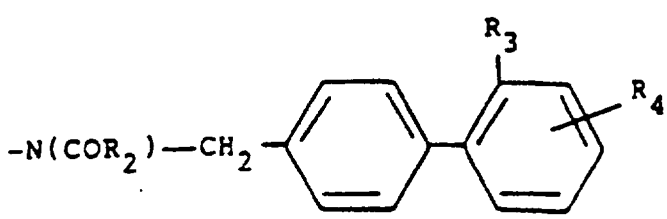

ou

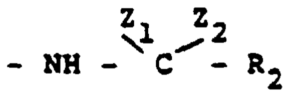

est cyclisé puis un N-oxyde éventuellement ainsi obtenu est réduit ou

b) un benzimidazole de formule générale

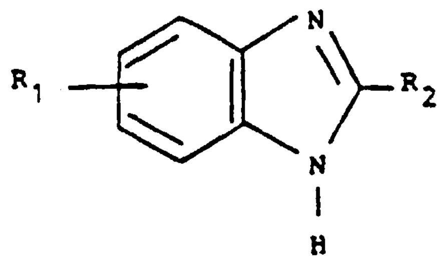

(III)

dans laquelle

$R_1$ et $R_2$ sont définis comme dans les revendications 1 à 4, est mis à réagir avec un composé du biphényle de formule générale

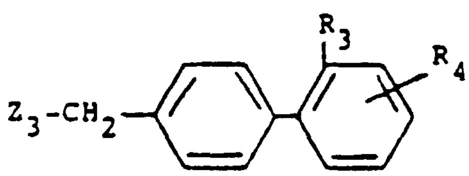

(IV)

dans laquelle
$R_3$ et $R_4$ sont définis comme dans les revendications 1 à 4 et
$Z_3$ représente un groupe partant nucléophile tel qu'un atome d'halogène ou un groupe sulfonyloxy substitué, ou

c) pour la préparation d'un composé de formule générale I dans laquelle $R_3$ représente un groupe carboxyle, un composé de formule générale

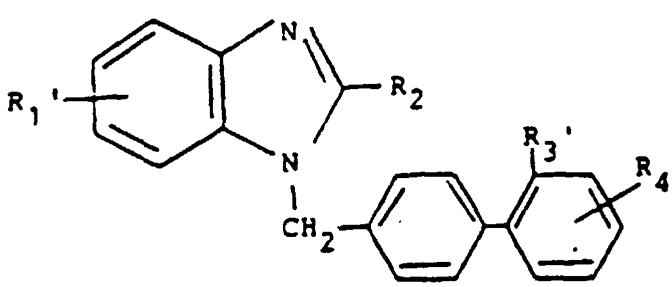

(V)

dans laquelle

$R_2$ et $R_4$ sont définis comme dans les revendications 1 à 4,
$R_1$' possède les significations indiquées pour $R_1$ dans les revendications 1 à 4 et représente un groupe 3-alcoxycarbonylpropionyle ou 3-alcoxycarbonyl-2-méthylpropionyle dans lequel la partie alcoxy peut

contenir dans chaque cas 1 à 3 atomes de carbone, et $R_3$' représente un groupe qui peut être converti en un groupe carboxyle par hydrolyse, thermolyse ou hydrogénolyse, est converti en un composé carboxylé correspondant ou

d) pour la préparation d'un composé de formule générale I dans laquelle $R_3$ représente un groupe 1H-tétrazolyle, un reste protecteur est clivé d'un composé de formule générale

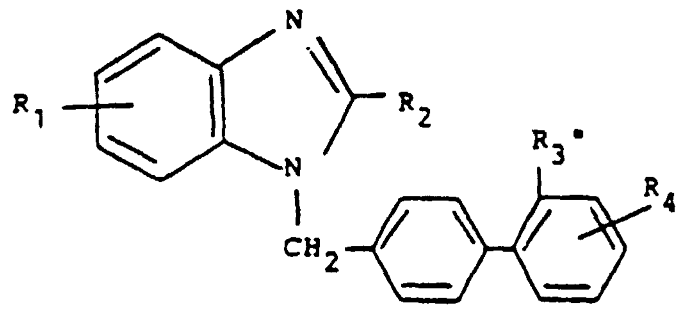

(VI)

dans laquelle

$R_1$, $R_2$ et $R_4$ sont définis comme dans les revendications 1 à 4 et
$R_3$" représente un groupe 1H-tétrazolyle protégé en position 1 ou 3 par un reste protecteur, ou

e) pour la préparation d'un composé de formule générale I dans laquelle $R_3$ représente un groupe 1H-tétrazolyle, un composé de formule générale

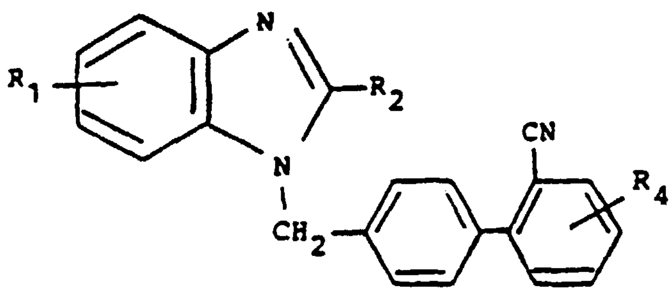

(VII)

dans laquelle
$R_1$, $R_2$ et $R_4$ sont définis comme dans les revendications 1 à 4, est mis à réagir avec l'acide azothydrique ou avec ses sels, ou
f) pour la préparation de composés de formule générale I dans laquelle $R_1$ représente un groupe pentaméthylèneoxazolin-2-yle, un composé de formule générale

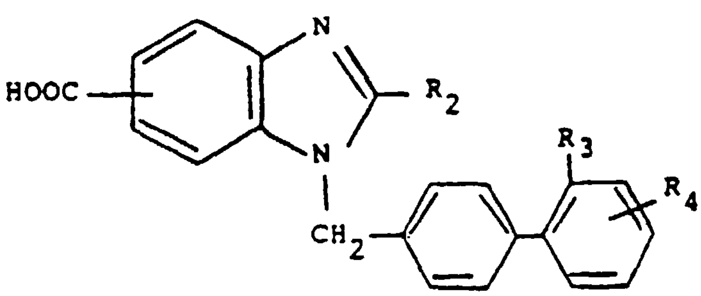

(VIII)

dans laquelle
$R_2$ à $R_4$ sont définis comme dans les revendications 1 à 4, est mis à réagir avec le 1-aminométhyl-cyclohexanol en présence d'un agent activant l'acide ou

g) pour la préparation d'un composé de formule générale I dans laquelle $R_1$ représente un groupe 2-oxo-3,4-tétraméthylène-pyrrolidin-1-yle, un composé de formule générale

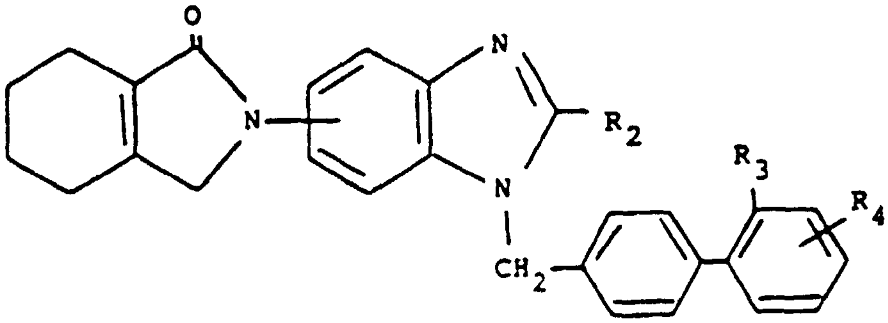

(IX)

dans laquelle

$R_2$, $R_3$ et $R_4$ sont définis comme dans les revendications 1 à 4, est hydrogéné ou

h) pour la préparation de composés de formule générale I dans laquelle $R_1$ représente un groupe amino substitué par un groupe bicyclohexylcarbonyle ou biphénylcarbonyle, qui peuvent être substitués chacun en outre sur l'atome N par un groupe alkyle de 1 à 3 atomes de carbone, un groupe aminocarbonylamino substitué par un groupe bicyclohexyle ou biphényle, qui peut être substitué en outre sur l'atome N par un ou deux groupes alkyle de 1 à 3 atomes de carbone chacun, un groupe maléamido ou maléimido éventuellement mono- ou disubstitué par un groupe alkyle de 1 à 3 atomes de carbone ou par un groupe phényle, les substituants pouvant être identiques ou differents, un groupe alkylamino ou phénylalkylamino substitué par un groupe alkylsulfonyle de 4 à 6 atomes de carbone ou par un groupe phénylalkylsulfonyle, dans lesquels la partie alkyle peut contenir dans chaque cas 1 à 3 atomes de carbone, un groupe amino ou alkylamino substitué par un groupe naphtalènesulfonyle, qui peuvent être substitués dans le cycle naphtalène par un groupe dialkylamino, par un ou deux groupes alcoxy, la partie alkyle pouvant contenir dans chaque cas 1 à 3 atomes de carbone, un groupe 7-nitrobenzofurazan-4-yl-aminoalcanoylamino, dans lequel la partie alcanoyle peut contenir 2 ou 3 atomes de carbone, un groupe benzofuranecarbonylamino ou 7-nitro-benzofurazan-4-ylamino ou encore, lorsque $R_3$ représente un groupe carboxyle et $R_2$ représente un groupe n-butyle, $R_1$ en position 6 représente un groupe amino substitué par un groupe phénylsulfonyle, cyclohexylméthylaminocarbonyle, 2-carboxycyclohexylméthylcarbonyle, 2-tert-butoxycarbonyl-cyclohexylméthylcarbonyle, 2-carboxy-3,4,5,6-tétrahydrobenzoyle, N-méthylphénylaminocarbonyle ou 3-cyclohexylpropyle, un groupe méthylamino substitué par un groupe propylsulfonyle, phénylsulfonyle, méthylphénylsulfonyle ou chlorophénylsulfonyle, un groupe n-pentylamino substitué par un groupe phénylsulfonyle ou méthoxyphénylsulfonyle, un groupe n-propylamino substitué par un groupe méthylphénylsulfonyle ou méthoxyphénylsulfonyle, un groupe isopropylamino substitué par un groupe benzoyle ou chlorophénylsulfonyle, un groupe N-acétyl-cyclohexylméthylamino, 3,4,5,6-tétrahydrophtalimido, hexahydrohomophtalimido, N-méthanesulfonyl-2-phényléthylamino ou N-chlorophénylsulfonylbenzylamino

ou, lorsque $R_3$ représente un groupe carboxyle et $R_2$ représente un groupe n-butyle, $R_1$ en position 5 ou 6 représente un groupe 2-oxo-1,2-dihydro-3,4-tétraméthylènepyrrolidin-1-yle

ou, lorsque $R_3$ représente un groupe carboxyle et $R_2$ représente un groupe méthyle, éthyle, n-propyle, n-butyle ou méthylmercapto, $R_1$ en position 6 représente un groupe pyrrolidinocarbonylamino

ou encore, lorsque $R_3$ représente un groupe tétrazolyle et $R_2$ représente un groupe n-butyle, $R_1$ en position 5 ou 6 représente un groupe n-pentylamino substitué par un groupe méthylaminocarbonyle ou cyclohexylaminocarbonyle ou en position 6 un groupe 3,3-diméthyl-glutarimido ou 4,4-tétraméthylène-glutarimido,

ou encore, lorsque $R_3$ représente un groupe tétrazolyle et $R_2$ représente un groupe éthyle ou n-propyle, $R_1$ en position 6 représente un groupe N-benzènesulfonyl-méthylamino,

ou encore, lorsque $R_3$ représente un groupe tert-butoxycarbonyle et $R_2$ représente un groupe N-butyle, $R_1$ en position 6 représente un groupe 2-carboxycyclohexylméthylcarbonylamino ou pyrrolidinocarbonylamino, un composé de formule générale

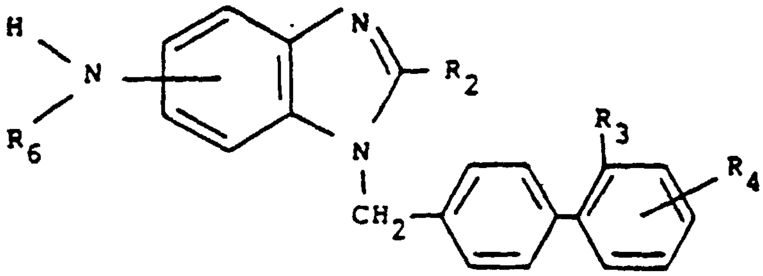

(X)

dans laquelle

$R_2$, $R_3$ et $R_4$ sont définis comme dans les revendications 1 à 4 et
$R_6$ représente un atome d'hydrogène, un groupe n-pentyle, cyclohexylméthyle, alkyle ou phénylalkyle de 1 à 3 atomes de carbone dans la partie alkyle dans chaque cas, est mis à réagir avec un composé de formule générale

$$Z_4 - W - R_7 \qquad (XI)$$

dans laquelle
$Z_4$ représente un groupe partant nucléophile,
W représente un groupe -CO- ou $-SO_2-$ et
$R_7$ représente un groupe 2-hydroxycarbonyl-éthényle dans lequel la partie éthényle est mono- ou disubstituée par un groupe alkyle de 1 à 3 atomes de carbone ou par un groupe phényle et les substituants peuvent être identiques ou différents,
un groupe alkyle de 3 à 6 atomes de carbone, un groupe phénylalkyle de 1 à 3 atomes de carbone dans la partie alkyle, un groupe naphtalène éventuellement substitué par un groupe dialkylamino, par un ou deux groupes alcoxy, dans lesquels la partie alkyle peut contenir dans chaque cas 1 à 3 atomes de carbone, un groupe méthyle, phényle, méthylphényle, méthoxyphényle, chlorophényle, biphényle, bicyclohexyle, 2-carboxy-cyclohexylméthyle, 2-carboxy-3,4,5,6-tétrahydrophényle, 3-carboxy-1,1-diméthyl-propyle, 3-carboxy-2,2-tétraméthylènepropyle, 7-nitro-benzofurazan-4-yl-aminométhyle ou 7-nitro-benzofurazan-4-yl-aminoéthyle, ou encore, lorsque W représente un groupe -CO-, un groupe $R_8NR_9$ dans lequel
$R_8$ représente un atome d'hydrogène ou un groupe alkyle de 1 à 3 atomes de carbone,
$R_9$ représente un groupe méthyle, cyclohexyle, cyclohexylméthyle, phényle, biphényle ou bicyclohexyle ou $R_8$ et $R_9$ représentent avec l'atome d'azote situé entre eux un groupe pyrrolidino

ou encore $Z_4$ représente avec $R_9$ une autre liaison carbone-azote,
ou encore R- représente avec W un groupe 7-nitrobenzofurazan-4-ylamino, ou

i) pour la préparation de composés de formule générale I dans laquelle $R_1$ représente un groupe tétrahydrobenzimidazolyle ou imidazopyridinyle ou un groupe benzimidazolyle éventuellement substitué dans le noyau phényle par un atome de fluor, de chlore ou de brome, par un groupe alkyle de 1 à 3 atomes de carbone, par un groupe alcoxy de 1 à 3 atomes de carbone ou par un groupe trifluorométhyle, le groupe NH des cycles imidazole cités précédemment pouvant être substitué en outre par un groupe alkyle de 1 à 6 atomes de carbone ou par un groupe cycloalkyle de 3 à 7 atomes de carbone, un groupe hydroxycycloalkylaminocarbonyle de 5 à 7 atomes de carbone dans la partie cycloalkyle, qui peut être substitué en outre sur l'atome N par un groupe alkyle de 1 à 3 atomes de carbone, ou un groupe hydroxyalkylaminocarbonyle linéaire ou ramifié de 4 à 6 atomes de carbone dans la partie alkyle, un composé de formule générale

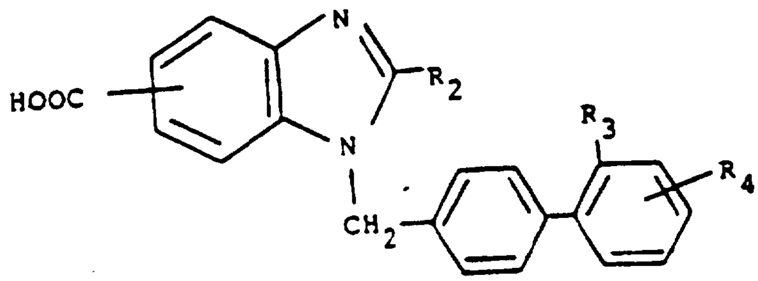

(XII)

dans laquelle

$R_2$ à $R_4$ sont définis comme dans les revendications 1 à 4, ou ses dérivés réactifs sont mis à réagir avec une amine de formule générale

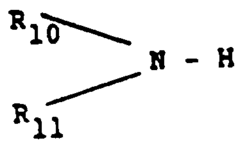

(XIII)

dans laquelle
$R_{10}$ représente un atome d'hydrogène, un groupe cycloalkyle ou un groupe alkyle de 1 à 6 atomes de carbone et $R_{11}$ représente un groupe hydroxyalkyle de 4 à 6 atomes de carbone, un groupe hydroxycycloalkyle de 5 à 7 atomes de carbone ou un groupe 2-aminophényle, qui peut être substitué dans le noyau phényle par un atome de fluor, de chlore ou de brome, par un groupe alkyle de 1 à 3 atomes de carbone, par un groupe alcoxy de 1 à 3 atomes de carbone ou par un groupe trifluorométhyle, un groupe 2-aminocyclohexyle ou 2-aminopyridyle, avec décarboxylation éventuellement simultanée ou

k) pour la préparation de composés de formule générale I dans laquelle $R_1$ représente un groupe dihydropyridazin-3-one ou pyridazin-3-one qui peut être substitué en position 2 par un groupe aikyle de 1 à 3 atomes de carbone éventuellement substitué par un groupe phényle ou dans le squelette carboné par un ou deux groupes alkyle de 1 à 3 atomes de carbone chacun, un acide carboxylique de formule générale

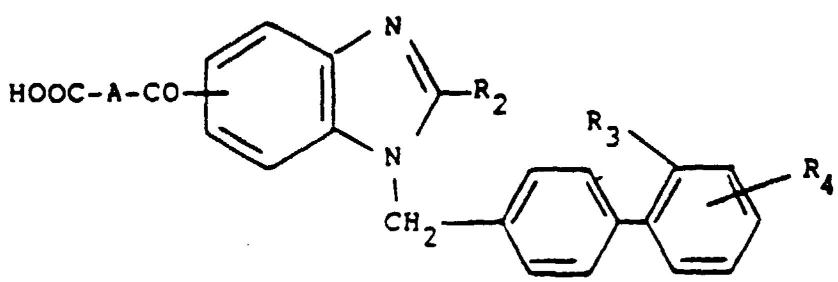

(XIV)

dans laquelle

$R_1$ à $R_4$ sont définis comme dans les revendications 1 à 4 et A représente un groupe éthylène ou éthénylène éventuellement substitué par un ou deux groupes alkyle de 1 à 3 atomes de carbone chacun, ou ses dérivés d'acide réactifs sont mis à réagir avec une hydrazine de formule générale

$$H_2N - NHR_{12} \qquad (XV)$$

dans laquelle
$R_{12}$ représente un atome d'hydrogène ou un groupe alkyle de 1 à 3 atomes de carbone éventuellement substitué par un groupe phényle, et
si nécessaire, un reste protecteur utilisé pendant les réactions a) à k) pour la protection de groupes réactifs est clivé et/ou
si on le souhaite, un mélange d'isomères 1, 3 d'un composé de formule générale I ainsi obtenu est ensuite résolu par séparation d'isomères en ses isomères 1 et 3 ou un composé de formule générale I ainsi obtenu est converti en son sel,
en particulier pour l'utilisation pharmaceutique en son sel physiologiquement acceptable avec un acide ou une base inorganique ou organique.